# EUROPEAN PATENT SPECIFICATION

(11) **EP 1 539 968 B1**
(45) Date of publication and mention of the grant of the patent: **26.04.2017**
(21) Application number: 03759174.0
(22) Date of filing: 03.07.2003
(51) Int. Cl.: C12N 15/82, C12N 5/04, A01H 5/00, A01H 5/10

(54) **PLASTID GENETIC ENGINEERING VIA SOMATIC EMBRYOGENESIS**
GENTECHNISCHE MANIPULATION VON PLASTIDEN ÜBER SOMATISCHE EMBRYOGENESE
PRODUCTION DE PLASTES PAR GENIE GENETIQUE PAR L'INTERMEDIAIRE D'UNE EMBRYOGENESE SOMATIQUE

(30) Priority: 03.07.2002 US 393651 P
(43) Date of publication of application: 15.06.2005
(60) Divisional application: 10013115.0
(73) Proprietor: The Trustees of the University of Pennsylvania, Philadelphia, PA 19104-6283 (US)
(72) Inventor: DANIELL, Henry, Winter Park, FL 32792 (US)
(74) Representative: Zwicker, Jörk
(86) International application number: PCT/US2003/021157
(87) International publication number: WO 2004/005480

(56) References cited:
- US-A- 5 932 479
- KHAN M S ET AL: "FLUORESCENT ANTIBIOTIC RESISTANCE MARKER FOR TRACKING PLASTID TRANSFORMATION IN HIGHER PLANTS" NATURE BIOTECHNOLOGY, NATURE PUB. CO, NEW YORK, NY, US, vol. 17, September 1999 (1999-09), pages 910-915, XP002943529 ISSN: 1087-0156
- DANIELL HENRY ET AL: "Expression of the native cholera toxin B subunit gene and assembly as functional oligomers in transgenic tobacco chloroplasts" JOURNAL OF MOLECULAR BIOLOGY, vol. 311, no. 5, 31 August 2001 (2001-08-31), pages 1001-1009, XP002381982 ISSN: 0022-2836
- HIBBERD J M ET AL: "Transient expression of green fluorescent protein in various plastid types following microprojectile bombardment" PLANT JOURNAL, BLACKWELL SCIENTIFIC PUBLICATIONS, OXFORD, GB, vol. 16, no. 5, December 1998 (1998-12), pages 627-632, XP002275465 ISSN: 0960-7412
- NAGAMORI EIJI ET AL: "Release of embryogenic carrot cells with high regeneration potency from immobilized alginate beads" JOURNAL OF BIOSCIENCE AND BIOENGINEERING, vol. 88, no. 2, August 1999 (1999-08), pages 226-228, XP002381983 ISSN: 1389-1723
- MOGHAIEB REDA ELWANY ABEDELHALEEM ET AL: "Expression of betaine aldehyde dehydrogenase gene in transgenic tomato hairy roots leads to the accumulation of glycine betaine and contributes to the maintenance of the osmotic potential under salt stress" SOIL SCIENCE AND PLANT NUTRITION, vol. 46, no. 4, December 2000 (2000-12), pages 873-883, XP002381984 ISSN: 0038-0768
- KUMAR SHASHI ET AL: "Plastid-expressed betaine aldehyde dehydrogenase gene in carrot cultured cells, roots, and leaves confers enhanced salt tolerance" PLANT PHYSIOLOGY (ROCKVILLE), vol. 136, no. 1, September 2004 (2004-09), pages 2843-2854, XP002381985 ISSN: 0032-0889
- DANIELL ET AL: 'Marker free transgenic plants: engineering the chloroplast genome without hte use of antibiotic selection' CURR. GENET. vol. 39, no. 2, April 2001, pages 109 - 116, XP002979049
- YE ET AL: 'Plastid-expressed 5-enolpyruvylshikimate-3-phosphate synthase genes provide high level glyphosate tolerance in tobacco' PLANT JOURNAL vol. 25, no. 3, February 2001, pages 261 - 270, XP002187551
- DANIELL H ET AL: "Breakthrough in chloroplast genetic engineering of agronomically important crops", TRENDS IN BIOTECHNOLOGY, ELSEVIER PUBLICATIONS, CAMBRIDGE, GB, vol. 23, no. 5, 1 May 2005 (2005-05-01), pages 238-245, XP025290703, ISSN: 0167-7799, DOI: DOI:10.1016/J.TIBTECH.2005.03.008 [retrieved on 2005-05-01]
- SHASHI KUMAR ET AL: "Stable transformation of the cotton plastid genome and maternal inheritance of transgenes", PLANT MOLECULAR BIOLOGY, KLUWER ACADEMIC PUBLISHERS, DORDRECHT, NL, vol. 56, no. 2, 1 September 2004 (2004-09-01), pages 203-216, XP019262563, ISSN: 1573-5028, DOI: DOI:10.1007/S11103-004-2907-Y
- DANIELL HENRY ET AL: "Milestones in chloroplast genetic engineering: An environmentally friendly era in biotechnology", February 2002 (2002-02), TRENDS IN PLANT SCIENCE, VOL. 7, NR. 2, PAGE(S) 84-91 ISSN: 1360-1385

### SEQUENCE LISTING

<110> Daniell, Henry
<120> Plastid Genetic Engineering Via Somatic Embryogenesis
<130> CHL-T107C2Z2
<140> US 10/519,821
   <141> 2004-12-30
<150> PCT/US2003/021157
   <151> 2003-07-03
<160> 3
<170> PatentIn version 3.2
<210> 1
   <211> 2800
   <212> DNA
   <213> Artificial Sequence
<220>
   <223> aadA/BADH expression cassette
<400> 1
   agcttgcggg ccccccctcg aggtcgacgg tatcgatgag cctgattatc cctaagccca     60
   atgtgagttt ttctagttgg atttgctccc ccgccgtcgt tcaatgagaa tggataagag    120
   gctcgtggga ttgacgtgag ggggcaggga tggctatatt tctgggagcg aactccgggc    180
   gaatatgaag cgcatggata caagttatgc cttggaatga aagacaattc cgaatccgct    240
   ttgtctaccc gatacaagtg agttgtaggg aggcaaccat ggcagaagcg gtgatcgccg    300
   aagtatcgac tcaactatca gaggtagttg gcgtcatcga gcgccatctc gaaccgacgt    360
   tgctggccgt acatttgtac ggctccgcag tggatggcgg cctgaagcca cacagtgata    420
   ttgatttgct ggttacggtg acggtgaccg taaggcttga tgaaacaacg cggcgagctt    480
   tgatcaacga ccttttggaa acttcggctt cccctggaga gagcgagatt ctccgcgctg    540
   tagaagtcac cattgttgtg cacgacgaca tcattccgtg gcgttatcca gctaagcgcg    600
   aactgcaatt tggagaatgg cagcgcaatg acattcttgc aggtatcttc gagccagcca    660
   cgatcgacat tgatctggct atcttgctgg caaaagcaag agaacatagc gttgccttgg    720
   taggtccagc ggcggaggaa ctctttgatc cggttcctga acaggatcta tttgaggcgc    780
   taaatgaaac cttaacgcta tggaactcgc cgcccgactg ggctggcgat gagcgaaatg    840
   tagtgcttac gttgttccgc atttggtaca gcgcagtaac cggcagaatc gcgccgaagg    900
   atgtcgctgc cgactgggca atggagcgcc tgccggccca gtatcagccc gtcatacttg 960
   aagctagaca ggcttatctt ggacaagaag aagatcgctt ggcctcgcgc gcagatcagt 1020
   tggaagaatt tgttcactac gtgaaaggcg agatcaccaa ggtagtcggc aaataaaaag 1080
   ccgaatctag agcgatcctg gcctagtcta taggaggttt tgaaaagaaa ggagcaataa 1140
   tcattttctt gttctatcaa gagggtgcta ttgctccttt cttttttctt ttttatttat 1200
   ttactagtat tttacttaca tagacttttt tgtttacatt atagaaaaag aaggagaggt 1260
   tattttcttg catttattca tgattgagta ttctattttg attttgtatt tgtttgggct 1320
   gcgcggggag accacaacgg tttccctcta gaaataattt tgtttaactt taagaaggag 1380
   atataccatg gcgttcccaa ttcctgctcg tcagctattc atcgacggag agtggagaga 1440
   acccattaaa aaaaatcgca tacccgtcat caatccgtcc actgaagaaa tcatcggtga 1500
   tattccggca gccacggctg aagatgtgga ggttgcggtg gtggcagctc gaagagcctt 1560
   taggaggaac aattggtcag caacatctgg ggctcatcgt gccacatact tgcgtgctat 1620
   tgctgctaag ataacagaaa aaaaagatca tttcgttaaa ctggaaacca ttgattctgg 1680
   gaaacctttt gatgaagcag tgctggacat tgatgacgtt gcttcatgtt ttgaatattt 1740
   tgccggacaa gcagaagctc ttgatggtaa acaaaaggct ccagtcaccc tgcctatgga 1800
   aaggttcaaa agtcatgttc tcaggcagcc ccttggtgtt gttggattaa tatccccatg 1860
   gaattaccca cttctaatgg ctacatggaa aattgctcca gcacttgctg ctgggtgtac 1920
   agctgtactt aagccatccg agttggcatc tgtgacttgt ctagaattcg gtgaagtttg 1980
   caacgaagtg ggacttcctc caggcgtgtt gaatatcttg acaggattag gtccagatgc 2040
   tggtgcacca ttagtatcac accccgatgt tgacaagatt gcctttactg ggagtagtgc 2100
   cactggaagc aaggttatgg cttctgctgc ccaattggtt aagcctgtta cattagaact 2160
   tgggggtaaa agtcctattg tagtgtttga agatgttgat attgataaag ttgtggaatg 2220
   gactattttt ggctgtttct ggacaaatgg tcaaatatgt agtgcaacgt ctagactgct 2280
   tgtgcatgaa agtattgcag ctgagtttgt tgataagctt gtaaaatgga cgaaaaacat 2340
   taaaatttct gacccatttg aagaaggatg ccggcttggc cctgttatta gtaaaggaca 2400
   gtacgacaaa attatgaagt tcatatcaac agcaaagagt gagggggcaa ctattttgta 2460
   tggaggttcc cgtcctgagc atttgaagaa aggttattac attgaaccca ccattgtaac 2520
   tgatatctcc acatccatgc aaatatggaa agaggaagtt tttggccctg tcttgtgtgt 2580
   taaaacattt agttccgaag atgaagccat tgcattggca aatgatacag agtacggttt 2640
   agctgctgct gtgttttcta atgatcttga aagatgtgag aggataacga aggctctaga 2700
   agttggagct gtttgggtta attgctcaca accatgcttt gttcaagctc cttggggagg 2760
   catcaagcgt agtggttttg gacgtgaact tggagaatgg 2800
<210> 2
   <211> 3119
   <212> DNA
   <213> Artificial Sequence
<220>
   <223> gfp/BADH expression cassette
<400> 2
   cgggcccccc ctcgaggtcg acggtatcga tgagcctgat tatccctaag cccaatgtga 60
   gtttttctag ttggatttgc tcccccgccg tcgttcaatg agaatggata agaggctcgt 120
   gggattgacg tgagggggca gggatggcta tatttctggg agcgaactcc gggcgaatat 180
   gaagcgcatg gatacaagtt atgccttgga atgaaagaca attccgaatc cgctttgtct 240
   accgggagac cacaacggtt tccctctaga aataattttg tttaacttta agaaggagat 300
   atacccatgt ccatgagtaa aggagaagaa cttttcactg gagttgtccc aattcttgtt 360
   gaattagatg gtgatgttaa tgggcacaaa ttttctgtca gtggagaggg tgaaggtgat 420
   gcaacatacg gaaaacttac ccttaaattt atttgcacta ctggaaaact acctgttcca 480
   tggccaacac ttgtcactac tttctcttat ggtgttcaat gcttttcaag atacccagat 540
   catatgaagc ggcacgactt cttcaagagc gccatgcctg agggatacgt gcaggagagg 600
   accatctctt tcaaggacga cgggaactac aagacacgtg ctgaagtcaa gtttgaggga 660
   gacaccctcg tcaacaggat cgagcttaag ggaatcgatt tcaaggagga cggaaacatc 720
   ctcggccaca agttggaata caactacaac tcccacaacg tatacatcac ggcagacaaa 780
   caaaagaatg gaatcaaagc taacttcaaa attagacaca acattgaaga tggaagcgtt 840
   caactagcag accattatca acaaaatact ccaattggcg atggccctgt ccttttacca 900
   gacaaccatt acctgtccac acaatctgcc ctttcgaaag atcccaacga aaagagagac 960
   cacatggtcc ttcttgagtt tgtaacagct gctgggatta cacatggcat ggatgaacta 1020
   tacaaataat ctagaaagcc gaattctgca gatcgaacac ggaattcaat ggaagcaatg 1080
   ataaaaaaat acaaatagaa aaggaaaggg aggaaataca aaaaaataga agagaaaagt 1140
   catacaaagt tatatacaaa tgactacccc cctttttgta tttccttaat ttatttcctt 1200
   aattgaattt cgatggatac aagttatgcc ttggaatgaa tttcggttga ttaggactag 1260
   cgataagctt gatatcgaat tcggcttgat atcgtcgacg tagagaagtc cgtatttttc 1320
   caatcaactt cattaaaaat ttgaatagat ctacatacac cttggttgac acgagtatat 1380
   aagtcatgtt atactgttga ataaaaagcc ttccattttc tattttgatt tgtagaaaac 1440
   tagtgtgctt gggagtccct gatgattaaa taaaccaaga ttttccatgg cgttcccaat 1500
   tcctgctcgt cagctattca tcgacggaga gtggagagaa cccattaaaa aaaatcgata 1560
   cccgtcatca atccgtccac tgaagaaatc atcggtgata ttccggcagc cacggctgaa 1620
   gatgtggagg ttgcggtggt ggcagctcga agagccttta ggaggaacaa ttggtcagca 1680
   acatctgggg ctcatcgtgc cacatacttg cgtgctattg ctgctaagat aacagaaaaa 1740
   aaagatcatt tcgttaaact ggaaaccatt gattctggga aaccttttga tgaagcagtg 1800
   ctggacattg atgacgttgc ttcatgtttt gaatattttg ccggacaagc agaagctctt 1860
   gatggtaaac aaaaggctcc agtcaccctg cctatggaaa ggttcaaaag tcatgttctc 1920
   aggcagcccc ttggtgttgt tggattaata tccccatgga attacccact tctaatggct 1980
   acatggaaaa ttgctccagc acttgctgct gggtgtacag ctgtacttaa gccatccgag 2040
   ttggcatctg tgacttgtct agaattcggt gaagtttgca acgaagtggg acttcctcca 2100
   ggcgtgttga atatcttgac aggattaggt ccagatgctg gtgcaccatt agtatcacac 2160
   cccgatgttg acaagattgc ctttactggg agtagtgcca ctggaagcaa ggttatggct 2220
   tctgctgccc aattggttaa gcctgttaca ttagaacttg ggggtaaaag tcctattgta 2280
   gtgtttgaag atgttgatat tgataaagtt gtggaatgga ctatttttgg ctgtttctgg 2340
   acaaatggtc aaatatgtag tgcaacgtct agactgcttg tgcatgaaag tattgcagct 2400
   gagtttgttg ataagcttgt aaaatggacg aaaaacatta aaatttctga cccatttgaa 2460
   gaaggatgcc ggcttggccc tgttattagt aaaggacagt acgacaaaat tatgaagttc 2520
   atatcaacag caaagagtga gggggcaact attttgtatg gaggttcccg tcctgagcat 2580
   ttgaagaaag gttattacat tgaacccacc attgtaactg atatctccac atccatgcaa 2640
   atatggaaag aggaagtttt tggccctgtc ttgtgtgtta aaacatttag ttccgaagat 2700
   gaagccattg cattggcaaa tgatacagag tacggtttag ctgctgctgt gttttctaat 2760
   gatcttgaaa gatgtgagag gataacgaag gctctagaag ttggagctgt ttgggttaat 2820
   tgctcacaac catgctttgt tcaagctcct tggggaggca tcaagcgtag tggttttgga 2880
   cgtgaacttg gagaatgggg tatccagaat tacttgaata tcaagcaggt gactcaagat 2940
   atttctgatg aaccatgggg atggtacaag tctccttgaa agccgaattc cagcacactg 3000
   gcggccgtta ctagtggatc cactagtaac ggccgccagt gtgctggaat tcggctttct 3060
   agagcgatcc tggcctagtc tataggaggt tttgaaaaga aaggagcaat aatcatttt 3119
<210> 3
   <211> 2569
   <212> DNA
   <213> Artificial Sequence
<220>
   <223> aphA-6/nptII expression cassette
<400> 3
   cgggcccccc ctcgaggtcg acggtatcga tgagcctgat tatccctaag cccaatgtga 60
   gtttttctag ttggatttgc tcccccgccg tcgttcaatg agaatggata agaggctcgt 120
   gggattgacg tgagggggca gggatggcta tatttctggg agcgaactcc gggcgaatat 180
   gaagcgcatg gatacaagtt atgccttgga atgaaagaca attccgaatc cgctttgtct 240
   acctgcagcc cgggagacca caacggtttc cctctagaaa taattttgtt taactttaag 300
   aaggagatat accatggaat taccaaatat tattcaacaa tttatcggaa acagcgtttt 360
   agagccaaat aaaattggtc agtcgccatc ggatgtttat tcttttaatc gaaataatga 420
   aacttttttt cttaagcgat ctagcacttt atatacagag accacataca gtgtctctcg 480
   tgaagcgaaa atgttgagtt ggctctctga gaaattaaag gtgcctgaac tcatcatgac 540
   ttttcaggat gagcagtttg aattcatgat cactaaagcg atcaatgcaa aaccaatttc 600
   agcgcttttt ttaacagacc aagaattgct tgctatctat aaggaggcac tcaatctgtt 660
   aaattcaatt gctattattg attgtccatt tatttcaaac attgatcatc ggttaaaaga 720
   gtcaaaattt tttattgata accaactcct tgacgatata gatcaagatg attttgacac 780
   tgaattatgg ggagaccata aaacttacct aagtctatgg aatgagttaa ccgagactcg 840
   tgttgaagaa agattggttt tttctcatgg cgatatcacg gatagtaata tttttataga 900
   taaattcaat gaaatttatt ttttagatct tggtcgtgct gggttagcag atgaatttgt 960
   agatatatcc tttgttgaac gttgcctaag agaggatgca tcggaggaaa ctgcgaaaat 1020
   atttttaaag catttaaaaa atgatagacc tgacaaaagg aattattttt taaaacttga 1080
   tgaattgaat tgattccaag cattatctaa aatactccta gagcggcccg aacacggaat 1140
   tcaatggaag caatgataaa aaaatacaaa tagaaaagga aagggaggaa atacaaaaaa 1200
   atagaagaga aaagtcatac aaagttatat acaaatgact accccccttt ttgtatttcc   1260
   ttaatttatt tccttaattg aatttcgatg gatacaagtt atgccttgga atgaatttcg   1320
   gttgattagg actagatcgt cgacgtagag aagtccgtat ttttccaatc aacttcatta   1380
   aaaatttgaa tagatctaca tacaccttgg ttgacacgag tatataagtc atgttatact   1440
   gttgaataaa aagccttcca ttttctattt tgatttgtag aaaactagtg tgcttgggag   1500
   tccctgatga ttaaataaac caagattttc atatgattga acaagatgga ttgcacgcag   1560
   gttctccggc cgcttgggtg gagaggctat tcggctatga ctgggcacaa cagacaatcg   1620
   gctgctctga tgccgccgtg ttccggctgt cagcgcaggg gcgcccggtt ctttttgtca   1680
   agaccgacct gtccggtgcc ctgaatgaac tgcaggacga ggcagcgcgg ctatcgtggc   1740
   tggccacgac gggcgttcct tgcgcagctg tgctcgacgt tgtcactgaa gcgggaaggg   1800
   actggctgct attgggcgaa gtgccggggc aggatctcct gtcatctcac cttgctcctg   1860
   ccgagaaagt atccatcatg gctgatgcaa tgcggcggct gcatacgctt gatccggcta   1920
   cctgcccatt cgaccaccaa gcgaaacatc gcatcgagcg agcacgtact cggatggaag   1980
   ccggtcttgt cgatcaggat gatctggacg aagagcatca ggggctcgcg ccagccgaac   2040
   tgttcgccag gctcaaggcg cgcatgcccg acggcgatga tctcgtcgtg acccatggcg   2100
   atgcctgctt gccgaatatc atggtggaaa atggccgctt ttctggattc atcgactgtg   2160
   gccggctggg tgtggcggac cgctatcagg acatagcgtt ggctacccgt gatattgctg   2220
   aagagcttgg cggcgaatgg gctgaccgct tcctcgtgct ttacggtatc gccgctcccg   2280
   attcgcagcg catcgccttc tatcgccttc ttgacgagtt cttctgatct agagcgatcc   2340
   tggcctagtc tataggaggt tttgaaaaga aaggagcaat aatcattttc ttgttctatc   2400
   aagagggtgc tattgctcct ttcttttttt ctttttattt atttactagt attttactta   2460
   catagacttt tttgtttaca ttatagaaaa agaaggagag gttattttct tgcatttatt   2520
   catgattgag tattctattt tgattttgta tttgtttggg ctgcgagct               2569

## Description

Investigations reported in this application were supported in part by funding from NIH R 01 GM 63879.

### FIELD OF THE INVENTION

The field of this invention relates to genetically engineering a plant plastid. More specifically, this invention relates to the transformation of non-green plant cells through plastid transformation, and the subsequent regeneration the non-green plant cells through somatic embryogenesis.

### BACKGROUND

Plastids are ideal for genetic engineering because it offers a number of attractive advantages, including high-level transgene expression (Daniell et al., 2002), multi-gene engineering in a single transformation event (DeCosa et al., 2001; Ruiz et al., 2003; Daniell & Dhingra, 2002), transgene containment via maternal inheritance (Daniell 2002), lack of gene silencing (Lee et al., 2003; DeCosa et al., 2001), position effect due to site specific transgene integration (Daniell et al., 2002) and pleiotropic effects (Daniell et al., 2001; Lee et al., 2003). Chloroplast genetic engineering is most suitable for hyper-expression of vaccine antigens and production of valuable therapeutic proteins. Ever since we demonstrated expression of human-elastin derived polymers for various biomedical applications (Guda et al., 2000), we have extended this approach to express vaccines antigens for Cholera, Anthrax (Daniell et al., 2001, Daniell 2003), monoclonal antibody (Daniell et al., 2001) and human therapeutic proteins, including Human Serum Albumin, (Fernandez et al., 2003), Magainin (DeGray et al., 2001), Interferon (Daniell 2003) and Insulin like Growth Factor (Daniell, 2003). Several other laboratories have expressed Human Somatotropin Interferon-GUS fusion proteins to improve stability (Reddy et al., 2003) and tetanus vaccine antigens (Tregoning et al., 2003) in transgenic chloroplasts. Without any exception, all of these therapeutic proteins have been expressed in transgenic tobacco chloroplasts, meeting the zero-tolerance of food crops for plant-derived pharmaceuticals advocated by various environmental groups.

However, there is an urgent need for oral delivery of therapeutic proteins and vaccine antigens to dramatically reduce their production, purification, storage and transportation costs and minimize complications associated with intravenous delivery. Carrot (*Daucus carota* L.) is one of the most important vegetables used worldwide for human and animal consumption, due to its excellent source of sugars, vitamins A, C and fiber in the diet. The carrot plant is biennial, completing its life cycle in two years. In the first year the plant produces the fleshy taproot, which is edible. If left in the ground, plants flower in the second year after passing through a cold season (Yan, W. & Hunt, L.A Reanalysis of Vernalization Data of Wheat and Carrot, Annuals of Botany 84, 615-619 (1999). In addition, chloroplast genomes in the cultivated carrot crop are transmitted strictly through maternal inheritance (Vivek et al 1999). Thus, carrot is environmentally safe and is doubly protected against transgene flow via pollen and seeds to achieve zero-tolerance on transgene flow advocated for food crops. Carrot somatic embryos are single cell derived and multiply through recurrent embryogenesis; this provides uniform source of cell culture, which is one of the essential requirements for producing therapeutic proteins (homogeneous single source of origin). Carrot cells divide rapidly and large biomass is produced using bioreactors. Cultured carrot cells are edible and could be used directly to deliver precise dose of vaccine antigens or biopharmaceuticals. When delivered via edible carrots, there is no need to cook and this would preserve the structural integrity of therapeutic proteins during consumption. Viable for long duration on culture medium, encapsulated embryos are used as synthetic seeds for cryopreservation and controlled germination (Tessereau, H., B. Florin, M. C. Meschine, C. Thierry and V. Pétiard, 1994). Thus, transgenic carrot with enhanced medicinal or nutritional value can play a vital role in improving human or animal health

However, in order to engineer the carrot chloroplast genome, one has to overcome several major hurdles. So far, the chloroplast genome has been transformed only by using green leaves as explants, which contain large chloroplasts. The first challenge is to introduce foreign DNA into small proplastids and identify appropriate regulatory sequences and selectable markers that function in non-green plastids. The second challenge is to regenerate chloroplast transgenic plants via somatic embryogenesis and achieve homoplasmy, which lacks the benefit of subsequent rounds of selection offered by organogenesis, while using leaves as explants. Because of these reasons, chloroplast genetic engineering has been achieved only in a few solanaceous crops other than tobacco, such as tomato (Ruf et al., 2001) and potato (Sidorov et al., 1999), although the later crop remained sterile. Non-solanaceous crops continue to be a challenge to transform, even though regeneration was possible from green leaves via organogenesis. For example, *Arabidopsis* transgenic plants were sterile (Sidkar et al., 1998) and even stable integration or homoplasmy could not be achieved in oilseed rape (Bing Kai Hou et al., 2003).

Khan & Maliga studied plastid transformation in higher plants and tracked plastid transformation by using a fluorescent antibiotic resistance marker (M.S. Khan & P. Maliga, Fluorescent antibiotic resistance marker for tracking plastid, transformation in higher plants, Nature Biotechnology (September 1999) 17:910-915). Khan & Maliga describe the transformation of tobacco by bombarding tobacco leaves with plastid transformation vectors. New tobacco plants were generated from the leaves by organogenesis. Khan & Maliga also describe transformation of rice resulting in heteroplastomic rice plants.

Table 1 shows an exemplary list of the development of transgene expression in chloroplasts.

**TABLE 1**

| Transgene Expression in chloroplasts | | | | | |
|---|---|---|---|---|---|
| **Agronomic traits** | **Gene** | **Promoter** | **5'/3' Regulatory elements** | | **Reference** |
| Insect resistance | CrylA(c) | Prrn | *rbcL* / T*rps*16 | | Mc Bride et al 1995 |
| Herbicide resistance | CP4 (petunia) | Prrn | ggagg / T*psb*A | | Daniell et al 1998 |
| Insect resistance | Cry2Aa2 | Prrn | ggagg (native) / T*psb*A | | Kota et al 1999 |
| Herbicide resistance | CP4 (bacterial or synthetic) | Prrn | *rbc*L or T7 gene 10 / *Tips 16* | | Ye at al 2001 |
| Insect resistance | Cry2Aa2 operon | Prrn | Native 5'UTRs / *TpsbA* | | DeCosa et al 2001 |
| Disease resistance | MSI-99 | Prrn | ggagg / T*psb*A | | DeGray et al 2001 |
| Salt and drought tolerance | *tps* | Prrn | ggagg / T*psb*A | | Lee et al 2003 |
| Phytoremediation | *mer*A^{a}*lmer*B^{b} | Prrn | ggagg^{a,b} / T*psb*A | | Ruiz et al 2003 |
| | | | | | |

| **Biopharmaceutical proteins** | **Gene** | **Promoter** | **5'/3' regulatory elements** | **% tsp expression** | **Reference** |
|---|---|---|---|---|---|
| Protein based polymer | EG121 | Prrn | T7gene10 / *TpsbA* | Not tested | Guda et al 2000 |
| Human somatotropin | *hST* | Prrn^{a}, P*psb*A^{b} | T7gene10^{a} or *psb*A^{b} / T*ips*16 | 7.0%^{a} and 1.0%^{b} | Staub et al 2000 |
| Cholera toxin | *ctx*B | Prrn | ggagg / *TpsbA* | 4% | Daniell et al 2002 |
| Tetanus toxin | T*et*C (bacterial and synthetic) | Prrn | T7 gene 10^{a}, *atp*B^{b} / *TrbcL* | 25%^{a}, 10% | ^{b} Tregoning et al 2003 |
| Human Serum Albumin | *hsa* | Prrn^{a}, P*psb*A^{b} | ggagg^{a}, *psb*A^{b} / T*psb*A | 0.02%^{a}, 11.1%^{b} | Fernandez-San Milan et al 2003 |
| Interferon alpha 5 | INF□5 | Prrn | P*psb*A/T*psb*A | ND | Torres |
| Interferon alpha 2B | INF□2B | Prrn | P*psb*A/T*psb*A | 19% | Falconer |
| Interferon gamma | *ifn-g* | P*psb*A | P*psb*A*l*T*psb*A | 6% | Leelavathi and Reddy, 2003 |
| Monoclonal antibodies | | Prrn | ggagg/T*psb*A | ND | Daniell et al (photosynthesi s) |
| Insulin like growth factor | *Igf-*1 | Prrn | P*p*s*b*A/T*psb*A | 33% | Ruiz G |
| Anthrax protective antigen | Pag | Prrn | P*psb*A/T*psb*A | 4-5% | Watson |
| Plague vaccine | *Ca*F1-*Lcr* V | Prrn | P*psb*A/T*psb*A | 4.6 % | Singleton |

### SUMMARY OF THE INVENTION

One aspect of this invention describes a homoplasmic somatic embryo of a non-green carrot cell transformed with a plastid transformation vector comprising, as operably linked components, a first flanking sequence, a 5' regulatory sequence comprising a Prrn 16S rRNA promoter functional in proplastids and chloroplasts in light and in dark, a DNA sequence coding for a foreign gene, a DNA sequence encoding a selectable marker, and a second flanking sequence, wherein said vector facilitates stable integration into a carrot plastid genome, and wherein said first and second flanking sequence are substantially homologous to sequences in a spacer region of said plastid genome. Another aspect of this invention provides for a method of producing such a homoplasmic somatic embryo of a non-green carrot cell by somatic embryogenesis comprising the step of integrating a plastid transformation vector comprising, as operably linked components, a first flanking sequence, a 5' regulatory sequence comprising a Prrn 16S rRNA promoter functional in proplastids and chloroplasts in light and in dark, a DNA sequence coding for a foreign gene, a DNA sequence encoding a selectable marker, and a second flanking sequence, into a plastid genome of a carrot plastid. Still another aspect provides for a method of transforming a plastid through somatic embryogenesis comprising the steps of: integrating a plastid transformation vector comprising, as operably linked components, a first flanking sequence, a 5' regulatory sequence comprising a Prrn 16S rRNA promoter functional in proplastids and chloroplasts in light and in dark, a DNA sequence coding for a foreign gene, a DNA sequence encoding a selectable marker, and a second flanking sequence, into a plastid genome of a non-green carrot cell plastid, wherein said first and second flanking sequence are substantially homologous to sequences in a spacer region of said plastid genome. This application, along with the knowledge of the art, describes the engineering of the plastid genome of several major crops in which regeneration is mediated through somatic embryogenesis. Cereal crops (wheat, rice, corn, sugarcane), legumes (soybean, alfalfa), oil crops (sunflower, olive), cash crops (cotton, coffee, tea, rubber, flex, cork oak, pines), vegetables (eggplant, cucumber, cassava, chili pepper, asparagus etc.), fruits (apple, cherry, banana, plantain, melons, grape, guava), nuts (cashew, walnuts, peanuts), and trees (date palm etc.) are regenerated through somatic embryogenesis. Also described herein are constructs of chloroplast vectors for a variety of different species using the same primers (universal plastid primers).

### BRIEF DESCRIPTION OF THE FIGURES

Fig. 1 (A-B). shows the physical map of the carrot chloroplast transformation vectors.
Fig 1(A) shows carrot chloroplast transformation vector *pDD-Dc-gfplBADH* carries the *gfp* and *BADH* genes expressed under the regulation of T7 gene 10 5' untranslated region (UTR)/ *rps* 16 3'UTR and P*psb*A 5' and 3' UTR respectively. The Prrn promoter of 16S r-RNA gene, having both PEP and NEP recognition sites, drives expression of the cassette.
Fig 1(B) shows the carrot chloroplast transformation vector pDD-Dc-*aad*A/*BADH* carries the *aadA* and *BADH* gene. Expression of *aadA* gene is under the regulation of Shine-Dalgarno sequence and *psbA* 3'UTR while that of *BADH* is regulated by gene10 5' and *rps* 16 3'UTR. AflIII/PvuII digested ~ 4.9 kb DNA fragment used as a probe for Southern analysis of the transgenic plants and landing sites for primers 3P/3M and 16SF/1M used to confirm the presence of the transgene integration into carrot plastids are shown.
Fig. 2(A-C) shows the expression of GFP in carrot cultures transformed with chloroplast vector pDD*-Dc-gfplBADH;* visible under confocal fluorescent microscope at fluorescence emission in green at 488nm blue Argon (laser).
Fig. 2(A) shows the untransformed control carrot culture,
Fig. 2(B) shows the transformed embryogenic calli,
Fig.2(C) shows the transformed embryogenic carrot calli differentiated into globular somatic embryos and
Fig. 2(D) shows a somatic embryo differentiated into cotyledonary stage.
Fig. 3 (A-D) shows the visual selection of green transgenic cells versus yellow non-transgenic carrot cells culture.

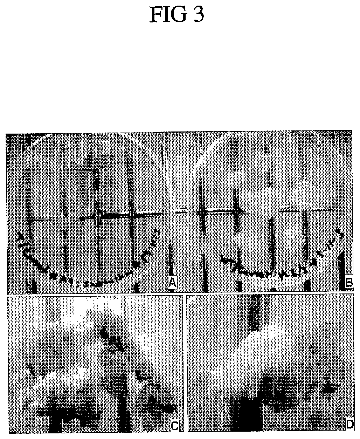

Fig. 3(A-B) shows the transgenic carrot cell culture turned green due to the expression BADH (Plate A) while wild type culture remained yellow (Plate B). Transgenic carrot cell culture can be distinguished as green-transgenic cell culture vs yellow non-transgenic carrot cell culture, when heteroplasmic transgenic cell line was placed on medium without any selection agent (Plate C and D).

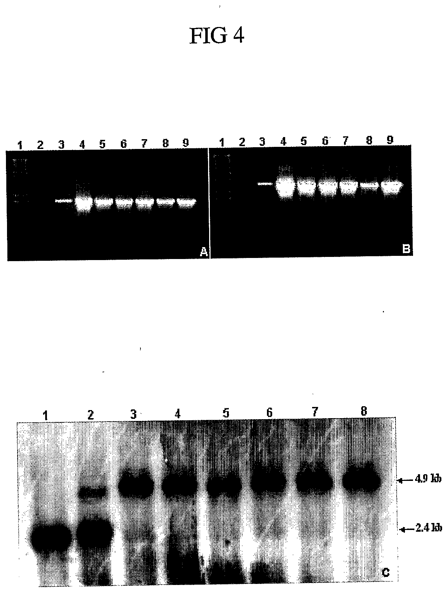

Fig. 4 (A-C) shows the transgene (*aadA* and *badh*) integration into the carrot plastid genome was confirmed by PCR and Southern blot analysis.
Fig 4(A) shows the use of internal primers 3P (land on flanking sequence) and 3M (land on *aadA* gene) ~1.65 kb size PCR product was amplified at 64°C annealing temperature, confirmed transgene integration into plant cell lines.

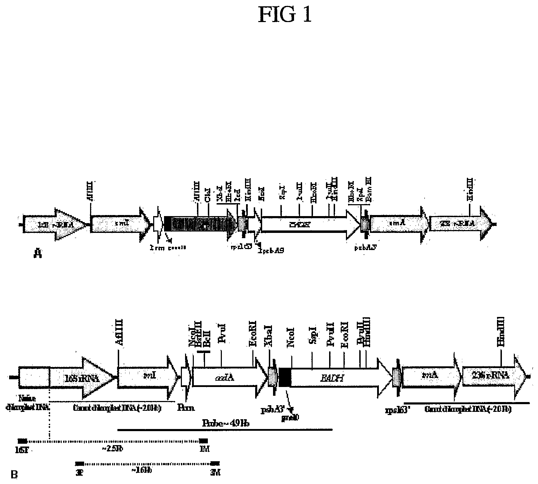

Fig. 4(B) shows the use of a set of primer 16SF (landing on the native chloroplast genome) and 1M (landing on the *aadA* gene) yield ~ 2.5 kb size PCR product at 64°C annealing temperature, confirmed plastid specific integration of the transgenes. Lane 2 stand for DNA from non-transgenic carrot cells and lanes 2-9 represents DNA from seven transgenic carrot cell lines. Primers landing sites for primer pairs (3P/3M and 16SF/1M) is shown in Fig. 1B.
Fig 4(C) shows the southern blot analysis of plastid genome of untransformed and transformed carrot with vector pDD*-Dc-aadAlBADH.* Carrot genomic DNA (5 µg per lane) digested with AflIII and PvuII and transferred to nitrocellulose membrane, was hybridized with the 4.9 kb radioactive labeled P³² DNA probe (containing 2.4 kb flanking sequence and 2.5 kb transgene sequence, see Fig. 1B). Lane 1, control DNA from untransformed transgenic plant showed 2.4 kb size band while heteroplasmic transgenic plant from cell line one, lane 2 showed both bands. Homoplasmy in transgenics plants from different cell lines (lanes 3-8) was achieved by repetitive subcultures of transgenic cells in liquid medium.

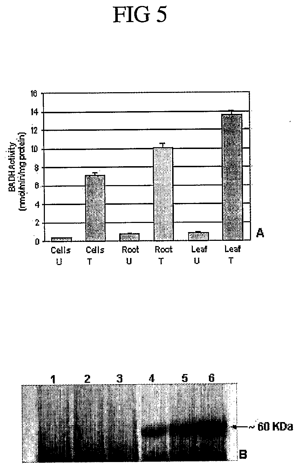

Fig. 5 (A-B) shows BADH enzyme activity (nmol/min/mg/protein) and BADH expression was analyzed in protein extracts from untransformed and transformed carrot with plastid vector pDD*-Dc-aadAlBADH.*
Fig 5(A) shows the reduction of NAD⁺ dependent BADH enzyme was analyzed for the formation of NADH at 340 nm in presence of betaine aldehyde. Very low BADH activity was detected in untransformed cells suspension (U), carrot root (U) and leaf (U). On the other hand, high BADH activity was recorded about 54 % in transformed carrot cells suspension (T), about 72% in root (T) in comparison to leaf (T) tissues.
Fig. 5(B) shows that the BADH expression was analyzed by western blot. Whole cell extracts from transformed and untransformed carrot cell culture, root and leaf tissues were prepared and 50 µg total soluble protein from each sample was run on 10% SDS-PAGE and protein transferred to Immuno-blot™ PVDF membrane and hybridized with polyclonal anti-BADH serum, raised in rabbits against native BADH. Antigenic peptides were detected using horseradish peroxidase-linked secondary antibody. Lane 1, 2, 3 contain whole carrot extract from untransformed cell culture, root and leaf and lane 4, 5, 6 contain whole carrot extract from transformed cell culture, root and leaf. Transgenic cells expresses about 50 % less (lane 4), root about 25 % less (lane 5) BADH protein than leaf (lane 6).

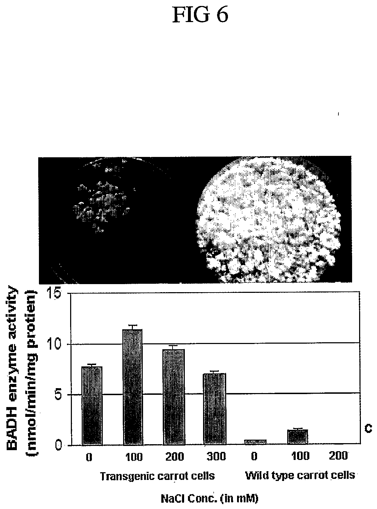

Fig. 6 (A-C) shows the effect of different salt concentrations on growth of untransformed and transformed carrot cell suspension cultures with chloroplast vector pDD*-Dc-aadAlBADH.*
Fig. 6(A) shows the dry mass in untransformed carrot cell culture.
Fig. 6(B) shows transformed cell cultures produced in liquid medium containing 100 mM NaCl.
Fig. 6(C) shows the stimulation of BADH activity in presence of salt. Untransformed and transformed carrot cells in suspension cultures were placed on shaker at 130 rpm speed for two weeks in liquid medium containing 0, 100, 200 and 300 mM NaCl. Elevated level of BADH activity in transgenic cell cultures was noticed when liquid growth medium containing 100 and 200 mM NaCl.

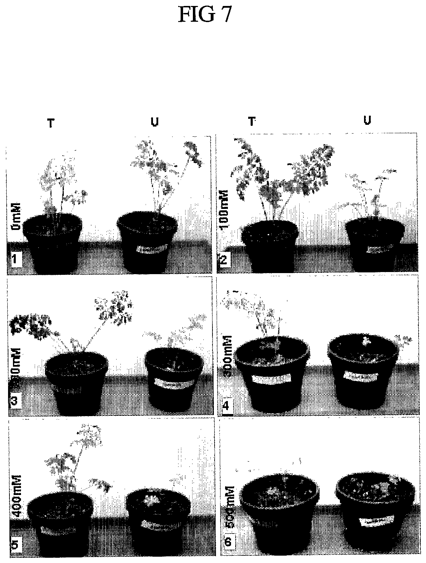

Fig. 7 Effect of salt (100-500 mM NaCl) on untransformed (U) and transformed (T) carrot plants. Transgenic plants were tested for one month on different concentration of NaCl. Plants were irrigated with water containing different concentrations NaCl at alternative days up to four weeks.

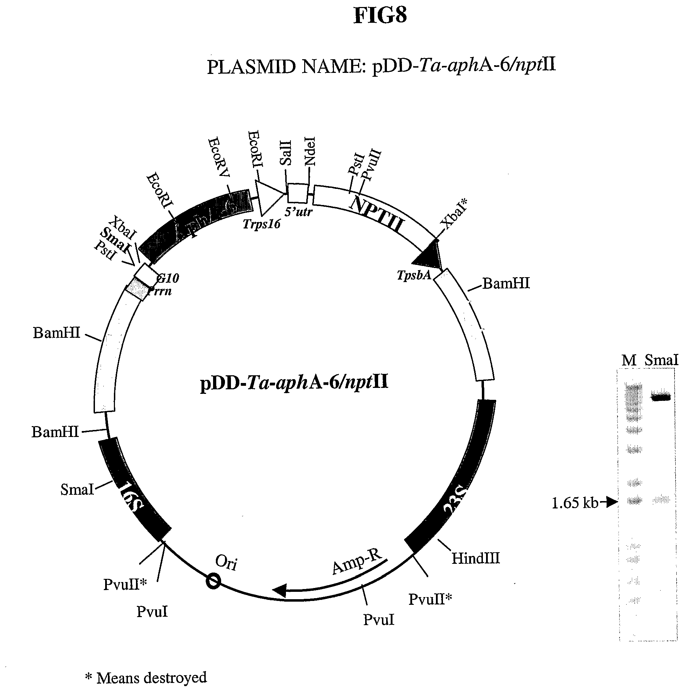

Fig. 8 shows the plasmid pDD-Ta-aphA-6/nptII. More particularly the plasmid illustrates pDA-76 (aphA-6/nptII expression cassette), having a backbone vector pBluescript II KS, a selectable marker/host cell Ampicillin/Kan/ XL-1 Blue MRF' Tc, and a flanking region from Triticum aestivum (Ta).

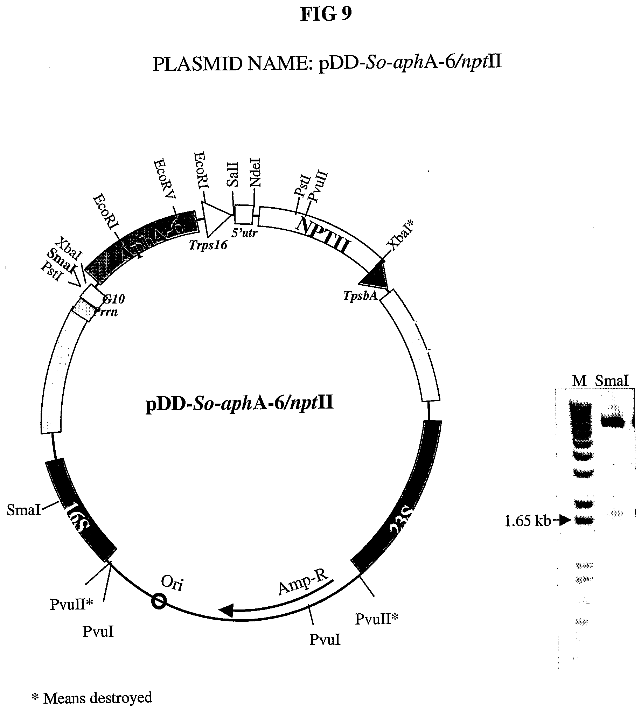

Fig 9 shows the plasmid pDD-So-aphA-6/nptII. More particularly the plasmid illustrates pDA-76 (aphA-6/nptII expression cassette), having a backbone vector pBluescript II KS, a selectable marker/host cell Ampicillin/Kan/ XL-1 Blue MRF' Tc, and a flanking region from Saccharum officinarum (So).

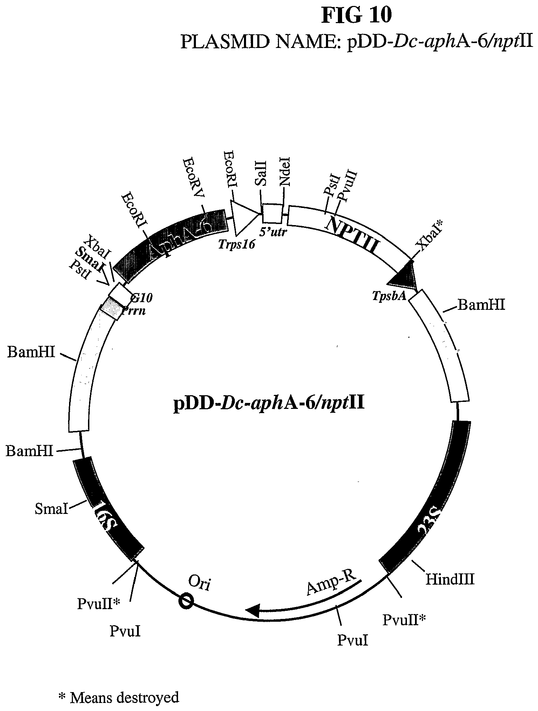

Fig 10 shows the plasmid pDD-Dc-aphA-6/nptII. More particularly the plasmid illustrates pDA-76 (aphA-6/nptII expression cassette), having a backbone vector pBluescript II KS, a selectable marker/host cell Ampicillin/Kan/ XL-1 Blue MRF' Tc, and a flanking region from *Daucus carota* (Dc).

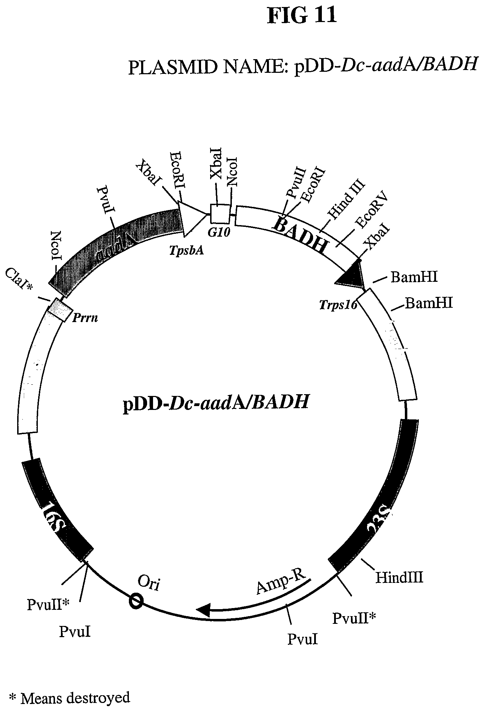

Fig 11 shows the plasmid pDD-Dc-aadA/BADH. More particularly the plasmid illustrates pDA-29 (aadA/BADH expression cassette) having a backbone vector pBluescript II KS, a selectable marker/host cell Ampicillin/spectinomycinXL-1 Blue MRF' Tc, and a flanking region from *Daucus carota* (Dc).

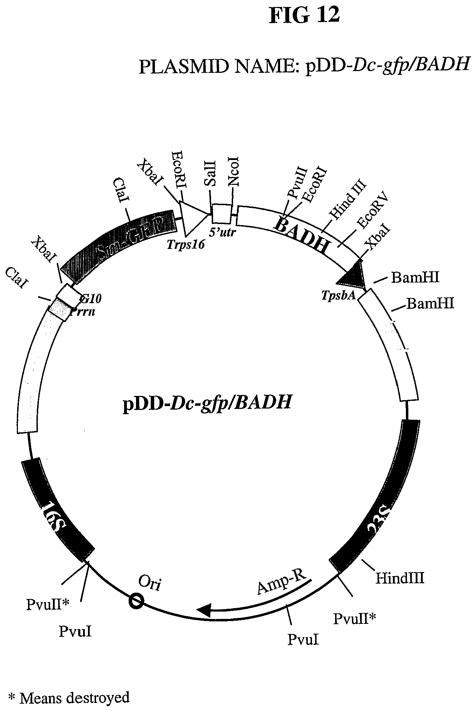

Fig. 12 shows the plasmid pDD-Dc-gfp/BADH. More particularly the plasmid pDA-30 (gfp/BADH expression cassette), having a backbone vector pBluescript II KS, a selectable marker/host cell Ampicillin/Kan/ XL-1 Blue MRF' Tc, and a flanking region from *Daucus carota* (Dc).

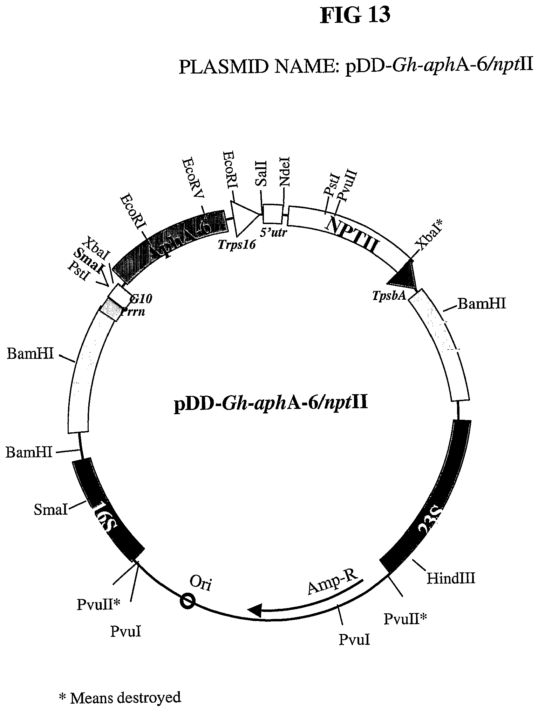

Fig. 13 shows the plasmid pDD-Gh-aphA-6/nptII. More particularly the plasmid illustrates pDA-76 (aphA-6/nptII expression cassette), having a backbone vector pBluescript II KS, a selectable marker/host cell Ampicillin/Kan/ XL-1 Blue MRF' Tc, and a flanking region from Gossypium hirsutum (Gh).

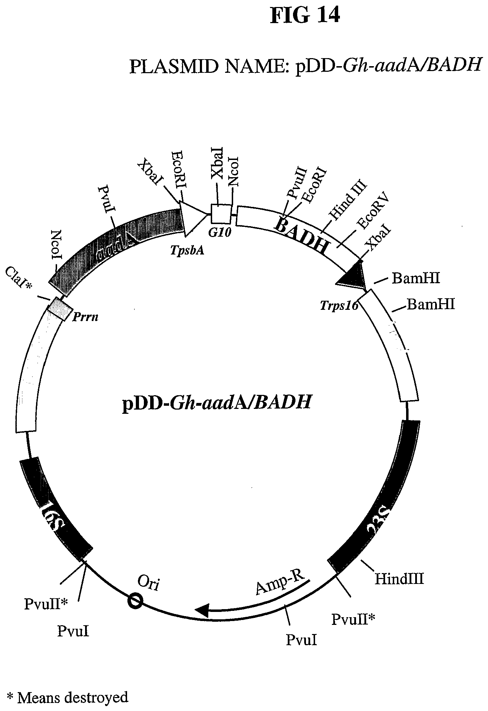

Fig. 14 shows the plasmid pDD-Gh-aadA/BADH. More particularly the plasmid illustrates pDA-29 (aadA/BADH expression cassette), having a backbone vector pBluescript II KS, a selectable marker/host cell Ampicillin/spectinomycinXL-1 Blue MRF' Tc and a flanking region from Gossypium hirsutum (Gh).

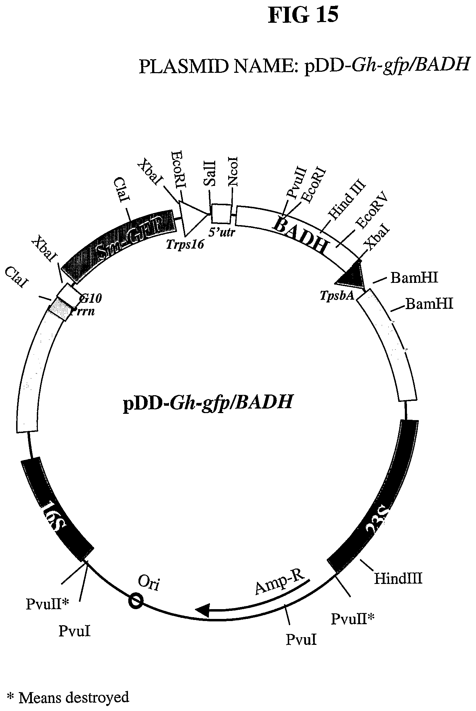

Fig. 15 shows the plasmid pDD-Gh-gfp/BADH. More particularly the plasmid illustrates pDA-30 (gfp/BADH expression cassette), having a backbone vector pBluescript II KS, a selectable marker/host cell Ampicillin/XL-1 Blue MRF' Tc, and a flanking region from Gossypium hirsutum (Gh).

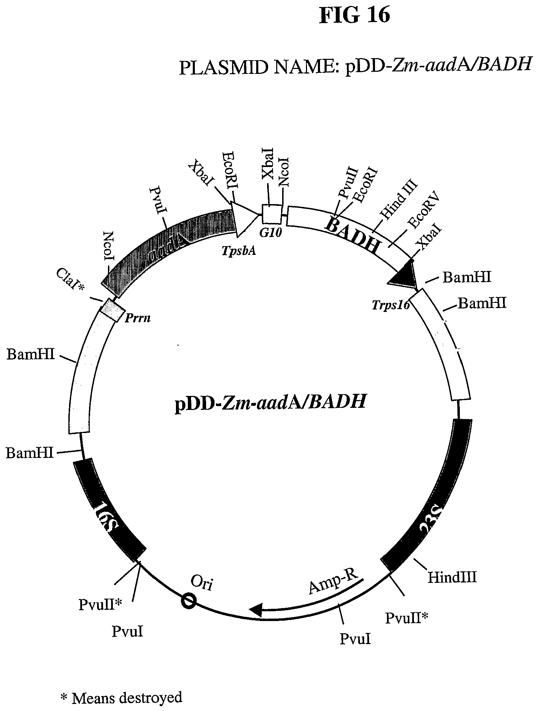

Fig. 16 shows the plasmid pDD-Zm-aadA/BADH. More particularly the plasmid illustrates pDA-29 (aadA/BADH expression cassette), having a backbone vector pBluescript II KS, a selectable marker/host cell Ampicillin/spectinomycinXL-1 Blue MRF' Tc, and a flanking region from Zea mays (Zm).

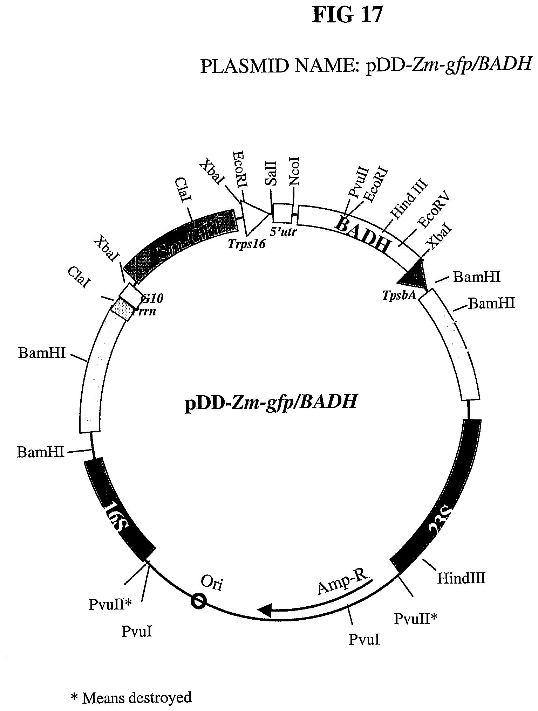

Fig. 17 shows the plasmid pDD-Zm-gfp/BADH. More particularly the plasmid illustrates pDA-30 (gfp/BADH expression cassette), having a backbone vector pBluescript II KS, a selectable marker/host cell Ampicillin/XL-1 Blue MRF' Tc, and a flanking region from Zea mays (Zm).

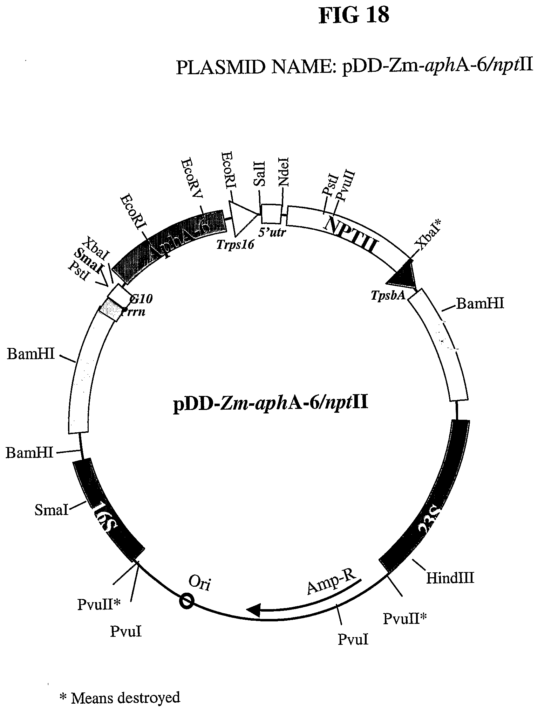

Fig. 18 shows the plasmid pDD-Zm-aphA-6/nptII. More particularly the plasmid illustrates pDA-76 (aphA-6/nptII expression cassette), having a backbone vector pBluescript II KS, a selectable marker/host cell Ampicillin/Kan/ XL-1 Blue MRF' Tc, and a flanking region from *Zea mays* (Zm).

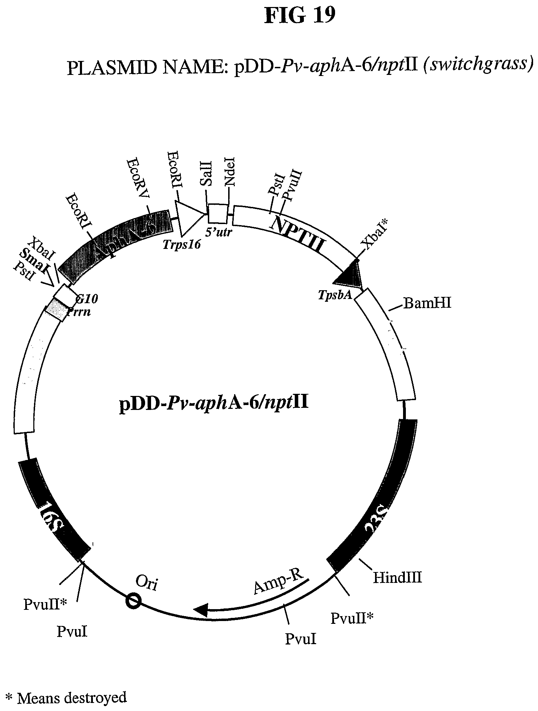

Fig. 19 shows the plasmid pDD-Pv-aphA-6/nptII (switchgrass). More particularly the plasmid illustrates pDA-76 (aphA-6/nptII expression cassette), having a backbone vector pBluescript II KS, a selectable marker/host cell Ampicillin/Kan/ XL-1 Blue MRF' Tc, and a flanking region from *Panicum virgatum* (Pv).

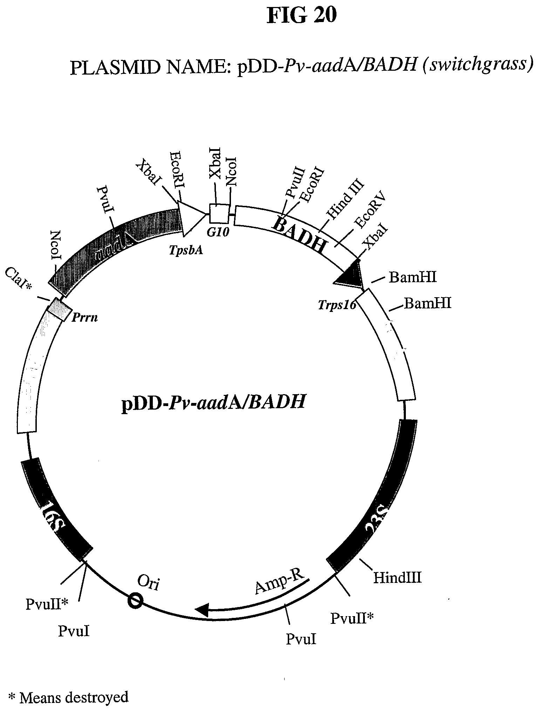

Fig. 20 shows the plasmid pDD-Pv-aadA/BADH (switchgrass). More particularly the plasmid illustrates pDA-29 (aadA/BADH expression cassette), having a backbone vector pBluescript II KS, a selectable marker/host cell : Ampicillin/spectinomycinXL-1 Blue MRF' Tc, and a flanking region from *Panicum virgatum* (Pv).

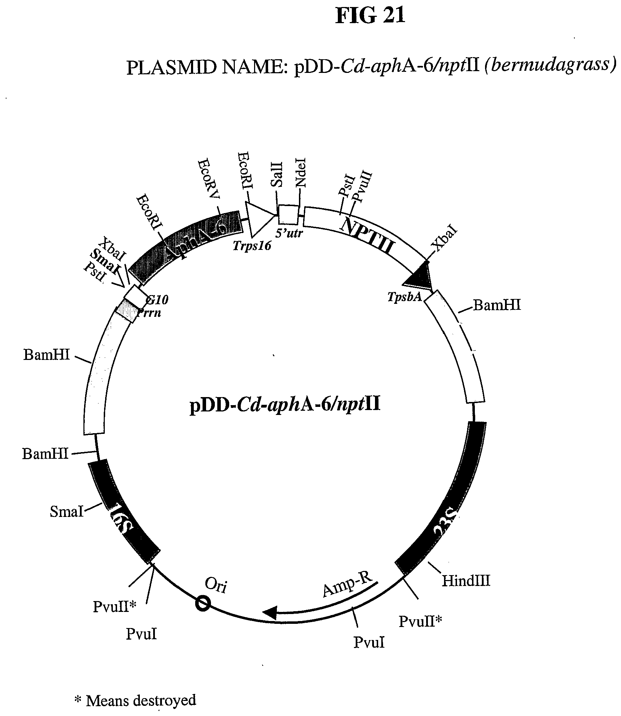

Fig.21 shows the plasmid pDD-Cd-aphA-6/nptII (bermudagrass). More particularly the plasmid illustrates pDA-76 (aphA-6/nptII expression cassette), having a backbone vector pBluescript II KS, a selectable marker/host cell: Ampicillin/Kan/ XL-1 Blue MRF' Tc, and a flanking region from *Cynodon dactylon* (Cd).

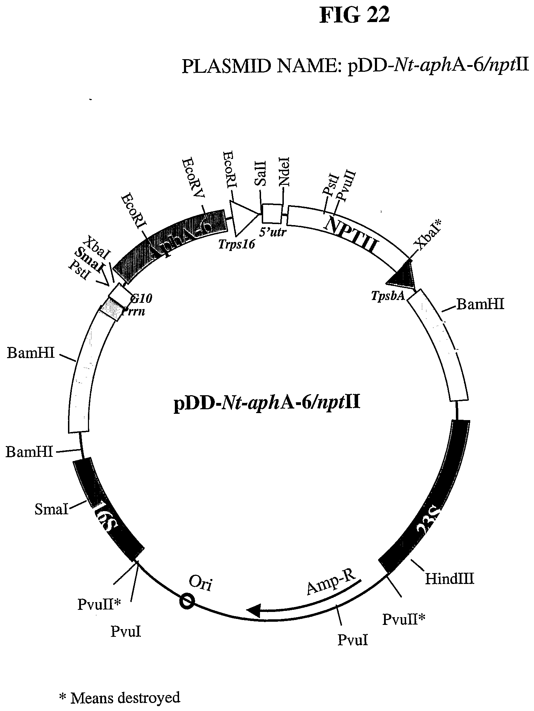

Fig. 22 shows the plasmid pDD-Nt-aphA-6/nptII. More particularly the plasmid illustrates pDA-76 (aphA-6/nptII expression cassette), having a backbone vector pBluescript II KS, a selectable marker/host cell Ampicillin/Kan/ XL-1 Blue MRF' Tc, and a flanking region from *Nicotiana tabacum* (Nt).

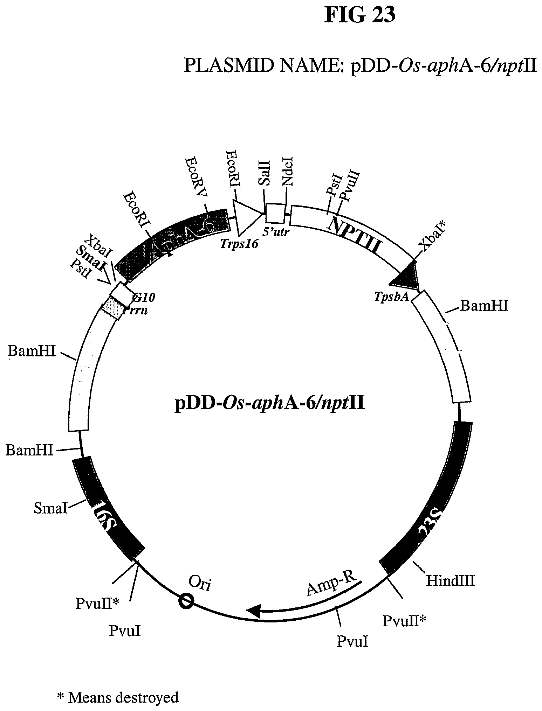

Fig. 23 shows the plasmid pDD-Os-aphA-6/nptII. More particularly the plasmid illustrates pDA-76 (aphA-6/nptII expression cassette), having a backbone vector pBluescript II KS, a selectable marker/host cell Ampicillin/Kan/ XL-1 Blue MRF' Tc, and a flanking region from *Oryza sativa* (Os).

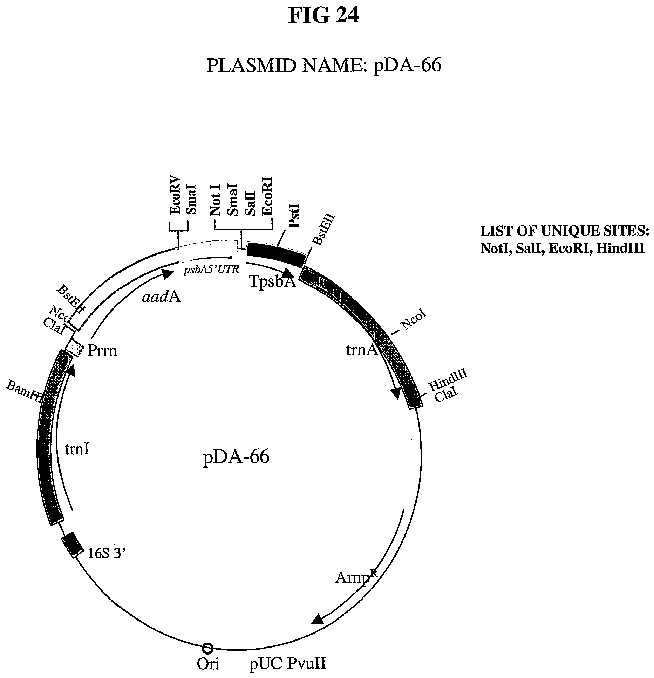

Fig. 24 shows the plasmid pDA-66. More particularly the plasmid illustrates psbA 5'UTR BACKBONE VECTOR pUC 19, which is a Derivative of pLD-CtV basic vector (modified MCS) having a selectable marker/host cell Ampicillin/Kan/ XL-1 Blue MRF' Tc, and a flanking region from Tobacco.

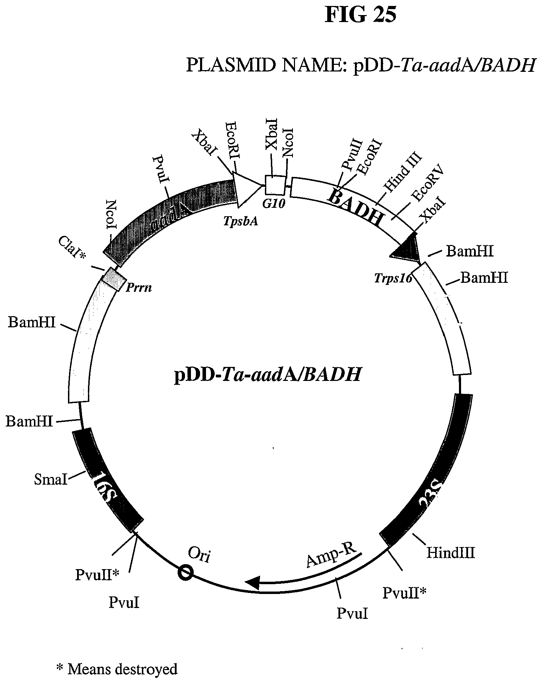

Fig. 25 shows the plasmid pDD-Ta-aadA/BADH. More particularly the plasmid illustrates pDA-29 (aadA/BADH expression cassette), having a backbone vector pBluescript II KS, a selectable marker/host cell Ampicillin/spectinomycinXL-1 Blue MRF' Tc, and a flanking region from *Triticum aestivum (Ta).*

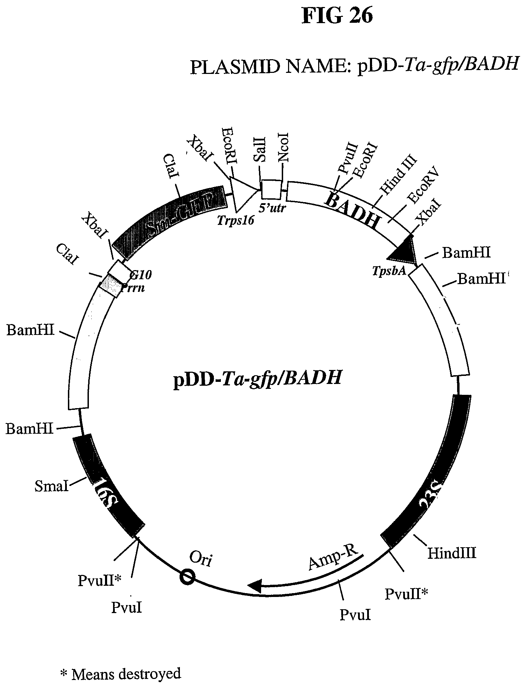

Fig. 26 shows the plasmid pDD-Ta-gfp/BADH. More particularly the plasmid illustrates pDA-30 (gfp/BADH expression cassette), having a backbone vector pBluescript II KS, a selectable marker/host cell Ampicillin/XL-1 Blue MRF' Tc, and a flanking region from *Triticum aestivum* (Ta).

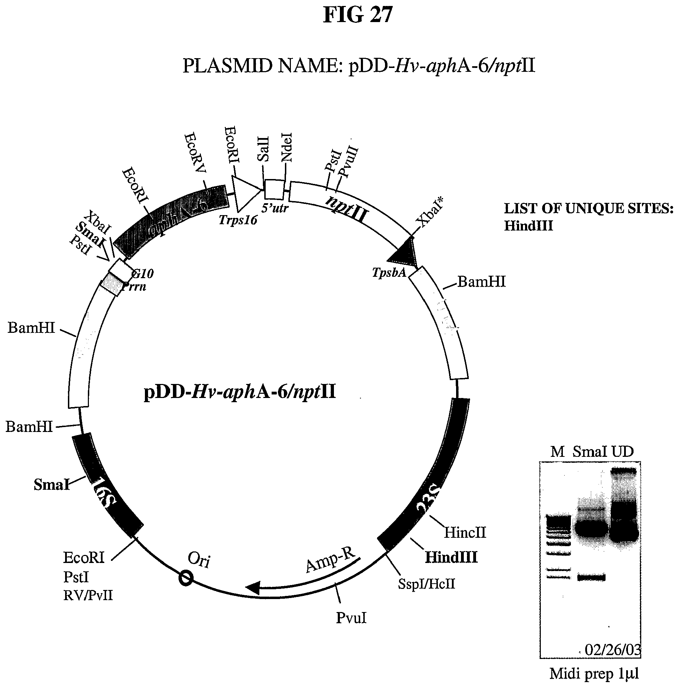

Fig. 27 shows the plasmid pDD-Hv-aphA-6/nptII. More particularly the plasmid illustrates pDA-76 (aphA-6/nptII expression cassette), having a backbone vector pBluescript II KS, a selectable marker/host cell Ampicillin/Kan/ XL-1 Blue MRF' Tc, and a flanking region from *Hordeum vulgare (Hv).*

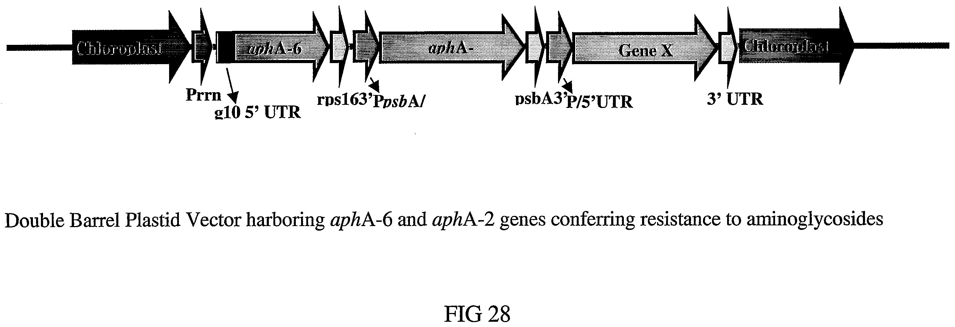

Fig. 28 is a schematic view of a Double Barreled Plastid Vector harboring aphA-6 and aphA-2 genes conferring resistance to aminoglycosides according to the description contained herein.

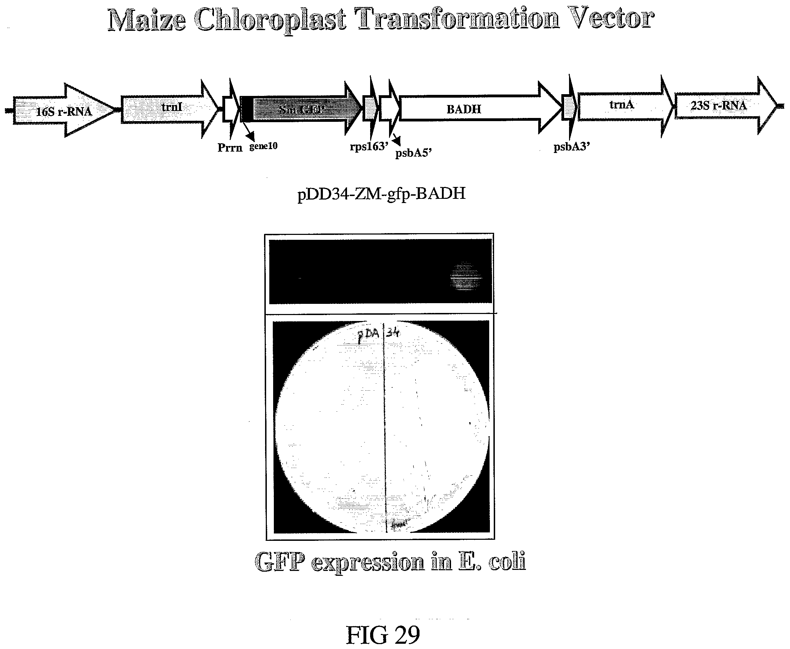

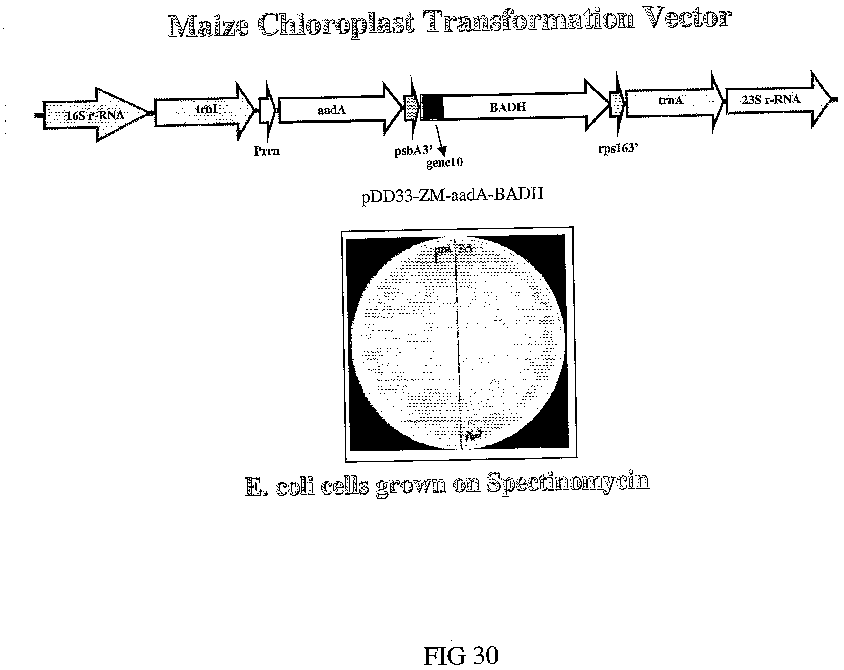

Fig. 29A and 30A illustrate the construction of maize chloroplast transformation vector, where flanking regions were amplified using PCR. The PCR products were cloned and the expression cassette was inserted in the transcriptionally active spacer region between trnI/trnA genes. The expression cassette of Fig. 30A has the Prrn promoter driving the expression of GFP and BADH, which are regulated by (5') gene10/rps16 3' and psbA 5'/3' UTRs respectively. The expression cassette of 31A has the Prrn promoter driving the expression of aadA and BADH. The latter gene is regulated by (5') gene10/rps16 3' UTRs.

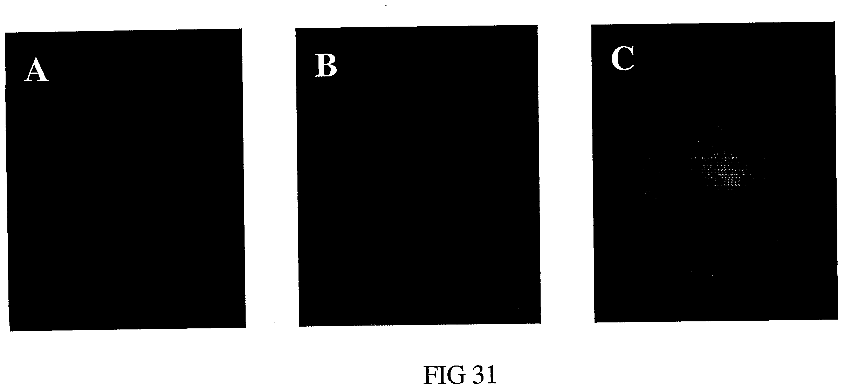

Figs. 29B and 30B shows the functions of the genes in the maize chloroplast transformation vectors which were tested in E. coli. For observing GFP expression, cells were plated on LB agar (Amp) plates and incubated at 37°C overnight. Cells harboring pDD34-ZM-GFP-BADH were seen to fluoresce when exposed to UV light, as is seen in Fig 30B. To test the aadA gene expression, cells harboring pDD33-ZM-aadA-BADH plasmid were plated on LB agar plates containing spectinomycin (100mg/ml) and incubated at 37°C overnight. Transformed cells grow on spectinomycin, as can be seen in Fig. 31B.

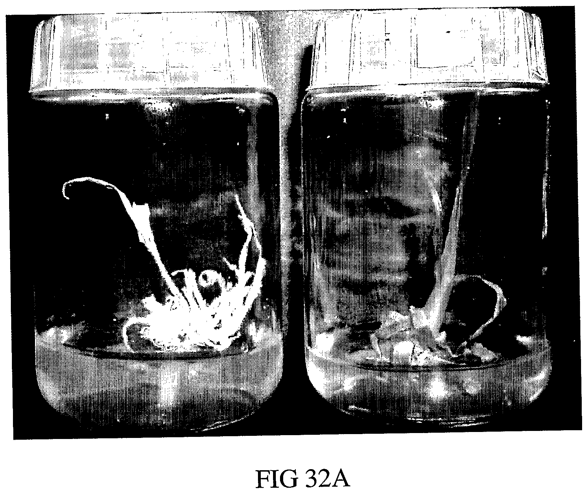

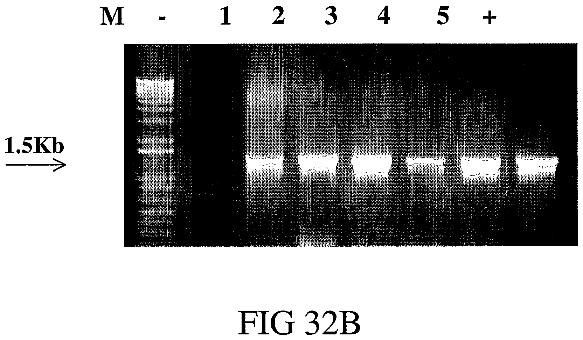

Fig. 31 shows GFP expression in embryogenic maize cultures studied under the confocal microscope. Fig. 32A is a non-transgenic control, while Figs. 32B-C are transformed maize embryogenic calli. The selection in Figs. 30-31 was initiated two days after bombardment by transferring the bombarded calli to callus induction medium containing BA or streptomycin. After eight weeks, a number of the healthy growing calli from different bombardment experiments were examined for GFP expression under the fluorescent stereomicroscope and the confocal microscope. Somatic embryos were regenerated on maize regeneration medium containing BA or streptomycin.
Figs. 32(A-B) shows maize plants on regeneration medium containing streptomycin or betaine aldehyde. Fig. 32A illustrates maize chloroplast transgenic plants which were capable of growth on the selection agent indicating that construction of transgenic maize, while untransfomed maize plants did not grow on the selection medium.
Fig 32B shows PCR confirmation of chloroplast transgenic plants using appropriate primers. Lanes 1-3, plants transformed with pDD34-ZM-gfp-BADH and Lanes 4-5, plants transformed with pDD33-ZM-aadA-BADH. Lanes - and + represent the negative and positive controls respectively. Genomic DNA was isolated from the leaf tissues and PCR was performed on transformed and non-transformed tissues using appropriate primers.

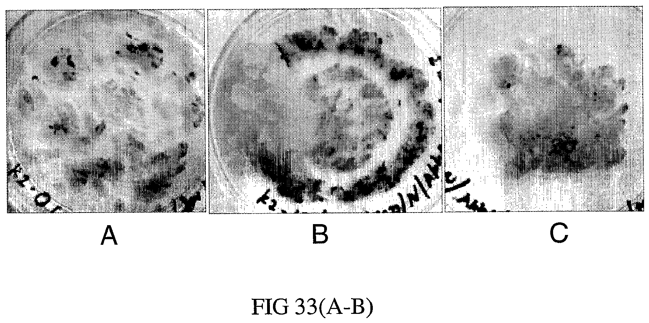

Fig. 33(A-C) shows the Transformed cotton cultures (Gossypium hirsutum cv. Coker310FR) with chloroplast vector pDD-C-aphA6/aphA2; selected on medium MST1 (0.1 mg/12,4-D and 0.5 mg/l kinetin) supplemented with 50 mg/l kanamycin.
Fig. 33(A) shows the untransformed control cotton calli.
Fig. 33(B) shows the transformed primary cotton calli,
Fig. 33(C) shows the transformed cotton calli subcultured from transgenic primary cotton calli (Plate B).

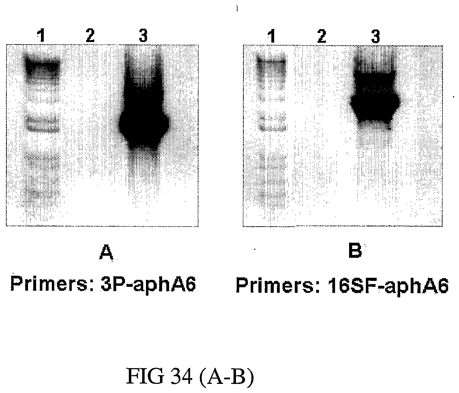

Fig 34 (A-B) show the transgene (aphA6 and aphA2) integration into the cotton plastid genome was confirmed by PCR.
Fig. 34(A) shows the use of internal primers 3P (land on flanking sequence) and aphA6-rev (land on aphA6 gene) ~1.7 kb size PCR product was amplified at 64°C annealing temperature, confirmed transgene integration into cotton calli.
Fig 34(B) shows the use of a set of primer 16SF (landing on the native chloroplast genome) and aphA6-rev (landing on the aphA6 gene) yield ~ 2.5 kb size PCR product at 64°C annealing temperature, confirmed plastid specific integration of the transgenes. Lane 1 represents the 1kb Plus molecular marker (ladder). Lane 2 stand for DNA from non-transgenic cotton calli and lane 3 represents DNA from transgenic cotton calli selected on 50 mg/l kanamycin.

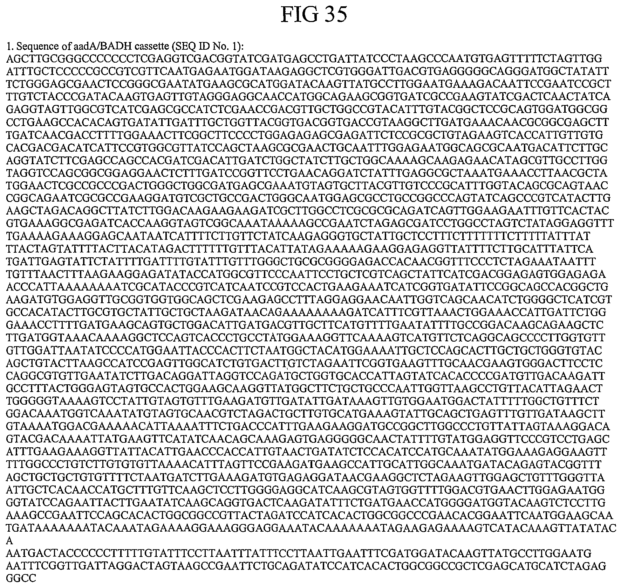

Fig. 35 shows the sequence of the aadA/BADH expression cassette (SEQ ID No. 1).

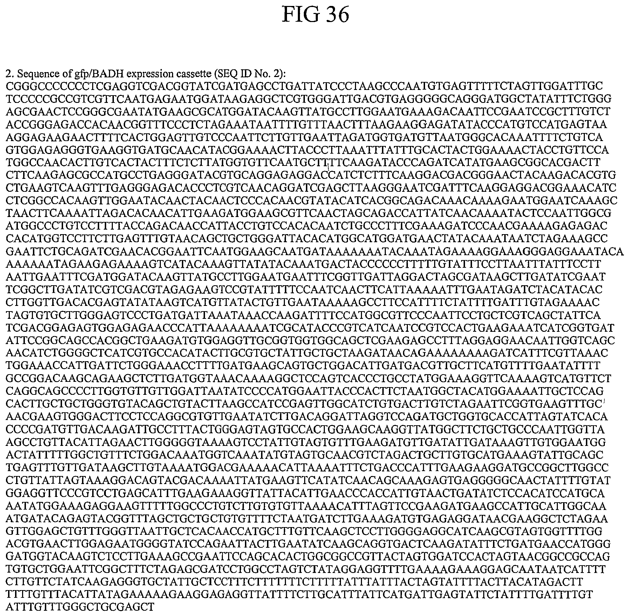

Fig. 36 shows the sequence of the gfp/BADH expression cassette (SEQ ID No. 2).

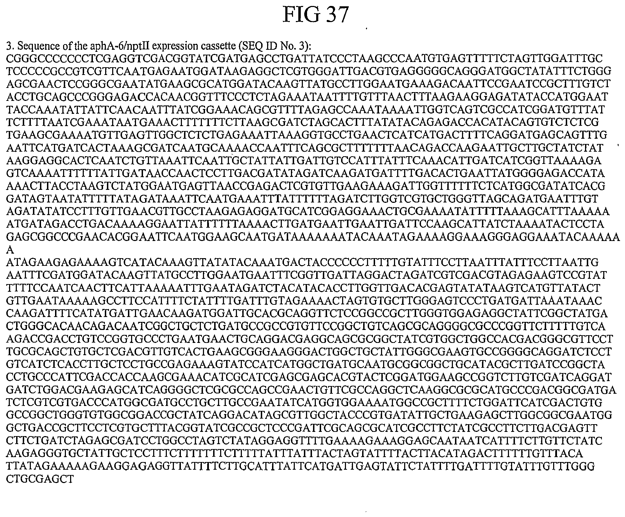

Fig. 37 shows the sequence of the aphA-6/nptII expression cassette (SEQ ID No. 3).

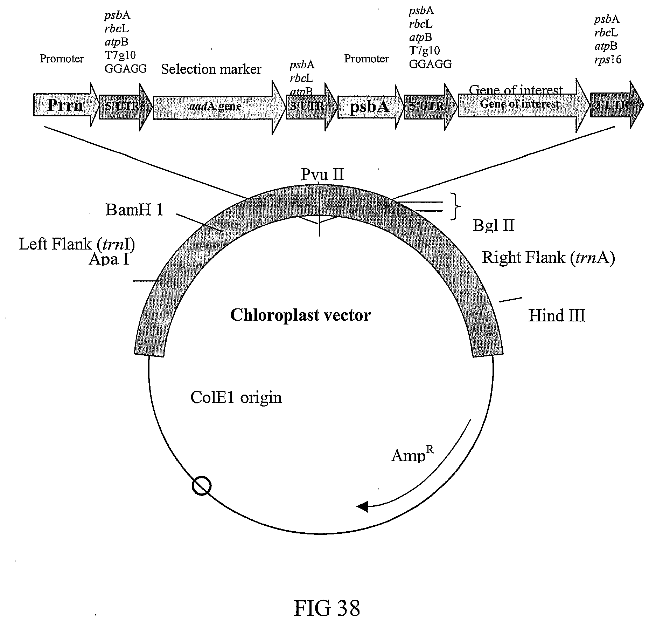

Fig. 38 shows a schematic view of a general plastid transformation vector.

### DETAILED DESCRIPTION OF THE INVENTION

In one aspect of the invention a homoplasmic somatic embryo of a non-green carrot cell is provided that is transformed with a plastid transformation vector comprising, as operably linked components, a first flanking sequence, a 5' regulatory sequence comprising a Prrn 16S rRNA promoter functional in proplastids and chloroplasts in light and in dark, a DNA sequence coding for a foreign gene, a DNA sequence encoding a selectable marker, and a second flanking sequence, wherein said vector facilitates stable integration into a carrot plastid genome, and wherein said first and second flanking sequence are substantially homologous to sequences in a spacer region of said plastid genome. Another aspect of this invention is a method of producing such a homoplasmic somatic embryo of a non-green carrot cell by somatic embryogenesis comprising the step of integrating a plastid transformation vector comprising, as operably linked components, a first flanking sequence, a 5' regulatory sequence comprising a Prrn 16S rRNA promoter functional in proplastids and chloroplasts in light and in dark, a DNA sequence coding for a foreign gene, a DNA sequence encoding a selectable marker, and a second flanking sequence, into a plastid genome of a carrot plastid. Another aspect of this invention provides for a method of transforming a plastid through somatic embryogenesis comprising the steps of: integrating a plastid transformation vector comprising, as operably linked components, a first flanking sequence, a 5' regulatory sequence comprising a Prrn 16S rRNA promoter functional in proplastids and chloroplasts in light and in dark, a DNA sequence coding for a foreign gene, a DNA sequence encoding a selectable marker, and a second flanking sequence, into a plastid genome of a non-green carrot cell plastid, wherein said first and second flanking sequence are substantially homologous to sequences in a spacer region of said plastid genome.

Also described herein are plastid transformation vectors capable for use in non-green explants which can lead to somatic embryogenesis of the plant cells. Also described herein is transgene expression in non-green edible plant parts. Further described herein are transformation of monocots, legumes, vegetables, fruit crops, and transgene expression within the non-green plant parts of these plants. Also described herein is the expression of heterologous proteins using a plastid transformation vector suitable for transforming the non-green plant parts. Further described herein are methods of transforming plastid genomes to express, via somatic embryogenesis, heterologous proteins. Further described herein are transformed plants and progeny thereof, which express the protein of interest. Also described herein is the introduction of foreign DNA into the small proplastids of plants, and the identification of selectable markers which function in non-green plastids. Further described herein are regenerated chloroplast transgenic plants via somatic embryogenesis to achieve homoplasmy.

The preferred aspects of this application are applicable to all plastids of carrot plants. These plastids include the chromoplasts, which are present in the fruits, vegetables, and flowers; amyloplasts; proplastids in the roots of carrot plants; leucoplasts and etioplasts (which express in the dark), both of which are present in the non-green parts of carrot plants. The aspects of this application are also applicable to various developmental stages of chloroplast, wherein the chloroplast are not fully green.

### Definitions

To better understand the current disclosure, the following definitions, which shall hold their meaning throughout this application unless otherwise noted, are provided to put the application in proper context.

Heterologous generally means derived from a separate genetic source. Of course this invention contemplates the use of heterologous and homologous DNA, as well as operons suitable for expression in plant plastids.

An expression cassette, is generally understood in the art as a cloning vector that contains the necessary regulatory sequences to allow transcription and translation of a cloned gene or genes.

Properly folded should be understood to mean a protein that is folded into its normal conformational configuration, which is consistent with how the protein folds as a naturally occurring protein expressed in its native host cell.

When refering to plants throughout the application it should be understood that the current description describes the transformation of the plastids of all organisms and plants which contain plastids. For purposes of clarity the phrase "higher plants" generally includes solanaceous and non-solanaceous plants, and the exemplary list of crops, fruits, flowers, vegetables, beans, medicinal plants, and all other plants which one skilled in the art would recognize as being a higher plant.

Substantially homologous as used throughout the ensuing specification and claims, is meant a degree of homology to the native Human Serum Albumin sequence in excess of 50%, most preferably in excess of 80%, and even more preferably in excess of 90%, 95% or 99%. Substantial sequence identity or substantial homology as used herein, is used to indicate that a nucleotide sequence or an amino acid sequence exhibits substantial structural or functional equivalence with another nucleotide or amino acid sequence. Any structural or functional differences between sequences having substantial sequence identity or substantial homology will be de minimis; that is, they will not affect the ability of the sequence to function as indicated in the desired application. Differences may be due to inherent variations in codon usage among different species, for example. Structural differences are considered de minimis if there is a significant amount of sequence overlap or similarity between two or more different sequences or if the different sequences exhibit similar physical characteristics even if the sequences differ in length or structure. Such characteristics include, for example, ability to maintain expression and properly fold into the proteins conformational native state, hybridize under defined conditions, or demonstrate a well defined immunological cross-reactivity, similar biopharmaceutical activity, etc. Each of these characteristics can readily be determined by the skilled practitioner in the art using known methods.

Non-green plastids generally refers to any plastid that is not green. Examples of such plastids include, the chromoplasts, which are present in the fruits, vegetables, and flowers; amyloplasts; proplastids in the roots of higher plants; leucoplasts and etioplasts (which express in the dark) and different develop stages of chloroplast, wherein the chloroplast is not green. Further the non-green part of plants and plant cells is well characterized and understood in the art.

Spacer region is understood in the art to be the region between two genes. The chloroplast genome of plants contains spacer regions which highly conserved nuclear tide sequences. The highly conserved nature of the nuclear tide sequences of these spacer regions chloroplast genome makes the spacer region ideal for construction of vectors to transform chloroplast of a wide variety of plant species, without the necessity of constructing individual vectors for different plants or individual crop species. It is well understood in the art that the sequences flanking functional genes are well-known to be called "spacer regions". The special features of the spacer region are clearly described in the Applicant's U.S. Patent 7,129,391 B1 with a filing date of May 15, 1998 and entitled UNIVERSAL CHLOROPLAST INTEGRATION OF EXPRESSION VECTORS, TRANSFORMED PLANTS AND PRODUCTS THEREOF. It was well-known that there are at least sixty transcriptionally-active spacer regions within the higher plant chloroplast genomes (Sugita, M., Sugiura. M., Regulation of Gene Expression in Chloroplasts of Higher Plants, Plant Mol. Biol., 32: 315-326, 1996). Specifically, Sugita et al. reported sixty transcriptionally-active spacer regions referred to as transcription units, as can be seen in Table II of the article. Because the transcriptionally active spacer regions are known, a universal vector, as described in the Applicant's U.S. Patent 7,129,391 B1, can be used in the identified spacer regions contained within a variety of the higher plant chloroplast genomes. By utilizing the teachings in Sugita et al., intergenic spacer regions are easily located in the plastid genome. Consequently this allows one skilled in the art to use the methods taught in the Applicant's U.S. Patent 7,129,391 B1 to insert a universal vector containing the psbA, the 5' untranslated region (UTR) of psbA and the gene coding for the protein of interest into the spacer regions identified by Sugita et al., and found across higher plants.

Selectable marker provides a means of selecting the desired plant cells, vectors for plastid transformation typically contain a construct which provides for expression of a selectable marker gene. Marker genes are plant-expressible DNA sequences which express a polypeptide which resists a natural inhibition by, attenuates, or inactivates a selective substance, i.e., antibiotic, herbicide, or an aldehyde dehydrogenase such as Betaine aldehyde dehydrogenase (described in the Applicant's Application No. U.S. 2002/0137214 A1 filed on April 18, 2001). Alternatively, a selectable marker gene may provide some other visibly reactive response, i.e., may cause a distinctive appearance or growth pattern relative to plants or plant cells not expressing the selectable marker gene in the presence of some substance, either as applied directly to the plant or plant cells or as present in the plant or plant cell growth media.

In either case, the plants or plant cells containing such selectable marker genes will have a distinctive phenotype for purposes of identification, i.e., they will be distinguishable from non-transformed cells. The characteristic phenotype allows the identification of cells, cell groups, tissues, organs, plant parts or whole plants containing the construct. Detection of the marker phenotype makes possible the selection of cells having a second gene to which the marker gene has been linked.

The use of such a marker for identification of plant cells containing a plastid construct has been described in the literature. In the examples provided below, a bacterial aadA gene is expressed as the marker. Expression of the aadA gene confers resistance to spectinomycin and streptomycin, and thus allows for the identification of plant cells expressing this marker. The aadA gene product allows for continued growth and greening of cells whose chloroplasts comprise the selectable marker gene product. Numerous additional promoter regions may also be used to drive expression of the selectable marker gene, including various plastid promoters and bacterial promoters which have been shown to function in plant plastids.

Inverted Repeat Regions are regions of homology, which are present in the inverted repeat regions of the plastid genome (known as IRA and IRB), two copies of the transgene are expected per transformed plastid. Where the regions of homology are present outside the inverted repeat regions of the plastid genome, one copy of the transgene is expected per transformed plastid.

Structural equivalent should generally be understood meaning a protein maintaining the conformational structure as the native protein expressed in its natural cell.

### Vectors

The current application contemplates the use of vectors capable of plastid transformation, particularly for plastid transformation. Such vectors include plastid expression vectors such as pUC, pBR322, pBLUESCRIPT, pGEM, and all others identified by Daniel in U.S. Patent No. 5,693,507 and U.S. Patent No. 5,932,479. Included are also vectors whose flanking sequences are located outside of the embroidered repeat of the chloroplast genome.

The universal vector is described in WO 99/10513 which was published on March 4, 1999, and U.S. Patent 7,129,391 B1 which was filed on May 15, 1998.

Basic pLD vector, developed in this laboratory for chloroplast transformation, was used (Daniell *et al.,* 1998; Daniell *et al.,* 2001b; De Cosa *et al.,* 2001; Guda *et al.,* 2000; *Kota et al.,* 1999). High levels of foreign protein expression in chloroplasts (3-21 % of tsp) have been shown for different proteins using the SD 5' sequence (Daniell et al., 2001b; DeGray et al., 2001; Kota et al., 1999).

It should be noted that the vectors described herein are illustrative examples and vectors can be constructed with different promoters such as was described in U.S. Patent 7,129,391 B1, different selectable markers such as those described in U.S. Patent Application 2002/0137214 A1, and different flanking sequences suitable for integration into a variety of plant plastid genomes.

### GENERAL METHODOLOGY FOR TRANSFORMING THE PLASTID GENOME

This illustrative example shows generally all of the necessary steps to practice the Applicants invention. Of course other suitable methods, which are known in the art may be substituted or used to supplement the example methodology described herein.

### Isolation of genomic DNA from plants.

Mortar and pestle, liquid nitrogen, fresh dark green leaves. DNeasy Plant Mini Kit (QIAGEN Inc.)

### PCR amplification of chloroplast flanking sequence.

*Materials for PCR reaction:* Genomic DNA (50-100ng/µl), dNTPs, 10x *pfu* buffer, Forward primer, Reverse primer, autoclaved distilled H₂O and Turbo *pfu* DNA Polymerase.

### Vector construction.

1. Plasmid pUC19 or pBlueScript SK (+/-).
2. Species specific PCR amplified chloroplast DNA flanking sequences.
3. A promoter functional in plastids, 5'UTR of chloroplast gene, selectable marker gene, gene of interest and chloroplast 3'UTR.
4. Restriction enzymes and buffers.
5. T4 DNA polymerase to remove 3' overhangs to form blunt ends and fill-in of 5' overhangs to form blunt ends or Klenow large fragment (fill-in of 5' overhangs to form blunt ends), alkaline phoshatase for dephoshorylation of cohesive ends, DNA ligase to form phosphodiester bonds and appropriate buffers.
6. Water baths or incubators set at different temperatures.

### Preparation for biolistics.

1. Autoclaved Whatman filter paper #1 (55 mm in diameter) dried in oven.
2. 100% ethanol.
3. Autoclaved tips in box, autoclaved kimwipes tissues wrapped in aluminum foil.
4. Sterile gold particles stored at -20°C in 50% glycerol (*see* Notes 1 and 2).
5. Sterile rupture discs (1100 psi) and macrocarriers sterilized by dipping in 100% ethanol.
6. Autoclaved steel macrocarrier holders and stopping screens.
7. Freshly prepared 2.5 mM CaCl₂: weigh 1.84 g and dissolve in 5 mL H₂O and filter sterilized with 0.2 µm filter.
8. 0.1 M spermidine (highly hygroscopic): dilute 1M spermidine stock to 10x and aliquot 100 µL in 1.5 mL Eppendrop tubes to store at -20°C. Discard each tube after single use.

### Medium preparation for plant tissue culture.

### 2.5.1. Tobacco.

Medium for 1000 mL: 4.3 g MS salts (INVITROGEN Inc.), H₂O (molecular biology grade), 100 mg/L myo-inositol, 1 mg/L thiamine-HCl, 3% sucrose for shoot induction and 2% sucrose for root induction, 1mg/L 6-benzyl aminopurine (BAP; use 1 mL from 1mg/mL stock), 0.1 mg/L indole-3- acetic acid (use 0.1 mL from 1 mg/mL IAA stock), 1 mg/L indole-3-butyric acid for root induction (use 1 mL from 1mg/mL IBA stock). Add 500 mg/L spectinomycin in autoclaved medium when it cools to 45°C - 50°C (use 5 mL filter sterilized spectinomycin from 100 mg/mL stock).

### Edible crops.

### Potato.

Medium for 1000 mL: 4.3 g MS salts, B5 vitamins (make 100x solution in 100 mL H₂O by dissolving: 1 g myo-inositol, 10 mg nictonic acid, 10 mg pyridoxine-HCl, 100 mg thiamine-HCl; use 10 mL, store remaining solution at 4°C), 5 mg/l zeatin riboside (use 0.5 mL from 1 mg/mL ZR stock), 0.1 mg/l α-napthaleneacetic acid (use 0.1 mL from 1 mg/mL NAA stock), 40 to 500 mg/L spectinomycin.

### Tomato

Medium for 1000 mL: 4.3 g MS salts, B5 vitamins (10 mL from 10x stock), 0.2 mg/l indole-3-acetic acid (use 0.2 mL from 1 mg/mL IAA stock), 3 mg/l of 6-benzylaminopurine (use 3 mL from 1 mg/mL BAP stock). 300 or 500 mg/L spectinomycin.

For all plant growth media adjust to pH 5.8 with 1N KOH or 1N NaOH and add 6g/L phytagel (Sigma) before autoclaving at 121°C for 20 min. For preparation of 1mg/mL stock of BAP, IAA, IBA, NAA, ZR respectively: weigh 10 mg powder and dissolve first in 1 or 2 drops of 1N NaOH and make up the final volume to 10 mL; store all plant growth regulators at 4°C for 1-2 months).

### Molecular analysis of Transgenic plants.

### PCR analysis for gene Integration into tobacco chloroplasts

PCR reaction for 50 µL: 1.0 µl genomic DNA (50-100 ng/µl), 1.5 µl dNTPs (stock10 mM), 5.0 µl (10x PCR buffer), 1.5 µl Forward primer (to land on the native chloroplast genome; stock 10 µM), 1.5 µl Reverse primer (to land on the transgene; stock 10 µM), 39.0 µl autoclaved distilled H₂O and 0.5 µl *Taq* DNA polymerase.

### Analysis of homoplasmy by Southern blots.

1. Depurination solution: 0.25 N HCl (use 0.4 mL HCl from 12.1 N HCl; Fisher Scientific USA, to make up final volume 500 mL with distilled H₂O).
2. Transfer buffer: 0.4 N NaOH, 1 M NaCl (weigh 16 g NaOH and 58.4 g NaCl and dissolve in distilled H₂O to make up the final volume to 1000 mL).
3. 20X SSC: 3M NaCl, 0.3 M sodium citrate trisodium salt (weigh 175.3 g NaCl, 88.2 g Na₃C₆H₅O₇.2H₂O 900 mL H₂O and adjust pH 7.0 using 1 N HCl and make up the final volume to 1000 mL with distilled H₂O and autoclave).
4. 2X SSC: Add 20 mL of 20X SSC in 180 mL of distilled H₂O.

### Protein analysis by Western blots.

1. Acrylamide/Bis: ready made from Fischer (USA), stored at 4°C.
2. 10% SDS: dissolve 10 g SDS in 90 mL deionized water, make up the volume to 100 mL, store at room temperature.
3. Resolving gel buffer: 1.5 M Tris-HCl (add 27.23 g Tris base in 80 mL water, adjust to pH 8.8 with 6 N HCl and make up the final volume to 150 mL. Store at 4°C after autoclaving).
4. Stacking gel buffer: 0.5 M Tris-HCl (add 6.0 g Tris base in 60 mL water. Adjust to pH 6.8 with 6 N HCl. Make up the volume to 100 mL. Store at 4°C after autoclaving).
5. Sample Buffer (SDS Reducing Buffer): In 3.55 mL water add 1.25 mL 0.5 M Tris-HCl (pH 6.8), 2.5 mL glycerol, 2.0 mL (10% SDS), 0.2 mL (0.5% Bromophenol blue). Store at room temperature. Add 50 µL (β-Mercaptoethanol (βME) to 950 µL sample buffer prior to its use.
6. 1OX running buffer (pH 8.3): Dissolve 30.3 g Tris Base, 144.0 g Glycine and 10.0 g SDS in ~ 700 mL water (add more water if not dissolving). Bring up the volume to 1 L and store at 4°C.
7. 10x PBS: Weigh 80 g NaCl, 2 g KC1, 26.8 g Na₂HPO₄7 H₂O (or 14.4 g Na₂HPO₄) 2.4 g KH₂PO₄ in 800 mL water. Adjust pH to 7.4 with HCl and make up the volume to 1 L. Store at room temperature after autoclaving.
8. 20% APS: Dissolve 200 mg ammonium persulfate in 1 mL water (make fresh every two weeks).
9. Transfer buffer for 1500 mL: Add 300 mL 10x running buffer, 300 mL methanol, 0.15 g SDS in 900 mL water and make volume to 1 L.

**Plant Extraction Buffer:**

| | Used Concentration | Final Concentration |
|---|---|---|
| 60µl | 5M NaCl | 100 mM |
| 60 µl | 0.5 M EDTA | 10 mM |
| 600 µl | 1 M Tris-HCl | 200 mM |
| 2 µl | Tween-20 | .05% |
| 30 µL | 10% SDS | 0.1% |
| 3 µL | BME | 14 mM |
| 1.2 mL | 1 M Sucrose | 400 mM |
| 1 mL | Water | |
| 60 µL | 100 mM PMSF | 2mM |

Add PMSF just before use (vortex to dissolve PMSF crystals).

PMSF (Phenylmethyl sulfonyl fluoride): Dissolve 17.4 mg of powdered PMSF in 1 mL of methanol by vortexing and store at -20°C for up to a month.

### Methods

### Isolation of genomic DNA from plants.

Extract the genomic DNA from fresh green leaves using DNeasy Plant kit (QIAGEN Inc.) following vender's instructions.

### Amplification of chloroplast flanking sequence.

Species-specific flanking sequences from the chloroplast DNA or genomic DNA of a particular plant species is amplified with the help of PCR using a set of primers that are designed using known and highly conserved sequence of the tobacco chloroplast genome.

Conditions for running PCR reaction: There are three major steps in a PCR, which are repeated for 30 to 40 cycles. (1) *Denaturation at 94°C:* to separate double stranded chloroplast DNA. (2) *Annealing at 54 to 64°C:* primers bind to single stranded DNA with formation of hydrogen bonds and the DNA polymerase starts copying the template. (3) *Extension at 72°C:* DNA Polymerase at 72°C extends to the template that strongly forms hydrogen bond with primers. Mismatched primers will not form strong hydrogen bonds and therefore, all these temperatures may vary based on DNA sequence homology. The bases complementary to the template are coupled to the primer on the 3' side. The polymerase adds dNTPs from 5' to 3', reading the template in 3' to 5' direction and bases are added complementary to the template.

### Chloroplast Transformation vector.

The left and right flanks are the regions in the chloroplast genome that serve as homologous recombination sites for stable integration of transgenes. A strong promoter and the 5' UTR and 3' UTR are necessary for efficient transcription and translation of the transgenes within chloroplasts. For multiple gene expression, a single promoter may regulate the transcription of the operon, and individual ribosome binding sites must be engineered upstream of each coding sequence (2) (Fig. 10). The following steps are used in vector construction:
1. Amplification of flanking sequences of plastid with primers that are designed on the basis of known sequence of the tobacco chloroplast genome (between 16S-23S region of chloroplast).
2. Insert the PCR product containing the flanking sequence of the chloroplast genome into pUC19 plasmid digested with *Pvu*II restriction enzyme (to eliminate the multiple cloning site), dephoshorylated with the help of alkaline phoshatase (CIP) for 5 min at 50°C (to prevent recircularization of cloning vector). Inactivate CIP enzyme at 68°C for 10 min.

Clone chloroplast transformation cassette (which is made blunt with the help of T4 DNA polymerase or Klenow filling) into a cloning vector digested at the unique PvuII site in the spacer region, which is conserved in all higher plants examined so far.

### Delivery of foreign genes into chloroplasts via particle gun.

This is most successful and a simple technique to deliver transgenes into plastids and is referred as Biolistic PDS-1000/ He Particle Delivery System (18,19). This technique has proven to be successful for delivery of foreign DNA to target tissues in a wide variety of plant species and integration of transgenes has been achieved in chloroplast genomes of tobacco ***(2),** Arabidopsis **(20),*** potato **(*21*),** tomato **(*25*)** and transient expression in wheat **(22),** carrot, marigold and red pepper ***(23)** (see* **Note 5).**

### Preparation of gold particle suspension.

1. Suspend 50-60 mg gold particles in 1 mL 100% ethanol and vortex for 2 min.
2. Spin at maximum speed ∼10, 000 x g (using tabletop microcentrifuge) for 3 min.
3. Discard the supernatant.
4. Add 1ml fresh 70% ethanol and vortex for 1 min.
5. Incubate at room temperature for 15 min and shake intermittently.
6. Spin at 10,000 x g for 2 min.
7. Discard supernatant, add 1ml sterile distilled H₂O, vortex for 1min, leave at room temperature for 1min, and spin at 10, 000 x g for 2 min.
8. Repeat above washing process three times with H₂O (step 7).
9. Resuspend the gold-pellet in 1 mL 50% glycerol, store stock in -20°C freezer.

### Precipitation of the chloroplast vector on gold particles for five samples.

1. Take 50 µl the gold particles in 1.5 mL tube after vortexing for a 1 min.
2. Add 10 µl DNA (about 1 µg/µl plasmid DNA), and vortex the mixture for 30 sec.
3. Add 50 µl of 2.5 M CaCl₂ and vortex the mixture for 30 sec.
4. Add 20 µl of 0.1 M spermidine and vortex the mixture for 20 min at 4°C.

### Washing of chloroplast vector coated on gold particles.

1. Add 200 µl 100% ethanol and vortex for 30 sec.
2. Spin at 3000 x g for 40 sec.
3. Pour off ethanol supernatant.
4. Repeat ethanol washings five times.
5. In the last step, pour off ethanol carefully and add 35-40 µl ethanol (100%).

### Preparation of macro carriers .

**1. Sterilize macrocarriers by dipping in 100% ethanol for 15 min and insert them into sterile steel ring holder with the help of a plastic cap when air-dried.**
2. Vortex the gold-plasmid DNA suspension and pipet 8-10 µl in the center of macrocarrier and let it air dry.

### Gene gun setup for bombardment of samples.

1. Wipe the gun chamber and holders with 100% ethanol using fine tissue paper (do not wipe the door with alcohol).
2. Turn on the vacuum pump.
3. Turn on the valve (Helium pressure regulator) of Helium gas tank (anticlockwise).
4. Adjust the gauge valve (adjustable valve) ∼200 to 250 psi above the desired rupture disk pressure (clockwise) using adjustment handle.
5. Turn on the gene gun.
6. Place the rupture disc (sterilized by dipping in 100% ethanol for 5 min) in the rupture disc-retaining cap and tightly screw to the gas acceleration tube.
7. Place a stopping screen in the macrocarrier launch assembly and above that place macrocarrier with gold particles with chloroplast vector facing down towards screen. Screw assembly with a macrocarrier coverlid and insert in the gun chamber.
8. Place an intact leaf or explants to be bombarded on a filter paper (Whatman No. 1) soaked in medium containing no antibiotics. Place sample plate over target plate shelf, insert in the gun chamber and close the bombardment chamber door.
9. Press Vac switch to build pressure (up to 28 inches of Hg) in the vacuum gauge display. Turn same switch down at hold point and press Fire switch until you hear a burst sound of the ruptured disc.
10. Press Vent switch to release the vacuum and open the chamber to remove sample.
11. Shut down the system by closing the main valve (Helium pressure regulator) on the Helium gas cylinder. Create some vacuum in the gene gun chamber and keep using fire switch on and off until both pressure gauges' show zero reading. Release the vacuum pressure and turn off the gene gun and vacuum pump.
12. Incubate bombarded sample plates in the culture room for two days in the dark (i.e. covered with aluminum foil) and on the third day cut explants in appropriate pieces and place on the selection medium.

### Plant tissue culture and chloroplast transformation.

### Tobacco chloroplast transformation.

A highly efficient and reproducible protocol has been established for *Nicotiana tabacum* cv. Petit Havana (Daniell, H. (1997) Methods in Mol. Biol. Recombinant gene expression protocols. 62,463-489.
1. Bombard 4 weeks old dark green tobacco leaves on the abaxial (bottom side) side with the chloroplast vector and incubate leaves in the dark for 2 days on selection free medium.
2. On the third day cut bombarded leaf explants into small square pieces (5 mm) and place explants facing abaxial surface towards selection medium containing MS salts, 1mg/l thiamine HCl, 100mg/l myo-inositol, 3% sucrose, 1mg/l BAP and 0.1 mg/l IAA along with 500 mg/l spectinomycin as a selective agent.
3. Transgenic shoots should appear after three to five weeks of transformation. Cut the shoot leaves again into small square explants (2 mm) and subject to a second round of selection for achieving homoplasmy on fresh medium.
4. Regenerate transgenic shoots (confirmed by PCR for transgene integration) on rooting medium containing MS salts, 1mg/l thiamine HCl, 100mg/l myo-inositol, 2% sucrose and 1mg/l IBA with 500mg/l spectinomycin.
5. Transfer transgenic plants into pots under high humidity and move them to green house or growth chamber for further growth and characterization.

### Plastid transformation of edible crops.

The concept of universal vector for using the chloroplast DNA from one plant species to transform another species (of unknown sequence) was developed by the Daniell group (8). Using this concept both tomato and potato chloroplast genomes were transformed as described below.

### Potato chloroplast transformation.

Using the tobacco chloroplast vector, leaf tissues of potato cultivar FL1607 was transformed via biolistics, and stable transgenic plants were recovered using the selective *aad*A gene marker and the visual green fluorescent protein (GFP) reporter gene (*21*).
1. Bombard potato leaves (3-4 week old) and incubate in the dark for 2 days on selection free medium.
2. Third day excise leaves into small square pieces (5 mm) and place on MS medium containing B5 vitamins, 5 mg/l ZR, 0.1 NAA, and 3% sucrose. Gradually increase spectinomycin selection pressure (40 to 400 mg/l) after every two weeks subculture under diffuse light.
3. Regenerate shoots from transgenic potato calli on MS medium containing B5 vitamins, 0.01mg/L NAA, 0.1mg/L GA3, 2% sucrose and 40-400 mg/l spectinomycin.
4. Transfer transgenic shoots on basal MS medium containing B5 vitamins, 2% sucrose and 40-400 mg/l spectinomycin for root induction. Transfer transgenic plantlets to growth chamber.

### Tomato chloroplast Transformation.

Using the tobacco chloroplast vector, tomato (*Lycopersicon esculentum* cv. IAC Santa Clara) plants with transgenic plastids were generated using very low intensity of light **(25).**
1. Bombard four-week-old tomato leaves and incubate in the dark for 2 days on selection free medium.
2. Excise bombarded leaves into small pieces and place on shoot induction medium containing 0.2 mg/L IAA, 3 mg/L BAP, 3% sucrose and 300 mg/L spectinomycin.
3. Select spectinomycin-resistant primary calli after a three to four month duration without any shoot induction.
4. Regenerate shoots in about four weeks after transfer of transgenic calli to shoot induction medium containing 0.2 mg/L IAA, 2 mg/L ZR, 2% sucrose and 300 mg/L spectinomycin then root on hormone-free medium. Transfer regenerated transgenic plants into the greenhouse.

### Molecular analysis of transgenic plants.

### PCR screening of Transgenic shoots.

This method has been used to distinguish between mutants, nuclear and chloroplast transgenic plants. By landing one primer on the native chloroplast genome adjacent to the point of integration and a second primer on the *aadA* gene (26. PCR product of an appropriate size should be generated in chloroplast transformants. Since this PCR product cannot be obtained in nuclear transgenic plants or mutants, the possibility of nuclear integration or mutants should be eliminated.
1) Extract the genomic DNA from transgenic leaf tissue using DNeasy Plant kit (QIAGEN Inc.) by following vender's instructions. For lower amount of transgenic tissues, volume of buffers may be reduced appropriately.
2) Run PCR reaction with Taq DNA Polymerase (QIAGEN Inc.) using appropriate primers following the same conditions as described above for amplification of flanking sequences.

### Analysis of homoplasmy by Southern. blot.

In Southern blot analysis, tobacco plastid genome digested with suitable restriction enzymes should produce a smaller fragment (flanking region only) in wild type plants compared to transgenic chloroplast that include transgene cassette as well as the flanking region. In addition, homoplasmy in transgenic plants is achieved when only the transgenic fragment is observed.

### Transfer of DNA to membrane.

1. Digest the genomic DNA (~2 to 10 µg) with suitable restriction enzymes from transgenic samples (including wild type as a control) and run digested DNA on 0.8% agarose gel containing 5 µL EtBr (from 10 mg/mL stock) in 100 mL for four hours at 40V.
2. Soak gel in 0.25 N HCl (depurination solution) for 15 minutes and rinse gel twice in distilled H₂O for 5 minutes.
3. Soak gel for 20 minutes in transfer buffer to denature DNA.
4. Transfer overnight DNA from gel to nylon membrane (pre-soak first in water, then in transfer buffer for 5 minutes) using the transfer buffer.
5. Next day, rinse membrane twice with 2x SSC buffer for 5 minutes each and air-dry for 5 minutes on filter papers. Cross-link transferred DNA to membrane using GS GeneLinker UV Chamber (Bio-Rad) at appropriate (C3) setting.

### Preparation of probe.

1. Digest any plasmid (containing flanking sequences of the chloroplast genome) with appropriate restriction enzymes.
2. Denature 45 µL flanking DNA fragment (50-250 ng) at 95°C for 5 minutes, then place on ice for 2-3 minutes.
3. Add denatured probe to Ready-To-Go DNA Labeling Beads (-dCTP) tube (Amersham Biosciences, USA) and gently mix by flicking the tube.
4. Add 5 *µ*L radioactive α³²P (dCTP; Amersham Biosciences, USA) to probe mixture and incubate at 37°C for 1hour and filter the probe using ProbeQuant G-50 Micro Columns (Amersham Pharmacia Biotech Inc. USA).

### Prehybridization and hybridization.

Place the blot (DNA transfer side facing towards the solution) in a hybridization bottle and add 10 mL Quik-Hyb (Stratagene, USA).

Incubate for 1 hour at 68°C. Add 100 *µ*L sonicated salmon sperm (10 mg/mL stock; Stratagene, USA) to the labeled probe and heat at 94°C for 5 minutes and add to bottle containing membrane and Quik-Hyb solution. Incubate for 1 hour at 68°C.

### Washing and autoradiography.

1. Discard Quik-Hyb solution with probe and wash membrane twice in 50 mL (2x SSC buffer and 0.1% SDS) for 15 minutes at room temperature.
2. Wash membrane twice in 50 mL (0.1x SSC buffer and 0.1% SDS) for 15 minutes at 60°C.
3. Wrap the wash membrane in saran wrap and expose blot to x-ray film in the dark and leave at -70°C until ready for development.

### Determination of transgene expression by Western blot.

### Extraction of plant protein.

1. Grind 100 mg of leaf in liquid nitrogen and add 200 µL of extraction buffer to samples on ice.
2. Add appropriate volume of freshly prepared 2x Sample loading buffer to an aliquot plant extract (from a stock containing 50 µL *β*-mercaptoethanol and 950 µL sample loading buffer).
3. Boil samples for 4 minutes with loading dye and centrifuge for 2 minutes at 10, 000 x g, then immediately load 20 µL samples into gel.

### Running gel.

Load samples on gel and run for half hour at 100 V, then 1 hour at 150 V until the marker bands corresponding to your protein are in middle.

### Transfer of protein to membrane.

Transfer protein from gel to membrane using Mini Transfer Blot Module at 30 V overnight or 65 V for 2 hours or 100 V for 1 hour. Membrane wrapped in saran wrap can be stored at -20°C for a few days if necessary.

### Membrane blocking

1. After transfer, rinse membrane with water and incubate membrane in PTM (100 mL 1x PBS, 50 µL 0.05% Tween 20, and 3 g dry milk (3%) for 1 hour at room temperature.
2. Add primary antibody in suitable dilution for 15 mL and incubate for 2 hours at room temperature. Wash membrane twice with 1x PBS for 5 minutes each.
3. Add secondary antibody in proper dilution for 20 mL. Incubate for 1.5 hours at room temperature on a shaker.
4. Wash twice with PT (100 ml 1x PBS + 50 µL Tween 20) for 15 minutes and finally with 1x PBS for 10 minutes.

### Exposure of the blot to X-ray film.

1. Mix 750 µL of each chemiluminescent solution (Luminol Enhancer and Stable Peroxide) in 1.5 mL tube and add to membrane, cover thoroughly.
2. Wipe out extra solution and expose blot to x-ray film for appropriate duration and develop film.

### Seed sterilization.

1. Vortex small amount of seeds into microcentrifuge tube with 1 mL 70% ethanol for 1 minute. Discard ethanol after brief spin.
2. Add 1 mL disinfecting solution (1.5% Bleach and 0.1% Tween 20) in tube and vortex intermittently for 15 min. Discard solution after brief spin.
3. Wash the seed thrice with sterile distilled water.
4. Spray seeds with sterile water on plate containing RMOP basal medium supplemented with 500 µg/mL spectinomycin to determine maternal inheritance in transgenic chloroplast plants.

### Evaluation of results.

### Material inheritance in chloroplast transgenic plants.

Transgenes integrated into chloroplast genomes are inherited maternally. This is evident when transgenic seed of tobacco are germinated on RMOP basal medium containing 500 µg/mL spectinomycin. There should be no detrimental effect of the selection agent in transgenic seedlings whereas untransformed seedlings will be affected.

### CTB-GM1-gangliosides binding ELISA assay.

1. Coat microtiter plate (96 well ELISA plate) with monosialoganglioside-GM1 {3.0 µg/mL in bicarbonate buffer (15 mM Na₂CO₃, 35 mM NaHCO₃, pH 9.6)} and as a control, coat BSA (3.0 ug/mL in bicarbonate buffer) in few wells.
2. Incubate plate overnight at 4°C.
3. Block wells with 1% (w/v) bovine serum albumin (BSA) in 0.01 M phosphate-buffered saline (PBS) for two hours at 37°C.
4. Wash wells thrice with PBST buffer (PBS containing 0.05% Tween 20).
5. Incubate plate by adding soluble protein from transformed and untransformed plants and bacterial CTB in PBS.
6. Add primary antibodies (rabbit anti cholera serum diluted 1:8000 in 0.01 M PBST containing 0.5% BSA) and incubate plate for 2 hours at 37°C.
7. Wash well thrice with PBST buffer.
8. Add secondary antibodies diluted 1:50,000 (mouse anti rabbit IgG-alkaline phosphatase conjugate in 0.01 M PBST containing 0.5% BSA) and incubate plate for 2 hours at 37°C.
9. Develop plate with Sigma Fast pNPP substrate. Stop reaction by adding 3 M NaOH and read plate absorbance at 405 nm.

The macrophage lysis assay is as follows:
1. Isolate crude extract protein from 100 mg transgenic leaf using 200 µL of extraction buffer containing CHAPS detergent (4% CHAPS, 10 mM EDTA, 100 mM NaCl, 200 mM Tris-HCI, pH 8.0, 400 mM sucrose, 14 mM *β*-mercaptoethanol, 2 mM PMSF) and one without CHAPS detergent.
2. Spin samples for five minutes at 10, 000 x g and use both supernatant and homogenate for assay
3. Plate macrophage cells RAW 264.7 (grown to 50% confluence) into 96-wells plate, incubated in 120 µL Dulbecco's Modified Eagle's Medium (DMEM; from Invitrogen life technologies).
4. Aspirate medium from wells and add 100 µL medium containing 250 ng/mL proteins in crude leaf extract.
5. In control plate, add only DMEM with no leaf fraction to test toxicity of plant material and buffers.
6. In another plate, add 40 µL dilutions onto RAW 264.7 cells from plant samples, which serially diluted 2 fold, so that the top row had plant extract at 1:14 dilution.
7. Add 20 □L of MTT 3-[4,5-dimethylthiazol-2-yl]-2,5-diphenyltetrazolium bromide; Sigma) to each well containing cells (from a stock 5mg/ml MTT dissolved in lxPBS and filter sterilize) after 5 hours to assess the cell death.
8. Incubate the plate at 37°C for 5 hours. Remove media with needle and syringe. Add 200 □L of DMSO to each well and pipette up and down to dissolve crystals. Transfer to plate reader and measure absorbance at 550nm.

Active PA was found in both the supernatant and homogenate fractions. However, maximum macrophage lysis activity was noticed in supernatant when extraction buffer was used with CHAPS detergent.

### Cholera toxin (CTB) antigen as an edible vaccine.

Chloroplast transgenic plants are ideal for production of vaccines. The heatlabile toxin B subunits of *E. coli* enterotoxin (LTB), or cholera toxin of *Vibrio cholerae* (CTB) have been considered as potential candidates for vaccine antigens. Integration of the unmodified native *CTB* gene into the chloroplast genome has demonstrated high levels of CTB accumulation in transgenic chloroplasts (Daniell, H., et al. (2001). J. Mol. Biol. 311,1001-1009.). This new approach not only allowed the high level expression of native *CTB* gene but also enabled the multimeric proteins to be assembled properly in the chloroplast, which is essential because of the critical role of quaternary structure for the function of many vaccine antigens. The expression level of CTB in transgenic plants was between 3.5% and 4.1% tsp and the functionality of the protein was demonstrated by binding aggregates of assembled pentamers in plant extracts similar to purified bacterial antigen, and binding assays confirmed that both chloroplast-synthesized and bacterial CTB bind to the intestinal membrane GM1-ganglioside receptor, confirming correct folding and disulfide bond formation of CTB pentamers within transgenic chloroplasts (Fig. 11). Further, this disclosure contemplates the examples of edible vaccines expressed via the plastid, such as CTB(as described above), Anthrax, Plague, and all other vaccines known and described in the art including those described in WO 03/057834 A2, filed on 12/26/02. The aspects of this disclosure further contemplate the expression of other therapeutic proteins such as interferon, IGF-1, insulin, which will be expressed in non-green plant cells for oral delivery. An exemplary description of such therapeutic proteins can be found in the PCT applications which claim priority to U.S. provisional application Nos. 60/393,439;60/393,428;60/393,451; 60/393,649; and 60/393,438, wherein the expression of therapeutic proteins in the plant plastid is described.

### Oral delivery of vaccines and selection of Transgenic plants without the use of antibiotic selectable markers.

Betaine aldehyde dehydrogenase (*BADH*) gene from spinach has been used as a selectable marker to transform the chloroplast genome of tobacco (Daniell, H. et al., (2001) Curr. Genet. 39,109-116). Transgenic plants were selected on media containing betaine aldehyde (BA). Transgenic chloroplasts carrying BADH activity convert toxic BA to the beneficial glycine betaine (GB). Tobacco leaves bombarded with a construct containing both *aad*A and *BADH* genes showed very dramatic differences in the efficiency of shoot regeneration. Transformation and regeneration was 25% more efficient with BA selection, and plant propagation was more rapid on BA in comparison to spectinomycin. Chloroplast transgenic plants showed 15 to 18 fold higher BADH activity at different developmental stages than untransformed controls. Expression of high BADH level and resultant accumulation of glycine betaine did not result in any pleiotropic effects and transgenic plants were morphologically normal and set seeds as untransformed control plants.

### Production of human therapeutic proteins in transgenic chloroplasts.

### Human serum albumin (HSA) protein.

Human Serum Albumin (HSA) accounts for 60% of the total protein in blood and widely used in a number of human therapies. Chloroplast transgenic plants were generated expressing HSA (Fernandez-San Millan et al., (2003) Plant Bitechnol. J. 1,71-79). Levels of HSA expression in chloroplast transgenic plants was achieved up to 11.1% tsp. Formation of HSA inclusion bodies within transgenic chloroplasts was advantageous for purification of protein. Inclusion bodies were precipitated by centrifugation and separated easily from the majority of cellular proteins present in the soluble fraction with a single centrifugation step. Purification of inclusion bodies by centrifugation may eliminate the need for expensive affinity columns or chromatographic techniques.

### Purification of HSA.

1. Solubilize the HSA inclusion bodies from transformed tissues using extraction buffer containing 0.2M NaCl, 25 mM Tris-HCl (pH 7.4), 2mM PMSF and 0.1% Triton X-100.
2. Spin at 10, 000 x g. Suspend the pellet in buffer containing 6M Gu-HCl, . 0.1M *β*ME and 0.25 mM Tris-HCl (pH 7.4).
3. Dilute plant extract 100-fold in buffer containing 100 mM NaCl, 50 mM Tris-HCl (pH 8.5) and 1 mM EDTA.
4. Concentrate HSA protein by precipitation using a polyethylenglycol treatment at 37%.
5. Separate protein fractions by running a SDS-PAGE gel and stain gel with silver regent, following vender's instruction (Bio-Rad, USA).

### Electron microscopy and immunogold labeling.

1. Cut the transformed and untransformed leaf in 1-3 mm squares.
2. Fix them in 0.1 M cacodylate buffer pH 7.4 (2.5% glutaraldehyde, 2% paraformaldehyde and 5 mM CaCl₂) for 15 minutes under vacuum and 12 hours at 4°C.
3. Rinse samples twice in 0.1M cacodylate buffer (pH 7.4) after fixation.
4. Dehydrate fixed samples through a graded ethanol series to 95%, then implant in LRW resin at 60°C for 24 hours.
5. Cut ultra-thin sections using a Leica Ultracut T ultramicrotome and collect sections onto nickel grids.
6. Incubate sections in 0.05M glycine prepared in PBS buffer for 15 minutes to inactivate residual aldehyde groups.
7. Place grids onto drops of blocking solution (PBS containing 2% non-fat dry milk) and incubate for 30 minutes
8. Incubate sections for 1 hour in a goat anti-human albumin polyclonal antibody (dilution range from 1:1000 to 1:10,000 in blocking solution).
9. Wash sections with blocking solution 6 X 5 minutes each.
10. Incubate sections for 2 hours with a rabbit anti-goat IgG secondary antibody conjugate to 10 nm gold diluted 1:40 in blocking solution.
11. Wash sections 6 X 5 minutes in blocking solution and 3 X 5 minutes with PBS, and fixed sections in 2% glutaraldehyde diluted in PBS for 5 minutes,
12. Wash fixed sections in PBS 3 X 5 minutes, then in distilled water 5 X 2 min each.
13. Stain sections using uranyl acetate and lead citrate and examine samples under transmission electron microscope at 60kv.

### Notes

1. Gold particles suspended in 50% glycerol may be stored for several months at -20°C. Avoid refreezing and thawing spermidine stock; use once after thawing and discard the remaining solution. Use freshly prepared CaCl₂ solution after filter sterilization. Do not autoclave.
2. Precipitation efficiency of DNA on gold and spreading of DNA-gold particles mixture on macrocarriers is very important. For high transformation efficiency via biolistics, a thick film of gold particles should appear on macrocarrier disks after alcohol evaporation. Scattered or poor gold precipitation reduces the transformation efficiency.
3. Generally, a 1000 bp flanking sequence region on each side of the expression cassette is adequate to facilitate stable integration of transgenes.
4. Use of the 5' untranslated region (5' UTR) and the 3' untranslated region (3' UTR) regulatory signals are necessary for higher levels of transgene expression in plastids **(*13*).** The expression of transgene in the plant chloroplast depends on a functional promoter, stable mRNA, efficient ribosomal binding sites; efficient translation is determined by the 5' and 3' untranslated regions (UTR). Chloroplast transformation elements *Prrn, psbA5'UTR, 3'UTR* can be amplified from tobacco chloroplast genome.
5. Bombarded leaves after two-days dark incubation should be excised in small square pieces (5-7 mm) for first round of selection and regenerated transgenic shoots should be excised into small square pieces (2-4 mm) for a second round of selection.
6. Temperature for plant growth chamber should be around 26-28°C for appropriate growth of tobacco, potato and tomato tissue culture. Initial transgenic shoot induction in potato and tomato require diffuse light. However, higher intensity is not harmful for tobacco.
7. Transformation efficiency is very poor for both potato and tomato cultivars compared to tobacco.
8. Tobacco chloroplast vector gives low frequency of transformation if used for other plant species. For example, when petunia chloroplast flanking sequences were used to transform the tobacco chloroplast genome (DeGray, G. et al., (2001), Plant Physiol. 127,852-862.), it resulted in very low transformation efficiency.

Under diffuse light conditions, highly regenerating tomato cultivar (Microtom) shoots produce premature flowering that inhibit further growth of transgenic plants. Therefore, after the first shoot induction phase, shoots should be moved to normal light conditions.

### ILLUSTRATIVE EXAMPLE 1: SOMATIC EMBRYOGENESIS VIA CARROT TRANSFORMATION

Homoplasmic transgenic carrot plants exhibiting high levels of salt tolerance (up to 500 mM NaCl) were rapidly regenerated from carrot cell cultures, via somatic embryogenesis. Carrot chloroplast genome is strictly maternally inherited and plants do not produce seeds in the first year, offering complete containment of transgene flow. Carrot cells multiply rapidly and large biomass is produced using bioreactors; somatic embryos are derived from single cells; viable for long duration on culture medium, encapsulated embryos are used as synthetic seeds for cryopreservation and controlled germination; these features provide an ideal production system for plant made pharmaceutical proteins and their oral delivery. BADH expressing cells offer a visual selection by their green color, distinguishing them from untransformed yellow cells. A useful trait has been engineered via the chloroplast genome for the first time in a non-tobacco crop. This is the first plastid transformation achieved using non-green explants and somatic embryogenesis, opening the door to transform monocots, legumes, vegetables, fruit crops and transgene expression in non-green edible parts.

This Example presents the first report of stable and highly efficient plastid transformation of carrot using non-green tissues as explants, regenerated via somatic embryogenesis. An useful plant trait (salt tolerance) has been expressed for the first time in a non-solanaceous crop via the chloroplast genome. So far, only the tobacco chloroplast genome has been engineered to confer herbicide resistance (Daniell et al., 1998), insect resistance (McBride et al., 1995; Kota et al., 1999; DeCosa et al., 2001), disease resistance (DeGray et al., 2001), drought tolerance (Lee et al., 2003) and phytoremediation of toxic metals (Ruiz et al., 2003). In addition, expression of the BADH enzyme facilitated the visual selection of transgenic green cells from non-transgenic yellow cells. As a major crop consumed world-wide, this report should serve as a model for genetic engineering of plastid genomes in higher plant species that require the use of non-green tissues as explants and somatic embryogenesis for regeneration.

### Results and Discussion

### Construction of carrot plastid transformation vectors.

Carrot chloroplast vectors target the expression cassette to the *16S*/*trnI - trnA*/*23S* region of the chloroplast genome for integration via homologous recombination. The site of integration is similar to the universal chloroplast transformation vector (pLD) reported earlier from our laboratory (Daniell et al., 1998; Guda et al., 2000) but the size of the flanking sequence on either side of the expression cassette was doubled to enhance efficiency of homologous recombination. As a result the flanking sequence were increased to approximately 4kb. The chloroplast transformation vector pDD*-Dc-gfp*/*BADH* (Fig. 1A) is a carrot specific vector that harbors the *gfp* gene regulated by the gene 10 5'UTR / *rps*16 3'UTR (to facilitate expression in green as well as non-green tissues and screen transformants by GFP fluorescence) and the *badh* gene expressed under *the psbA* 5' and 3' UTRs (to facilitate expression in green tissues, regulated by light). All the 5' and 3' regulatory elements were PCR amplified from tobacco genomic DNA except for the gene 10 5'UTR which was derived from phage T7 gene 10. Transcription of both transgenes in this cassette is driven by the full length *Prrn 16S* rRNA promoter which contains regulatory elements for both the nuclear encoded and plastid encoded RNA polymerases, thereby facilitating transcription in green as well as non-green tissues. The chloroplast transformation vector pDD-*Dc-aadA*/*BADH* (Fig. 1B) harbors the *aad*A and *badh* genes whose expression is driven by the full length 16S rRNA promoter under the regulation of RBS (Ribosome Binding Site) / *3`psb*A UTR and the gene 10 5'UTR / *rps*16 3'UTR regulatory elements, respectively.

It should be further noted that the universal vector described in U.S. patent 7,129,391 B1 can be used to transform the plastid genome as described within this application.

### Transformation of carrot plastids and plant regeneration.

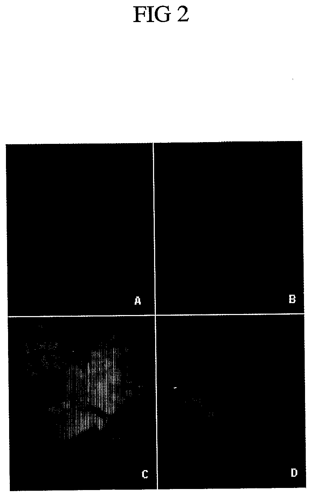

Yellow fine cell suspension culture induced from stem segments of carrot (*Daucus carota* L. cv. Half long) was bombarded with carrot chloroplast vectors pDD-*Dc-gfp*/*BADH* and pDD*-Dc-aad*A/*BADH* as described (Daniell, 1997; Kumar and Daniell, 2003). Carrot cultures transformed with pDD*-Dc-gfp*/*BADH* vector, produced GFP expressing calli and somatic embryos that were observed under confocal microscope (Fig. 2A-C) when selected on 20 mM betaine aldehyde (BA). Even though it was quite evident that somatic embryos expressing GFP could be regenerated rapidly on BA selection, there were two major limitations for using this vector. BA is quite expensive ($2000-$3000/ gm) and this limited the number of experiments that could be performed and the *badh* gene was regulated by the *psb*A promoter and UTR elements, that was under developmental and light regulation. Unfortunately, cultured carrot cells were non-green and in early stages of development, thus minimizing *badh* expression. Therefore, more efforts were made to transform carrot cells using another chloroplast vector. Using the chloroplast vector pDD*-Dc-aad*A/*BADH,* seven independent transgenic cell lines were recovered within 2-3 months from five bombardments on solid medium (MSB+ 3 mg/L 2,4-D, 1 mg/L kinetin) containing different concentrations of spectinomycin (150-450 mg/L). Further, transgenic calli were transferred to 350-mg/L spectinomycin for a month and multiplied using 500-mg/L spectinomycin. All Transgenic cell cultures were incubated under 100 1x light intensity at 26±2°C temperature. The MSB medium supplemented with 3-mg/L 2,4-D and 1 mg/L kinetin along with the selection agent was used as the growth medium to induce and multiply the transgenic cell cultures. In order to multiply transgenic cultures, they were subcultured on solid medium every 2-3 week and were grown in liquid medium (MSB+0.1 mg/L 2,4-D) at 130 rpm under diffuse light (50 1x). The medium without 2,4-D (MSB+0.2 mg/L kinetin) was used as the plant-regeneration-medium to convert embryogenic calli into plantlets. Transgenic plants maintained on basal MSB medium (containing 500 mg/L spectinomycin) were transferred to soil in pots to induce the mature taproot system and used for further characterization.

**Visible selection of transgenic carrot cells.** During in vitro cell culture studies of transgenic and non-transgenic carrot, it was interesting to note that carrot cells could be distinguished on the basis of color. Transgenic carrot cells, which carry the *badh* transgene, were always green in color whereas non-transgenic cells were yellow in color (see Fig. 3A-B). To test that transgenic bright green cells were truly transgenic, hetroplasmic (partially transformed plastids) carrot cell cultures were placed on a growth medium without selection and were allowed to segregate; green and yellow cells visually segregated within 3-4 weeks (Fig. 3C-D). Transgenic-green cells were confirmed positive for transgene integration while yellow cells were found to be untransformed. Further, suppression of untransformed cell division was possible, when cells were exposed to different concentrations of NaCl. This visible selection system would be vital to distinguish transformed from untransformed cells while eliminating the selectable antibiotic marker genes using direct repeats (Iamtham and Day, 2002) from transformed chloroplast genomes. It will be interesting to investigate the role of BADH enzyme in enhancing chlorophyll biosynthesis or stability in transgenic chloroplasts.

### Confirmation of transgenes integration into carrot plastids.

The carrot chloroplast vector pDD*-Dc-aad*A/*BADH* integrates the *aad*A and *badh* genes into the 16S-23S-spacer region of the plastid genome by homologous recombination. Transgene integration into carrot cell lines was confirmed by PCR (Fig. 4A) using internal primer set 3P (that lands on *trnI* region of plastids) and 3M (that lands on the *aad*A gene) producing 1.6 kb PCR product. This eliminates mutants that may be obtained on spectinomycin selection, caused by mutation of the chloroplast 16S rRNA gene. In order to distinguish between nuclear and chloroplast transgenic cell lines (Fig. 4B), 16S-F primer was landed on the native chloroplast genome, 200 bp upstream of integration site and 1M primer was landed on the *aadA* gene; this generated 2.5 kb size PCR product. Since this PCR product cannot be obtained in nuclear transgenic plants, the possibility of nuclear integration was eliminated. Thus, PCR analyses allow rapid screening of large number of putative transgenic lines and eliminate mutants or nuclear transgenic lines.

### Determination of homoplasmy in transgenic carrot plastids.

PCR confirmed transgenic carrot cell lines were repeatedly subcultured each week in liquid medium for several rounds of selection in the presence of selection agent. Southern blot analysis was performed using total genomic DNA isolated from untransformed and transformed carrot plants, developed from different transgenic cell lines and was digested with AflIII and PvuII (Fig. 4C). Presence of AflIII restriction site in the 16S-rRNA region (left flank) in both transformed and untransformed carrot plastids and a unique PvuII site in between *trnI* and *trnA* flanking regions of untransformed plastids as well as in the mid region of *badh* transgene, allowed excision of predicted size fragments in both the untransformed and transgenic lines. In order to confirm heteroplasmy or homoplasmy, genomic DNA of carrot plants digested with AflIII and PvuII was hybridized with the 4.9 kb radioactive DNA probe. This probe fragment was isolated from the chloroplast vector *pDD-Dc-aadA*/*BADH,* by digesting with AflIII and PvuII; this fragment includes the 2.4 kb *trnI* flanking sequence and 2.5 kb transgene sequences of the chloroplast vector (Fig. 1B). Transgenic plants developed after two subcultures in liquid medium on the selection agent (350 mg/L spectinomycin) showed heteroplasmy (Fig. 4C, lane 2), whereas plants that were developed from cell lines after 8-10 subcultures in liquid medium supplemented with high concentration of selecting agent (500 mg/L spectinomycin) showed homoplasmy (Fig. 4C, lanes 3-8).

### BADH enzyme activity in carrot cells, root and shoot.

BADH enzyme activity was assayed in crude extracts from untransformed and transformed carrot cell cultures, tap roots (carrot) and leaves as described (Daniell et al 2001). By assessing BADH enzyme activity, the expression of *badh* transgene was observed in cells and different parts of carrot plants, using 50 µg crude extract of protein from each sample, desalted using G-25 column. BADH enzyme in the presence of betaine aldehyde converts NAD⁺ to NADH and the reaction rate was measured by an increase in absorbance at 340 µm due to the reduction of NAD⁺. Crude extracts from transgenic plastids (cells, tap roots and leaves) demonstrate elevated levels of BADH activity compared to untransformed tissues of carrot (Fig. 5A). Higher BADH activity was observed in leaves, tap roots of carrot plant and transgenic cells in suspension culture, confirming that full length *16S* promoter *Prrn* and gene*10* UTR are highly suitable for expressing transgenes in various tissues. Because these regulatory elements are anticipated to function uniformly in all tissues, we presume that the difference in BADH activity observed might be due to variation in the plastid genome copy numbers. It is known that plastid genome copy numbers vary significantly in different tissues, with only 5% observed in roots compared to leaves (Sasaki et al 1990). However, the high BADH enzyme activity observed in carrot tap root (75% of leaves) may be due to the large number of chromoplasts present; this was evident by their orange or purple color, instead of colorless roots normally observed in plants that contain only 5% of plastid copies.

### BADH protein expression in carrot cells, root and shoot.

To further confirm the results of BADH activity in cells, tap roots and leaves, western blot analysis was done using crude extracts prepared from transformed and untransformed carrot tissues and a fraction from each sample (50 µg protein) was subjected to 10% SDS-PAGE. Protein transferred to nitrocellulose membrane was hybridized with polyclonal anti-BADH serum, raised in rabbits against native BADH (kindly provided by Dr. Elisa Soto; Figueroa-Soto et al 1999) and antigenic peptides were detected using horseradish peroxidase-linked secondary antibody. No *badh* expression was detected in untransformed carrot tissues (cells, tap roots and leaves; lanes 1-3). However, in transgenic samples (Fig. 5B), higher expression was observed in leaves (lane 6) and tap roots (lane 7) compared to carrot cell suspension cultures (lane 4). BADH protein accumulation in carrot root and leaf tissues were parallel to the results obtained with BADH enzyme activity in transgenic roots and shoots.

### Salt tolerance and BADH activity in cell suspension cultures of carrot.

In order to test whether salt stress affects the BADH activity, experiments were performed under different salt concentrations (0-500 mM NaCl) on carrot cell suspension cultures. Study performed for two weeks on carrot cells showed that transformed cells were able to sustain and proliferate in higher concentrations of NaCl in the liquid medium than untransformed cells (Fig. 6A-B). Using 500 mL conical flask containing 100 ml medium, carrot cultures produced about 11. 82 g cells in both transformed and untransformed (1475%) while, in presence of 100 mM NaCl, 8.75 g transgenic cells (1096%) and 1.29 g untransformed cells (161%) were obtained after two weeks (when initial culture used contained 0.8 g per flask, see Fig. 6A-B). Furthermore, BADH enzyme activity was stimulated in transgenic carrot cell cultures when they were exposed to 100 to 300 mM NaCl. Maximum noticeable BADH activity was seen in 100 and 200 mM NaCl (Fig. 6C) and such increase was insignificant in untransformed cells. This shows that full length *Prrn* promoter and gene *10* 5' UTR facilitate efficient transcription and translation in all tissues, irrespective of the developmental stage, despite low copy number of plastid genomes in non-green cells or roots.

### Effect of salt on carrot plants.

Different salt concentrations (100-500 mM NaCl) were tested on transformed and untransformed carrot plants transferred to soil in pots. Transgenic plants carrying the *badh* transgene thrive well up to 400 mM NaCl (Fig. 7) whereas, untransformed plants could not survive in pots after two weeks over 200 mM NaCl.

This application, along with the knowledge of the art, provides the necessary guidance and instructions to engineer the plastid genome of several major crops in which regeneration is mediated through somatic embryogenesis. Cereal crops (wheat, rice, corn, sugarcane), legumes (soybean, alfalfa), oil crops (sunflower, olive), cash crops (cotton, coffee, tea, rubber, flex, cork oak, pines), vegetables (eggplant, cucumber, cassava, chili pepper, asparagus etc.), fruits (apple, cherry, banana, plantain, melons, grape, guava), nuts (cashew, walnuts, peanuts), and trees (date palm etc.) are regenerated through somatic embryogenesis.

Previously, chloroplast transformation has been reported through bombardment of large number of leaf explants to obtain few transgenics. For example, in potato out of 200 bombardments only 2 transgenic shoots were recovered (Sidorov, 1999) after prolonged tissue culture. Authors reported that transformation of tomato plastids took almost two years (Ruf etal 2001). In contrast, stable transgene integration was confirmed in seven transgenic cell lines of carrot within 8-10 weeks from five bombardments and homolplasmy was achieved in cell cultures by repetitive 8-10 weekly subcultures in liquid medium. Carrot transgenic plants (ready to go in pots) were generated within a month from homoplasmic cell lines. Such high frequency of transformation obtained in carrot for transgene integration into plastid genomes might be due to several reasons. Long flanking sequences for homologous recombination were used from the same species i.e. native chloroplast genome of carrot (*Daucus carota* L. cv. Half long). In contrast, chloroplast vectors used for plastid transformation in both tomato and potato contained flanking sequences from the tobacco chloroplast genome. Similarly, when petunia chloroplast flanking sequences were used to transform the tobacco chloroplast genome (DeGray etal 2001), it resulted in very low transformation efficiency. Therefore, in order to advance this field of research, chloroplast genomes of useful crops should be sequenced. It is quite challenging to obtain flanking sequences for homologous recombination when no chloroplast DNA sequence is available in the Genbank (as it was the case for carrot).

Another significant observation in this study is high levels of transgene expression in proplastids of cultured carrot cells. Sidorov et al reported 50-fold less GFP accumulation in amyloplasts of potato tubers compared to leaves. In sharp contrast we report 53% BADH activity observe in cells comparison to leaves. This is the first report of transgene expression in proplastids even though chromoplasts have been reported to accumulate reasonable amounts of foreign gene products (Ruf et al 2001). The heterologous gene 10 5' UTR that regulates translation of *badh* gene was indeed promoterless. Chloroplast psbA 3' UTR is known to stabilize transcripts and is poor in transcription termination, with 30% termination efficiency. However, if higher levels of expression are desired in proplastids (such as corn seeds or in roots for nematode resistance), it may be desirable to insert a promoter upstream of gene 10 5'UTR to enhance transcription.

In order to achieve homoplasmy in carrot system, several rounds of selection (8-10) were required along with high selection pressure. In liquid culture, all surfaces of carrot cells are directly exposed to the growth medium. This implies a high selection pressure, which might possibly block the growth of untransformed plastids. Carrot cells in liquid medium multiply fast compared to solid medium containing 2,4-D. Once 2,4-D is removed from the carrot cells, they rapidly convert into mature somatic embryos and profusely give rise to plantlets. Yet another requirement to achieve homoplasmy is the availability of templates for integration into thousands of plastid genomes per cell. We have previously shown that the use of chloroplast flanking sequence that contain the complete chloroplast origin of replication offers large number of templates within chloroplasts (Daniell et al., 1990) and helps to achieve homoplasmy even in the first round of selection (Guda et al., 2000). Inclusion of the chloroplast origin of replication within the carrot chloroplast vectors (assuming that the *ori* is present in the same location as in other plant species) should have helped achieve homoplasmy within a few rounds of cell division.

Carrot cells have a great potential for rapid proliferation through somatic embryos, which produce secondary somatic embryos (recurrent embryogenesis) until exposed to maturation medium for conversion into plants and this culture can be maintained for several years in in vitro culture. In *Daucus carota* L. cv. US-*Harumakigosun*, embryogenic carrot calli maintained on suitable growth medium for five year produced a large number of plants (4x10⁷ plantlets/*L*-medium/day) continuously for 245 days without any decrease in the productivity (Nagamori etal 1999) through liquid cell culture using a rotating bioreactor. Another advantage with somatic embryos is that they can be used as synthetic seeds. Synthetic or artificial seeds have been defined as somatic embryos encapsulated in sodium aliginate beads or matrix for use in the commercial propagation of plants. For example, carrot somatic embryos encapsulated in alginate-gellan gum (dehydrated to 15% RH and rehydrated in moistured air to 90% RH) germinated up to 73% in soil (Timbert etal 1996) and the frequency of immobilized cell regeneration was improved about 1.5 times higher than that obtained through non-immobilized cells (Nagamori etal 1999). With synthetic seed technology, desirable elite genotypes in carrot and other plant species can be cryopreserved for long time (Tessereau etal 1994) and on demand these propagules can be used for synchronized planting of plants in the greenhouse or field. A single source for production in highly desired to minimize heterogeneity of therapeutic proteins.

Glycine betaine (GB) is a commonly occurring compound in all-living organisms and studies suggests that many higher plants, bacteria, marine and mammalian species accumulate GB under water deficiency or salt stress. In plants, bacteria and in mammalian kidney tissues, GB acts as an osmoprotectant. In porcine kidney BADH is localized in cortex and in the inner medulla, which is specifically localized on cells surrounding the tubules that are exposed to urea and high salt compounds (Figueroa-Soto etal 1999). Glycine betaine is one of the major compatible organic osmolytes identified in renal inner medulla of rats, which help renal cells to survive and function normally, despite being exposed to hypertonicity and lethal levels of urea.

These facts suggest that the presence of the BADH enzyme or accumulation of GB would be beneficial in foods consumed by humans or animals. In plants, salinity stress is a continuing and increasingly deleterious to the growth and yield of crop plants, owing to irrigation practices and increasing demands on fresh water supply; therefore, engineering of salt-tolerant crop plants has been a long-held and intensively sought objective (Apse MP and Blumwald E, 2002). One effective mechanism to reduce damage from these stresses is the accumulation of high intracellular levels of osmoprotectant compounds like glycine betaine in plants through genetic engineering (Rontein etal 2002).

### Experimental protocol.

### Construction of plastid transformation vectors.

DNA fragment representing carrot flanking sequence was amplified from carrot genomic DNA that was isolated from the leaves using DNeasy Plant mini kit (Qiagen Inc.) following manufacturer's protocol. The flanking sequence fragment was amplified with the primers generated from the tobacco chloroplast genome sequence, using Platinum Pfx DNA polymerase (Invitrogen Inc.). The amplified fragment represents the 16S/*trn*I-*trn*A123S region of the chloroplast genome and is approximately 4.2 kb in size. The PCR amplified DNA fragment was treated with T4 polynucleotide kinase (Promega) and cloned into PvuII digested pBluescript II KS, dephosphorylated with Shrimp Alakaline phosophatase (Promega). The kinase and dephosphorylation reactions were performed as per the manufacturer's instructions. The chloroplast promoters and regulatory sequences were amplified using PCR based on the information available for the tobacco chloroplast genome (Accession number - NC-001879). The carrot specific chloroplast transformation. vector pDD-*Dc-gfp*/*BADH* (Fig. 1 A) was constructed by inserting a blunt ended Cla-SacI fragment representing the *gfp*/*BADH* expression cassette into Pvull site of carrot chloroplast DNA flanking sequences. The *sm gfp* gene was obtained from TAIR. Similarly, the carrot chloroplast transformation vector pDD*-Dc-aadA*/*BADH* (Fig. 1B) was constructed by inserting blunt ended Apal fragment representing the aadA/BADH expression cassette into carrot chloroplast DNA flanking sequences at the PvuII site, after dephoshorylation. Bacterial and DNA manipulations were performed as per standard molecular biology protocols.

Of note, a potential vector for use is the double barrel plastid vector as described in WO 03/057834 A2, filed December 26, 2002. For purposes of simplicity we have included particular passages which describe the chloroplast double vector. The description provided below will help in the understanding of the plasmids shown in Fig 9-28 of this Application.

Chloroplast transformation has been accomplished only in a few Solanaceous crops so far. There are several challenges in extending this technology to other crops. So far, only green chloroplasts have been transformed in which the leaf has been used as the explant. However, for many crops, including monocots, cultured non-green cells or other non-green plant parts are used as explants. These non-green tissues contain proplastids instead of chloroplasts, in which gene expression and gene regulation systems are quite different. During transformation, transformed proplastids should develop into mature chloroplasts and transformed cells should survive the selection process during all stages of development. Therefore, the major challenge is to provide chloroplasts the ability to survive selection in the light and the dark, at different developmental stages. This is absolutely critical because only one or two chloroplasts are transformed in a plant cell and these plastids should have the ability to survive the selection pressure, multiply and establish themselves while all other untransformed plastids are eliminated in the selection process. The Double Barrel Plastid Vectors accomplish this by using genes coding for two different enzymes capable of detoxifying the same selectable marker (or spectrum of selectable markers), driven by regulatory signals that are functional in proplastids as well as in mature chloroplasts.

The plastid vector described herein is one of several such non-limiting examples. The chloroplast flanking sequence contains appropriate coding sequences and a spacer region into which the transgene cassette is inserted. Any spacer sequence within the plastid genome could be targeted for transgene integration, including transcribed and transcriptionally silent spacer regions. Both aphA-6 and aphA-2 (nptII) genes code for enzymes that belong to the aminoglycoside phosphotransferase family but they originate from different prokaryotic organisms. Because of prokaryotic nature of the chloroplast genome, these genes are ideal for use in transgenic chloroplasts without any codon optimization. Genes of prokaryotic origin have been expressed at very high levels in transgenic chloroplasts (up to 47% of total soluble protein, DeCosa et al., 2001). Both enzymes have similar catalytic activity but the aphA-6 gene product has an extended ability to detoxify kanamycin and provides a wider spectrum of aminoglycoside detoxification, including amikacin. The advantage of choosing kanamycin as a selectable marker is that it has no natural resistance, unlike spectinomycin resistance observed in most monocots or spontaneous point mutation of the 16 S rRNA gene observed during the selection process. In addition, kanamycin is not in human clinical use as an antibiotic and several crops containing kanamycin resistant nuclear transgenes have been already approved by FDA for human consumption (e.g. flavor savor tomatoes) and currently in the market place.

As shown in Fig 36, in this non-limiting example, all transgenes are regulated by the plastid Prrn promoter; this 16S rRNA promoter drives the entire rRNA operon in the native chloroplast genome and contains binding sites for both the nuclear encoded and plastid encoded RNA polymerases. Therefore, this promoter is capable of functioning in both proplastids and chloroplasts (green and non-green, in the light and dark). The aphA-6 gene is further regulated by the gene 10 5' UTR capable of efficient translation in the dark, in proplastids present in non-green tissues (see GFP expression in proplastids of non-green cells of corn and carrot in Figs 32 and 2 regulated by the 16S rRNA promoter and gene 10 UTR). The rps16 3' UTR has been used to stabilize aphA-6 gene transcripts. The aphA-2 (nptII8) gene, on the other hand is regulated by the psbA promoter, 5' and 3' UTRs, which are light regulated and highly efficient in the light, in chloroplasts (see A. Fernandez-San Millan, A. Mingeo-Castel, M. Miller and H. Daniell, 2003, A chloroplast transgenic approach to hyper-express and purify Human Serum Albumin, a protein highly susceptible to proteolytic degradation. Plant Biotechnology Journal, in press; also see WO 01/72959). Therefore, a combination of both aphA-6 and aphA-2 genes, driven by regulatory signals in the light and in the dark in both proplastids and chloroplasts, provides continuous protection for transformed plastids/chloroplasts around the clock from the selectable agent. The gene(s) of interest with appropriate regulatory signals (gene X) are inserted downstream or upstream of the double barrel selectable system. Because multiple genes are inserted within spacer regions (DeCosa et al 2001, Daniell & Dhingra, 2002), the number of transgenes inserted does not pose problems in transcription, transcript processing or translation of operons (WO 01/64024). In a variation of this example, aphA-6 and aphA-2 genes, coupled with different transgenes are inserted at different spacer regions within the same chloroplast genome using appropriate flanking sequences and introduced via co-transformation of both vectors

### Plant material transformation and regeneration of transgenic plants.

Sterile carrot plants (*Daucus carota* L. cv. Half long) were raised in plant tissue culture tubes containing MS salts (Murashige and Skoog, 1962), B5 vitamins (Gamborg et al 1968), 2% sucrose and 0.8% agar in the medium. The hypocotyls were cut into 0.5 mm segments and placed in 50 ml MSB liquid medium containing 3% sucrose, 0.1 mg/12,4-dichlorophenoxyacetic acid (2,4-D) and pH 5.7. After 3 weeks of continuous shaking at 26±2°C and 120 rpm, liberated cells were collected on a 100 µM mesh, centrifuged (150 x g for 10 min) and resuspended in fresh medium. Rapidly growing homogenous yellow cells were subcultured weekly. Fine cell suspension culture of carrot, filtered through a 100 µM mesh, were evenly spread on MSB solid medium supplemented with 3 mg/L 2,4-D and 1 mg/L kinetin and bombarded with carrot specific plastid vectors (Fig. 1A-B). Bombarded calli incubated for 2 days in the dark were selected on MSB medium containing 3 mg/L 2,4-D, 1 mg/L kinetin and different concentrations of betaine aldehyde (10, 15, 20 and 25 mM BA) and spectinomycin (150, 250, 350 and 450 mg/L). Cultures were incubated in 16/8 h day/night cycle at 50-1001x light intensity and 26±2°C temperature. Transgenic cultures were multiplied using both solid and liquid medium supplemented with selection agent. Transgenic plants produced through somatic embryogenesis (on MSB medium containing 0.2 mg/L) were transferred to soil in pots. The pots were covered with plastic bags to maintain high humidity for the first week and irrigated with progressively reduced concentrations of MS salts for the first week, followed by tap water in the second week.

**DNA extraction, PCR and Southern blot analysis.** Total genomic DNA was isolated using a Plant DNeasy kit (Quiagen Inc. USA) for PCR and Southern blot analysis of transgenic carrot. PCR reactions were performed by denaturing 50 ng DNA template at 94°C for 5 min, running 25 PCR cycles (1min at 94°C, 1min at 64°C, 3min at 72°C), final extension at 72°C for 10 min, using Taq DNA polymerase with 10x PCR buffer and primers pairs 3P/3M (land on flanking sequence/land on *aadA* gene) and 16SF/1M (landing on the native chloroplast genome/ the *aadA* gene).

For Southern blot analysis, plant DNA was digested with PvuII and AflIII and transferred to nylon membranes for hybridizing with a 4.9 kb probe, generated by digesting *pDD-Dc-aadA*/*badh* vector DNA with PvuII and AflIII and labeled with ³²P using the ProbeQuant G-50 Columns (Amersham, USA). Blot was hybridized with probe using the Stratagene Quick-HYB hybridization solution and vender's instructions (Stratagene, USA).

**BADH enzyme activity and immunoblot analysis.** Extraction and assay for BADH (Betaine aldehyde dehydrogenase) activity was done as described (Daniell etal 2001). 1 g carrot tissues were grounded in 2 mL buffer containing 50 mM Hepes-KOH (pH 8.0), 1 mM EDTA, 20 mM Sodium metabisulfite, 10 mM Sodium borate, 5 mM ascorbic acid and 5 mM DTT. Crude extract was centrifuged at 10, 000 x g at 4°C for 10 min and the supernatant was desalted using Sephadex G-25 Columns (Amersham Pharmacia biotech, USA). NAD⁺ reduction by BADH was measured spectrophotometrically at 340 nm after 1 min and 10 min in 1 mL assay buffer (50 mM Hepes-KOH, pH zu 1mM EDTA, 5 mM DTT, 1 mM NAD⁺) added with 1 mM BA at 25°C to start reaction.

For immunoblot analysis total soluble protein was isolated using 2x Laemmli buffer from 100 mg carrot tissues. The mixture was boiled for 5 min and centrifuged for 5 min at 10,000 x g. Supernatant containing 50 jig total soluble protein (quantified with Bradford assay) was loaded on a 10% SDS-PAGE gel and transferred on blotting membrane. Membrane was hybridized with polyclonal anti-BADH serum, raised in rabbits against BADH (provided by Dr. Elisa Soto). Hybridized peptides were detected using horseradish peroxidase-linked secondary antibody, using Lumi-Phos™ WB chemiluminescent reagent (Pierce, USA).

**Analysis of transgenic plants for salt tolerance.** Transgenic and non-transgenic carrot plants transferred to soil in pots of same morphogenic growth phase and height were analyzed for salt tolerance containing 0, 100, 200, 300, 400 and 500 mM NaCl, respectively. Plants were maintained in growth chamber and irrigated daily with saline water containing different levels of salt for one month.

### ILLUSTRATIVE EXAMPLE 2 COTTON TRANSFORMATION

### Material and Methods

Plant material and transformation: Delinted cotton (Gossypium hirsutum L. cv. Coker310FR) seeds were sterilized by dipping in 70% ethanol for 2 minutes followed an 8 minutes treatment with sodium hypochlorite solution containing approximately 4% available chlorine and then by treatment with 0.1 % mercuric chloride solution (w/v) for 5 minutes. After surface sterilization and four to five washes with sterile water, seeds were kept in sterile water for 4-5 hours for softening the seed coat which was completely removed before the seeds were placed on 1/2 MSB medium containing half strength MS salts (Murashige and Skoog, 1962) and B5 vitamins (Gamborg etal 1968) with 1.5% sucrose. Hypocotyl explants (4-6 mm long) of 5 day old seedlings were placed vertically on MST1 medium (containing MS salts, B5 vitamins, 0.1 mg/l 2,4-D, 0.5 mg/l kinetin and 3% glucose) for the induction of callus. Uniformly distributed pro-embryognic callus were bombarded with gold particle coated with chloroplast vector using the Helium-based biolistics particle gun (Bio-Rad). The transformation of cotton callus was optimized using the parameters: 0.6µm gold particles macro-carrier; 27 in. Hg chamber vacuum; 1550 psi Helium pressure; 6 mm rupture disc macro-carrier gap; 6 mm macro-carrier flying distance, 6 cm target distance and 10 µl chloroplast vector coated on gold particles. Cultures after bombardment were incubated in dark for 24 h and thereafter transferred to selection medium MST1 supplemented with 50 mg/l kanamycin and incubated in 16/8 h day/night cycle at 750 lux light intensity and 28±2°C temperature. Transgenic embryogenic cultures were multiplied on MST1 medium supplemented with 50 mg/l kanamycin (Fig 34) and transgene aphA-6 gene integration in cultures was confirmed by PCR (Fig 35).

### ILLUSTRATIVE EXAMPLE 3 EXPRESSION CASSETTE CONSTRUCTION

### Materials and Methods:

**Amplification and cloning of flanking sequences:** DNA fragment representing flanking sequences were amplified from plant genomic DNA that was isolated from the leaves using Qiagen plant extraction kit following manufacturer's protocol. The flanking sequence fragment was amplified with the primers, ADLF - 5' gtgtcagtgtcggcccagcagag 3' and ADLR - 5' aacaggggtcaaggtcggccag 3' using Platinum Pfx DNA polymerase (Invitrogen Inc.). The amplified fragment represents the 16S/*trn*I-*trn*A/23S region of the chloroplast genome and is approximately 4.2 kb in size. The PCR amplified DNA fragment was treated with T4 polynucleotide kinase (Promega) and cloned into PvuII digested pBluescript II KS dephosphorylated with Shrimp Alakaline phosophatase (Promega). The kinase and dephosphorylation reactions were performed as per the manufacturer's instructions. The clone harboring carrot specific flanking region was designated as pDA-35.

**Construction of the expression cassettes:** pDA-29 is a chloroplast specific expression cassette cloned in pBluescript II KS that carries the *aad*A gene conferring resistance to spectinomycin and streptomycin and *badh* gene that metabolizes the breakdown of toxic betaine aldehyde to glycinebetaine. Expression of the first gene is driven by the 16S Prrn promoter, under the regulation of a Shine-Dalgarno sequence at the 5' end and *psbA* 3' UTR at the 3' end. Expression of *badh* gene is regulated by heterologous T7 gene 10 5'UTR and rps16 3'UTR. The *aad*A gene was derived from pLD-CtV (Daniell et al. 1998) and the *badh* gene was derived from pLD-BADH (Daniell et al. 2001). Second chloroplast expression cassette pDA-30 carries the *sm-gfp* gene encoding for soluble modified green fluorescent protein (obtained from TAIR) and the *badh* gene. Expression of *sm*-*gfp* is driven by the 16S Prrn promoter and is regulated by heterologous T7 gene 10 5'UTR and rps16 3'UTR. The expression of *badh* gene is regulated by *psb*A promoter/5' UTR and 3' UTR. The promoters and regulatory sequences were amplified using PCR based on the information available for tobacco chloroplast genome (Shinozaki et al. 1987).

**Construction of chloroplast transformation vectors:** Chloroplast transformation vector pDD-XX-*aad*A/*badh* is a derivative of pDA vectors that harbor the flanking sequences with the pDA-29 expression cassette. The expression cassette from pDA-29 was obtained as an *Apa*I fragment, blunt-ended using klenow DNA polymerase (NEB) as per manufacturer's instructions and cloned into *Pvu*II digested and dephosphorylated pDA-35. The other species specific chloroplast transformation vector pDD-XX-*gfp*/*badh* harbors the carrot flanking sequence and the pDA-30 expression cassette. The expression cassette from pDA-30 was derived as a *Cla*I*lSac*I fragment, blunt-ended and cloned into *Pvu*II digested and dephosphorylated pDA-35. Bacterial and DNA manipulations were performed as per standard molecular biology protocols.

### ILLUSTRATIVE EXAMPLE 4: CORN TRANSFORMATION

For genetic engineering of the corn chloroplast genome, corn specific sequences, flanking the targeted integration site in the corn chloroplast genome (trnI and trnA) were amplified with specific PCR primers and subcloned to flank the betaine aldehyde dehydrogenase (BADH) selectable marker, and green fluorescent protein (GFP) reporter gene expression cassette.

Callus cultures were initiated from aseptically excised immature zygotic embryos (1-2 mm in length), produced on self-pollinated ears of HiII (F1) maize plants. Ears were surface sterilized in a solution containing 2.6% Sodium hypochlorite (prepared with commercial bleach) containing .1% Tween 20 (polyoxyethylene sorbitan monolaurate) for 20 miniutes under continuous shaking, then rinsed 4 timesin sterile distilled water. The Embryos were then placed on the callus induction medium CI-1, which contained N6 salts and vitamins (463.0mg/l (NH4)2SO4, 2830.0mg/lKNO3, 400mg/l KH2PO4, 166.0 mg/l CaCl2, 185 mg/l MgSO4. 7H2O, 37.3 mg/l Na2-EDTA, 27.85mg/l FeSO4.7H2O, 1.6 mg/l H3BO3, 4.4 mg/l MnSO4.H20, .8, KI, 1.5mg/l ZnSO4. 7H2O), 2% sucrose and 1.0 mg/l 2,4-D (2,4 dichloro-phenoxy acetic acid), with the rounded scutellar side exposed and the flat plumule-radicle axis side in contact with the medium. Callus cultures were maintained in darkness at 25-28 °C and subcultured every two weeks.

### Particle bombardment of embryogenic calli

Micro projectiles were coated with DNA (pDA34-ZM-gfp-BADH and pDA33-ZM-aadA-BADH) and bombardment was carried out with the biolistic device PDS1000/He (Bio-Rad).

Prior to bombardment, embryogenic calli were selected, transferred over sterile filter paper (Watman No.1), and placed on the surface of a fresh medium in standard Petrti plates (100x 15mm). Gold particles (0.6µm) were then coated with plasmid DNA as follows: 50µl of washed gold particles were mixed with 10µl DNA (1µg/µl), 50µl of 2.5M CaCl2, 20µl of 0.1M spermidine and vortexed. Particles were cneterfuged for a few seconds at 3000rpm and then the ethanol was poured off. Ethanol washing was repeated five times, then the pellet was resuspended in 30µl of 100% ethanol and placed on ice until it was used for bombardment (the coated particles were used within 2hours). Bombardment was carried out with the biolistic device PDS1000/He (Bio Rad) by loading the target sample at level 2 in the sample chamber under a partial vacuum (28 inches Hg).

The callus cultures were bombarded with the maize chloroplast transformation vectors using 1100 psi rupture discs. Following bombardment, the explants were transferred to a fresh medium; plates were sealed with micropore tape and incubated in darkness at 25-28 °C.

### Selection

Selection was intiated two days after bombardment. The bombarded calli were transferred to callus induction medium containing 5-20mM BA (betaine aldehyde) or 25-100mg/l streptomycin. Selection was also carried out using 50-150 mM NaCl in combination with the BA to maintain osmostic pressure.

### Regeneration

Regeneration was initiated 6 to 8 weeks after bombardment by transferring the calli to a medium R1 containing Ms salts and vitamins supplemented with 1.0 mg/l NAA (*α*-naphthalene acetic acid),2% sucrose, 2g/l myoinositol and .3% phytagel at pH 5.8. Regenerated plants were transferred to R2 containing ½ MS salts and vitamins, 3% sucrose and .3% phytagel at pH 5.8. Regenerated plants were maintained in light (16/8 hr photoperiod).

### Shoot multiplication

### The surface steriliztion and germination of corn seeds

Corn seeds were surface sterilized in a solution containg 2.6% Sodium hypochlorite (prepared from commercial bleach) containing .1% Tween 20 for 20 minutes under continuos shaking, then rinsed four times in sterile distilled water. Seeds were grown on MS medium at pH 5.8 in darkness. Nodal sections were excised aseptically from three day old seedlings. The nodal sections appear as clear demarcations on the germinated seedlings and represent the seventh node. When excised, the nodal cross sections are 1.3 to 1.5 mm in length.

### Particle bombardment of nodal sections

Prior to bombardment, 20-30 nodal sections were placed in the center of each petri plate with acropitila end up. Bombardment was carried out with the maize chloroplast vectors, using 1100, 1300 and 1550 psi rupture discs.

### Multiple shoot induction and selection

Nodal section explants are placed acropital end up on shoot multiplication medium SM1 composed of Ms salts and vitamins, 1.0 mg/l 6BA (6-Benzyl amino purine), 3% sucrose and 5g/l phytagel at pH 5.8 under continuous light at 25°C. Initiation of the shoot-tip clumps from the original shoot tips occurred 2 to 4 weeks after culture. Two days after bombardment, transformed nodal sections were transferred to shoot multiplicaton medium containing 5-20mM BA or 50-100 mg/l streptomycin selective agents. Subsequent subcultures at two week intervals were carried out by selecting, dividing and subculturing green clumps on selective shoot multiplication medium containing 5-20mM BA or 25-100 mg/l streptomycin.

### Regeneration

The Multiple shoot clumps were regenerated by transferring them to regeneration medium M1 containing MS salts and Vitamins, 5 mg/l IBA and 3% sucrose at pH 5.8. The developed shoots were regenerated by transferring the shoot tip clumps to M2 medium containing ½ MS salts and vitamins, 3 % sucrose and 3g/l phytagel at pH 5.8. It should be further rnoted that all the regeneration media are supplemented with 5-20mM BA or 25-100mg/l streptomycin as the selective agents.

To engineer the corn chloroplast genome free of antibiotic resistance genes, maize calli were bombarded with a chloroplast expression vector containing the green fluorescent protein (GFP) and the betaine adehyde dehydrogenase (BADH) genes as selectable or screenable markers. To compare the betaine aldehyde (BA) selection with streptomycin, another chloroplast expression vector was constructed containing the aadA and the BADH genes. The number of putative transgenic events was higher on BA selection than on streptomycin. Transgenic corn tissues screened on BA were examined using a laser-scanning confocal microscope. The GFP fluorescence was observed throughout the somatic embryos of corn. Chloroplast transformation of corn provides a suitable avenue for the production of edible vaccines and oral delivery of biopharmaceuticals.

### Corn chloroplast transformation vector

Corn chloroplast transformation vector facilitates the integration of transgene into the inverted repeat (IR) region of the corn chloroplast genome. The vector pLD-Corn-BADH contains the chimeric aadA gene and the BADH gene driven by the constitutive 16 S rRNA promoter and regulated by the 3' UTR region of psbA gene from petunia plastid genome. In this construct both, aadA and BADH possess the chloroplast preferred ribosomal binding site, GGAGG. Another vector used for corn chloroplast transformation pLD-corn-UTR-BADH has the constitutive 16 S rRNA promoter driving the expression of the dicistron, but BADH is under the regulation of the promoter and the 5' UTR of the psbA gene and the 3' UTR of psbA gene, for enhanced expression. Since the expression of the foreign protein is desired in chromoplasts of corn seeds, the gene of interest needs to be under the control of a regulatory sequence that is free from cellular control. In this context, examples of suitable candidate regulatory sequences are the T7 gene 10-leader sequence and cry2Aa2 UTR. The T7 gene 10-leader sequence is used to express foreign proteins in transgenic chromoplasts. The cry2Aa2 UTR has been shown by the inventor to accumulate as much foreign protein in chromoplasts as efficient as the psbA UTR in green tissues. Therefore the selectable marker for additional vectors use the BADH gene under the regulation of psbA promoter and 5'UTR, as psbA is one of the most efficiently translated chloroplast genes in green tissues. When green tissue or non-green embryogenic calli are used for introducing the transgene into the corn chloroplast genome, it is preferred to use the light regulated psbA promoter/ UTR or 16 S rRNA promoter/gene 10 UTR, respectively.

The ability to transform any of a number of plants so that these plants perform embryo genesis is further bolstered by the number of exemplary references listed below which illustrate a methodology of plant transformation through somatic embryogenesis in a wide variety of plants. The following references are examples of regeneration of crops via embryogenesis and/or a crop/plant transformation via the nuclear genome. As a result, one skilled in the art can use the protocols for somatic embryogenesis described in the exemplary references below, in concert with the chloroplast vectors described in the specification, to achieve plastid transformation through non-green plant cells. In other word, one skilled the art will recognize that the protocols below are applicable to the description in the content of the specification. This following list of references describe plant transformation through somatic embryogenesis:

### Crops:

### Maize:

J. F. Petolino, N. L. Hopkins, B. D. Kosegi, M. Skokut genetic transformation and hybridization: Whisker-mediated transformation of embryogenic callus of maize. 19: 781-786 (2000).

### Soybean:

H. N. Trick , J. J. Finer. Sonication-assisted Agrobacterium-mediated transformation of soybean [Glycine max (L.) Merrill] embryogenic suspension culture tissue. Plant Cell Reports 17: 482-488 (1998).

### Wheat:

A. Pellegrineschi , R. M. Brito , L. Velazquez , L. M. Noguera , W. Pfeiffer , S. McLean , D. Hoisington. The effect of pretreatment with mild heat and drought stresses on the explant and biolistic transformation frequency of three durum wheat cultivars. Plant Cell Rep 20: 955-960 (2002).

### Barley:

M. Manoharan , L. S. Dahleen. Genetic transformation of the commercial barley (Hordeum vulgare L.) cultivar Conlon by particle bombardment of callus Plant Cell Rep 21: 76-80 (2002).

Meyeong-Je C., Wen J. and Lemaux P.G. Transformation of recalcitrant barley cultivars through improvement of regenerability and decreased albinism. Plant Science 138: 229-244.

### Rice:

Sung-Ho Lee, Nigel W. Blackhall, J. Brian Power, Edward C. Cocking, David Tepfer and Michael R. Davey. Genetic and morphological transformation of rice with the rolA gene from the Ri TL-DNA of Agrobacterium rhizogenes, Plant Science, 161: 917-925 (2001).

### Sunflower:

S. Weber , W. Friedt, N. Landes, J. Molinier , C. Himber , P. Rousselin , G. Hahne , R. Horn. Improved Agrobacterium -mediated transformation of sunflower ( Helianthus annuus L.): assessment of macerating enzymes and sonication. Plant Cell Rep 21: 475-482 (2003).

### Cotton:

B. Chaudhary, S. Kumar, K. V. S. K. Prasad , G. S. Oinam , P. K. Burma, D. Pental. Slow desiccation leads to high-frequency shoot recovery from transformed somatic embryos of cotton (Gossypium hirsutum L. cv. Coker 310 FR). Plant Cell Rep. 21:955-960(2003)

### Sugarcane:

Gil A. Enriquez-Obregon , Roberto I. Vazquez-Padron , Dmitri L. Prieto-Samsonov , Gustavo A. De la Riva , Guillermo Selman-Housein. Herbicide-resistant sugarcane (Saccharum officinarum L.) plants by Agrobacterium-mediated transformation Planta 206: 20-27 (1998).

### Sugar beet:

S. Kifle , M. Shao , C. Jung , D. Cai. An improved transformation protocol for studying gene expression in hairy roots of sugar beet (Beta vulgaris L.) Plant Cell Reports 18: 514-519 (1999).

### Banana:

T. R. Ganapathi , N. S. Higgs , P. J. Balint-Kurti , C. J. Arntzen, G. D. May, J. M. Van Eck. Agrobacterium -mediated transformation of embryogenic cell suspensions of the banana cultivar Rasthali (AAB) Plant Cell Reports 20: 157-162 (2001).

### Cassava:

P. Zhang , G. Legris , P. Coulin , J. Puonti-Kaerlas. Production of stably transformed cassava plants via particle bombardment. Plant Cell Reports 19: 939-945(2000).

### Oat:

H. F. Kaeppler, G. K. Menon, R. W. Skadsen, A. M. Nuutila, A. R. Carlson. Transgenic oat plants via visual selection of cells expressing green fluorescent protein. Plant Cell Reports 19: 661-666 (2000).

Meyeong-Je C., Wen J. and Lemaux P.G. High-frequency transformation of oat via microprojectile bombardment of seed-derived highly regenerative cultures. Plant Science 148: 9-17 (1999).

### Medicinal plants:

Selection of interspecific somatic hybrids of Medicago by using agrobacterium-transformed tissues, Plant Science, Volume 69, Issue 2, 1990, Pages 189-198
M. R. Thomas, L. B. Johnson and F. F. White Genetic transformation of mature Taxus: an approach to genetically control the in vitro production of the anticancer drug, taxol, Plant Science, Volume 95, Issue 2, 1994, Pages 187-196. Kyung-Hwan Han, Paul Fleming, Kevin Walker, Matthew Loper, W. Scott Chilton, Ursula Mocek, Milton P. Gordon and Heinz G. Floss Giri, A., Banerjee, S., Ahuja, P.S. and Giri, C.C., 1997. Production of hairy roots in Aconitum heterophyllum Wall. using Agrobacterium rhizogenes. In Vitro Cellular and Developmental Biology Plant 33 4, pp. 293-296.

Pradel, H., Dumkelehmann, U., Diettrich, B. and Luckner, M., 1997. Hairy root cultures of Digitalis lanata. Secondary metabolism and plant regeneration. J Plant Physiol 151, pp. 209-215.

### Beans, vegetable and fruits:

Regeneration of Fertile Plants from Callus in Phaseolus polyanthus Greenman (Year Bean), Annals of Botany, Volume 88, Issue 3, September 2001, Pages 371-377. Mukund Zambre, Pascal Geerts, Alain Maquet, Marc Van Montagu, Willy Dillen and Geert Angenon

Expression of a bifunctional green fluorescent protein (GFP) fusion marker under the control of three constitutive promoters and enhanced derivatives in transgenic grape (Vitis vinifera), Plant Science, Volume 160, Issue 5, April 2001, Pages 877-887. Zhijian Li, Subramanian Jayasankar and D. J. Gray

Agrobacterium-mediated genetic transformation of grapevine somatic embryos and regeneration of transgenic plants expressing the coat protein of grapevine chrome mosaic nepovirus (GCMV), Plant Science, Volume 102, Issue 2, 1994, Pages. 161-170. O. Le Gall, L. Torregrosa, Y. Danglot, T. Candresse and A. Bouquet

A reliable system for the transformation of cantaloupe charentais melon (Cucumis melo L. var. cantalupensis) leading to a majority of diploid regenerants, Scientia Horticulturae, Volume 84, Issues 1-2, 28 April 2000, Pages 91-99

Monique Guis, Mohamed Ben Amor, Alain Latché, Jean-Claude Pech and Jean-Paul Roustan

M.A. McLean and P.J. Charest, The regulation of transgenic trees in North America. Silvae Genet. 49 (2000), pp. 233-239. (For Papaya)

Regeneration of transgenic peanut plants from stably transformed embryogenic callus, Plant Science, Volume 93, Issues 1-2, 1993, Pages 185-194

Peggy Ozias-Akins, Jennifer A. Schnall, William F. Anderson, Chong Singsit, Thomas E. Clemente, Michael J. Adang and Arthur K. Weissinger

Transformation of cucumber tissues by microprojectile bombardment: identification of plants containing functional and non-functional transferred genes, Gene, Volume 118, Issue 2, 10 September 1992, Pages 255-260. Paula P. Chee and Jerry L. Slightom

H. Tsukazaki , Y. Kuginuki , R. Aida , T. Suzuki. Agrobacterium-mediated transformation of a doubled haploid line of cabbage. Plant Cell Rep (2002) 21: 257-262

### Flowers:

Biolistic Transformation of Rose (Rosa hybridaL.), Annals of Botany, Volume 81, Issue 1, January 1998, Pages 109-114. R. MARCHANT, J. B. POWER, J. A. LUCAS and M. R. DAVEY

Production of transgenic plants of the Liliaceous ornamental plant Agapanthus praecox ssp. orientalis (Leighton) Leighton via Agrobacterium-mediated transformation of embryogenic calli, Plant Science, Volume 161, Issue 1, June 2001, Pages 89-97. Sakae Suzuki, Kanyaratt Supaibulwatana, Masahiro Mii and Masaru Nakano

Somatic embryogenesis and Agrobacterium-mediated transformation of chrysanthemum, Plant Science, Volume 97, Issue 1, 1994, Pages 95-101. Daniela Pavingerová, Josef Dostál, Rena Bísková and Vojtch Benetka

### Trees:

High levels of expression of full-length cryIA(c) gene from Bacillus thuringiensis in transgenic somatic walnut embryos, Plant Science, Volume 131, Issue 2, 2 February 1998, Pages 181-193. Abhaya M. Dandekar, Gale H. McGranahan, Patrick V. Vail, Sandra L. Uratsu, Charles A. Leslie and J. Steven Tebbets

M. Delledonne et al., Transformation of white poplar (Populus alba L.) with a novel Arabidopsis thaliana cysteine proteinase inhibitor and analysis of insect pest resistance. Mol. Breed. 7 (2001), pp. 35-42.

L.H. Zhu et al., Transformation of the apple rootstock M.9/29 with the rolB gene and its influence on rooting and growth. Plant Sci. 160 (2001), pp. 433-439.

R.L. Bell et al., Transformation of 'Beurre Bosc' pear with the rolC gene. J. Am. Soc. Hort. Sci. 124 (1999), pp. 570-574.

C. El Euch et al., Expression of antisense chalcone synthase RNA in transgenic hybrid walnut microcuttings. Effect on flavonoid content and rooting ability. Plant Mol. Biol. 38 (1998), pp. 467-479.

R. Scorza et al., Post-transcriptional gene silencing in plum pox virus resistant transgenic European plum containing the plum pox potyvirus coat protein gene. Transgenic Res. 10 (2001), pp. 201-209.

J.L. Norelli et al., Transgenic 'Malling 26' apple expressing the attacin E gene has increased resistance to Erwinia amylovora. Euphytica 77 (1994), pp. 123-128.

J.P. Reynoird et al., First evidence for improved resistance to fire blight in transgenic pear expressing the attacin E gene from Hyalophora cecropia. Plant Sci. 149 (1999), pp. 23-31.

V. Levee et al., Stable genetic transformation of white pine (Pinus strobus L.) after cocultivation of embryogenic tissues with Agrobacterium tumefaciens. Mol. Breed. 5 (1999), pp. 429-440.

A.R. Wenck et al., High-efficiency Agrobacterium-mediated transformation of Norway spruce (Picea abies) and loblolly pine (Pinus taeda). Plant Mol. Biol. 39 (1999), pp. 407-416.

D.D. Ellis et al., Stable transformation of Picea glauca (white spruce) by particle acceleration. Bio/Technology 11 (1993), pp. 84-89.

M. Fladung , Gene stability in transgenic aspen (Populus). I. Flanking DNA sequences and T-DNA structure. Mol. Gen. Genet. 260 (1999), pp. 574-581.

Introduction and expression of marker genes in sandalwood (Santalum album L.) following Agrobacterium-mediated transformation, Plant Science, Volume 131, Issue 1, 15 January 1998, Pages 53-63.Veena Shiri and K. Sankara Rao

Regeneration of a transgenic woody legume (Robinia pseudoacacia L., black locust) and morphological alterations induced by Agrobacterium rhizogenes-mediated transformation, Plant Science, Volume 88, Issue 2, 1993, Pages 149-157. Kyung-Hwan Han, Daniel E. Keathley, John M. Davis and Milton P. Gordon

### Conifer species:

K. Klimaszewska , D. Lachance , M. Bernier-Cardou , R. G. Rutledge. Transgene integration patterns and expression levels in transgenic tissue lines of Picea mariana, P. glauca and P. abies. Plant Cell Reports published online first DOI10.1007/s00299-003-0626-5(2003).

V. Levee , M.-A. Lelu , L. Jouanin , D. Cornu, G. Pilate. Agrobacterium tumefaciens-mediated transformation of hybrid larch (Larix kaempferi T L. decidua) and transgenic plant regeneration. Plant Cell Reports 16: 680-685 (1997).

S.L. Bishop-Hurley , R.J. Zabkiewicz , L. Grace , R.C. Gardner , A. Wagner , C. Walter. Conifer genetic engineering: transgenic Pinus radiata (D. Don) and Picea abies (Karst) plants are resistant to the herbicide Buster. Plant Cell Reports 20: 235-243 (2001)

L. -N. Tian, P. J. Charest, A. Seguin, R. G. Rutledge. Hygromycin resistance is an effective selectable marker for biolistic transformation of black spruce (Picea mariana) 19: 358-362 (2000).

### Sweet potato:

Transformation of sweet potato (Ipomoea batatas (L.) Lam.) with Agrobacterium tumefaciens and regeneration of plants expressing cowpea trypsin inhibitor and snowdrop lectin, Plant Science, Volume 107, Issue 2, 1 June 1995, Pages 215-227. C. A. Newell, J. M. Lowe, A. Merryweather, L. M. Rooke and W. D. O. Hamilton

### Flax:

Transgenic flax plants from Agrobacterium mediated transformation: incidence of chimeric regenerants and inheritance of transgenic plants, Plant Science, Volume 91, Issue 2, 1993, Pages 139-148. Jin-Zhuo Dong and Alan McHughen

### Coffee:

T. Hatanaka , Y. E. Choi , T. Kusano , H. Sano. Transgenic plants of coffee Coffea canephora from embryogenic callus via Agrobacterium tumefaciens-mediated transformation. 19: 106-110 (1999).

### Rubber:

P Montoro, N Teinseree, W Rattana, P Kongsawadworakul and N Michaux-Ferrière (2000) Effect of exogenous calcium on Agrobacterium tumefaciens-mediated gene transfer in Hevea brasiliensis (rubber tree) friable calli. Plant Cell reports. 19: 851-855 (2000).

### Tea:

Mondal , Bhattacharya , Ahuja, Chand. Transgenic tea [Camellia sinensis (L.) O. Kuntze cv. Kangra Jat] plants obtained by Agrobacterium-mediated transformation of somatic embryos. Plant Cell Reports 20: 712-720 (2001)

### Cocoa:

Moxalactam as a counter-selection antibiotic for Agrobacterium-mediated transformation and its positive effects on Theobroma cacao somatic embryogenesis, Plant Science, Volume 164, Issue 4, April 2003, Pages 607-615. Gabriela Antúnez de Mayolo, Siela N. Maximova, Sharon Pishak and Mark J. Guiltinan

### REFERENCES

1. Apse MP and Blumwald E (2002) Engineering salt tolerance in plants. Current opinion in Biotechnol. 13: 146-150.
2. Arakawa K., Mizuno K., Kishitani, S. & Takabe T. Immunological studies of betaine aldehyde dehydrogenase in barley. Plant Cell Physiol. 33, 833-840 (1992
3. Bing-Kai Hou, Yi-Hua Zhou, Li-Hong Wan, Zhong-Lin Zhang, Gui-Fang Shen, Zheng-Hua Chen, Zan-Min Hu. Chloroplast Transformation in Oilseed Rape. Transgenic Res. 12, 111-114 (2003).
4. Corneille S, Lutz K, Svab Z and Maliga P (2001) Efficient elimination of selectable marker genes from the plastid genome by the CRE-lox site-specific recombination system. Plant J. 27: 171-178.
5. Daniell, H. & Dhingra, A. Multigene engineering: Dawn of an exciting new era in biotechnology. Curr. Opin.Biotechnol. 13,136-141(2002).
6. Daniell, H. et al. Transient foreign gene-expression in chloroplasts of cultured tobacco cells after biolistic delivery of chloroplast vectors. Proc. Natl. Acad. Sci. U. S. A. 87, 88-92 (1990).
7. Daniell, H. Medical molecular farming: expression of antibodies, biopharmaceuticals and edible vaccines via chloroplast genome (Vasil, I.K. ed.), Kluwer Academic Publishers, Netherlands, pp 371-376 (2003).
8. Daniell, H. Molecular strategies for gene containment in transgenic crops. Nature Biotechnol. 20, 581-586 (2002).
9. Daniell, H. Transformation and foreign gene expression in plants mediated by micoprojectile bombardment. Methods Mol. Biol. 62, 463-489 91997).
10. Daniell, H., Datta, R., Varma, S., Gray, S. and Lee, S.B. Containment of herbicide resistance through genetic engineering of the chloroplast genome. Nat. Biotechnol. 16, 345-348 (1998).
11. Daniell, H., Dhingra, A. & San-Milan, A.F. in 12th International Congress on Photosynthesis, Vol. S40-04 1-6 (CSIRO Publishing, Brisbane, Australia; 2001).
12. Daniell, H., Khan, M.S. and Alison L. Milestones in chloroplast genetic engineering: an environmentally friendly era in biotechnology. Trends Plant Sci. 7, 84-91 (2002).
13. Daniell, H., Lee, S.B., Panchal, T. & Wiebe, P.O. Expression of cholera toxin B subunit gene and assembly as functional oligomers in transgenic tobacco chloroplasts. J. Mol. Biol. 311,1001-1009 (2001).
14. Daniell, H., Muthukumar, B. and Lee, S.B. Marker free transgenic plants: engineering the chloroplast genome without the use of antibiotic selection. Curr. Genet. 39, 109-116 (2001).
15. DeCosa B., Moar W., Lee S.B., Miller M. and Daniell H. Overexpression of the Bt Cry2Aa2 operon in chloroplasts leads to formation of insecticidal crystals. Nat. Biotechnol. 19, 71-74 (2001).
16. DeGray, G., Kanniah, R., Franzine, S., John, S. and Daniell, H. (2001) Expression of an antimicrobial peptide via the chloroplast genome to control phytopathogenic bacteria and fungi. Plant Physiol. 127: 852-862.
17. E. Harlow and D. Lane. Antibodies: A Laboratory Manual, Cold Spring Harbor Laboratory, Cold Spring Harbor, NY (1988).
18. Eiji Nagamori, Hiroyuki Honda and Takeshi Kobayashi. Release of Embryogenic Carrot Cells with High Regeneration Potency from Immobilized Alginate Beads, Journal of Bioscience and Bioengineering, Volume 88, Issue 2, 1999, Pages 226-228
19. Esteban C. Serra and Nestor Carrillo. DNA polymerase activity of tomato fruit chromoplasts. FEBS Letters. 275, 102-106 (1990)
20. Fernandez-San Millan, A., Mingo-Castel, A. & Daniell, H. A chloroplast transgenic approach to hyper-express and purify human serum albumin, a protein highly susceptible to proteolytic degradation. Plant Biotechnol. J. 1,71-79 (2003).
21. Figueroa-Soto CG, Lopez-CervantesG and Valenzuela-Soto EM (1999) Immunolocalization of Betaine Aldehyde Dehydrogenase in Porcine Kidney Biochemical and Biophysical Research Communications. 258: 732-736.
22. Gamborg, O.L., Miller, R.A. & Ojima, K. Nutrient requirements of suspension cultures of soybean root cells. Exp. Cell Res. 50, 151-158 (1968).
23. Guda, C., Lee, S.B. & Daniell, H. Stable expression of biodegradable protein based polymer in tobacco chloroplasts. Plant Cell Rep. 19, 257-262 (2000).
24. H. Tessereau, B. Florin, M. C. Meschine, C. Thierry and V. Pétiard. Cryopreservation of Somatic Embryos: A Tool for Germplasm Storage and Commercial Delivery of Selected Plants. Ann. of Botany. 74, 547-555 (1994)
25. Hajdukiewicz PTJ, Gilbertson L and Staub JM (2001) Multiple pathways for Cre/lox-mediated recombination in plastids. Plant J. 27: 161-170.
26. Huang FC, Klaus SMJ, Herz S, Zou Z, Koop HU, Golds TJ (2002) Efficient plastid transformation in tobacco using the aphA-6 gene and kanamycin selection. Mol. Gen. Genomics 268: 19-27.
27. Iamtham S and Day A (2000): Removal of antibiotic resistance genes from transgenic tobacco plastids. Nat. Biotechnol. 18: 1172-1176.
28. Kota, M. et al. Overexpression of the Bacillus thuringiensis (Bt) Cry2Aa2 protein in chloroplasts confers resistance to plants against susceptible and Bt-resistant insects. Proc. Natl. Acad. Sci. USA 96, 1840-1845 (1999).
29. Kumar, S. & Daniell, H. Engineering the chloroplast genome for hyper-expression of human therapeutic proteins and vaccine antigens. Methods Mol. Biol. (2003) in press.
30. Larkin, P.J. and Scocrowt, W.R.; 1981; Somaclonal variation - a novel source of variability from cell cultures for plant improvement. Theor. appl. Genet. 60; p. 197-214.
31. Lee, S.B. et al. Accumulation of trehalose within transgenic chloroplasts confers drought tolerance. Mol. Breeding 11, 1-13 (2003).
32. Leelavathi, S. & Reddy, V.S. Chloroplast expression of His-tagged GUS-fusion: a general strategy to overproduce and purify foreign proteins using transplastomic plants as bioreactors. Mol. Bleeding 11, 49-58 (2003).
33. M.M. Bradford, A rapid and sensitive method for the quantification of microgram quantities of protein utilising the principle of protein-dye-binding. Analytical Biochemistry 72, 248-254 (1976)
34. Maris P. Apse and Eduardo Blumwald. Engineering salt tolerance in plants. Curr. Openion in Bitechnol. 13, 146-150 (2002)
35. McBride, K.E. et al. Amplification of a chimeric Bacillus gene in chloroplasts leads to an extraordinary level of an insecticidal protein in tobacco. Bio/Technology 13, 362-365 (1995).
36. Moghaieb, REA., Tanaka, N., Saneoka, H., Hussein, HA., Yousef, SS., Ewada, MAF., Aly, M and Fujita, K. Expression of betaine aldehyde dehydrogenase gene in transgenic tomato hairy roots leads to the accumulation of glycine betaine and contributes to the maintenance of the osmotic potential under salt stress. Soil Science and Plant Nutrition, 46, 873-883 (2000).
37. Murashige T and Skoog F (1962) A revised medium for rapid growth and bioassays with tobacco tissue cultures. Physiol. Plant. 15: 473-497.
38. Nagamori, E., Honda, H. & Kobayashi, T. Release of Embryogenic Carrot Cells with High Regeneration Potency from Immobilized Alginate Beads. Journal of Bioscience and Bioengineering 88, 226-228 (1999).
39. O.L. Gamborg, R.A. Miller and K. Ojima, Nutrient requirements of suspension cultures of soybean root cells. Exp. Cell Res. 50 (1968), pp. 151-158.
40. Predieri S.; 2001; Mutation induction and tissue culture in improving fruits; Plant cell, tissue and organ culture 64; p. 185-210
41. R. Timbert, J. N. Barbotin and D. Thomas. Enhancing carrot somatic embryos survival during slow dehydration, by encapsulation and control of dehydration, Plant Sci. 120, 215-222 (1996)
42. Rontein D, Basset G, Denis and Hanson AD (2002) Metabolic Engineering of Osmoprotectant Accumulation in Plants. Metabolic Engineering 4: 49-56.
43. Ruf S, Hermann M, Berger I, Carrer H, and bock R (2001) Stable genetic transformation of tomato plastids and expression of a foreign protein in fruit. Nat. Biotechnol. 19: 870-875.
44. Ruiz, O.N., Hussein, H., Terry, N. & Daniell, H. Phytoremediation of organomercurial compounds via chloroplast genetic engineering. Plant Physiol. 132, 1-9 (2003).
45. Salt stress enhances choline uptake in the halotolerant cyanobacterium Aphanothece halophytica, Biochimica et Biophysica Acta (BBA) 1621, 102-109 (2003)
46. Sasaki, Y. Morioka, S. & Matsuno, R. Correlation of plastid DNA copy number with plastid gene-expression in various organs in mature pea-plants (Pisum sativum L). Plant Cell Physiol. 31, 925 - 931 (1990).
47. Shinozaki et al. 1987
48. Sidorov VA, Kasten D, Pang SZ, Hajdukiewicz PT, Staub JM and Nehra NS (1999) Technical advance: stable chloroplast transformation in potato: use of green fluorescent protein as a plastid marker. Plant J. 19: 209-216.
49. Sikadar SR, Serino G, Chaudhuri S and Maliga P (1998) Plastid transformation in Arabidopsis thaliana. Plant Cell. Rep. 18: 20-24.
50. Staub, J.M. et al. High-yield production of a human therapeutic protein in tobacco chloroplasts. Nat. Biotechnol. 18, 333-338 (2000).
51. Tessereau, H., Florin, B., Meschine, M.C., Thierry, C. & Pétiard, V. Cryopreservation of somatic embryos: A tool for germplasm storage and commercial delivery of selected plants. Annals of Botany 74, 547-555 (1994).
52. Timbert, R., Barbotin, J.N. & Thomas, D. Enhancing carrot somatic embryos survival during slow dehydration, by encapsulation and control of dehydration. Plant Sci. 120, 215-222 (1996).
53. Tobacco transformation ref.
54. Tregoning, J.S. et al. Expression of tetanus toxin Fragment C in tobacco chloroplasts. Nucleic Acids Res. 31, 1174-1179 (2003).
55. U.K. Laemmli, Cleavage of structural proteins during the assembly of the head of bacteriophage T4. Nature 259 (1970), pp. 680-685.
56. Valenzuela-Soto, E.M. and Muñoz-Clares, R.A., 1994. Purification and properties of betaine aldehyde dehydrogenase extracted from detached leaves of Amaranthus hypochondriacus L. subjected to water deficit. J. Plant Physiol. 143, pp. 145-152
57. Velasco-Garcia R, Gonzalez-Segura L and Munoz-Clares R A, (2000) Steady-state kinetic mechanism of the NADP+ -and NAD+ -dependent reaction catalysed by betaine aldehyde dehydrogenase from Pseudomonas aeruginosa. Biochem. J. 352: 675-683.
58. Vivek B.S., Ngo, Q.A. & Simon PW. Evidence for maternal inheritance of the chloroplast genome in cultivated carrot (Daucus carota L. ssp. Sativus) Theor Appl Genet. 98, 669-672 (1999).
59. Yan, W. & Hunt, L.A. Reanalysis of vernalization data of wheat and carrot. Annals of Botany 84, 615-619 (1999).

## Claims

1. A homoplasmic somatic embryo of a non-green carrot cell transformed with a plastid transformation vector comprising, as operably linked components, a first flanking sequence, a 5' regulatory sequence comprising a Prrn 16S rRNA promoter functional in proplastids and chloroplasts in light and in dark, a DNA sequence coding for a foreign gene, a DNA sequence encoding a selectable marker, and a second flanking sequence, wherein said vector facilitates stable integration into a carrot plastid genome, and wherein said first and second flanking sequence are substantially homologous to sequences in a spacer region of said plastid genome.

2. The homoplasmic somatic embryo of Claim 1, wherein said first and second flanking sequence are species-specific flanking sequences from the chloroplast DNA of carrot.

3. The homoplasmic somatic embryo of Claim 1, wherein said regulatory sequence comprises psbA 5' and psbA 3' elements.

4. The homoplasmic somatic embryo of Claim 1, wherein said regulatory sequences further comprise a 5'UTR capable of providing transcription and translation enhancement of said DNA sequence coding for a foreign gene.

5. The homoplasmic somatic embryo of Claim 1, wherein said regulatory sequences further comprise a 3'untranslated region (UTR) capable of conferring transcript stability to said DNA sequence coding for a foreign gene.

6. The homoplasmic somatic embryo of Claim 1, wherein said regulatory sequence further comprise a gene 10 5'UTR.

7. The homoplasmic somatic embryo of Claim 6, wherein said regulatory sequence further comprises a gene 10 5'UTR/rps 16 3'UTR.

8. The homoplasmic somatic embryo of claim 1, wherein said first flanking sequence is about 4kb.

9. The homoplasmic somatic embryo of claim 1, wherein said second flanking sequence is about 4kb.

10. The homoplasmic somatic embryo of Claim 4, wherein said 5'UTR is a 5'UTR of psbA.

11. The homoplasmic somatic embryo of Claim 5, wherein said 3'UTR is a 3'UTR of psbA.

12. The homoplasmic somatic embryo of Claim 1, wherein said selectable marker is an antibiotic- free selectable marker.

13. The homoplasmic somatic embryo of Claim 12, wherein said antibiotic-free selectable marker is Betaine aldehyde dehydrogenase (BADH).

14. The homoplasmic somatic embryo of Claim 1, wherein said DNA sequence encoding said selectable marker encodes an antibiotic resistance selectable marker.

15. The homoplasmic somatic embryo of Claim 14, wherein said antibiotic resistance selectable marker is aadA.

16. The homoplasmic somatic embryo of claim 14, wherein said antibiotic resistance selectable marker is an aminoglycosidase selected from the group consisting of aphA-2 or aphA-6.

17. The homoplasmic somatic embryo of claim 16, wherein said antibiotic is kanamycin.

18. A method of producing a homoplasmic somatic embryo of a non-green carrot cell according to any of claims 1 to 17 by somatic embryogenesis comprising the step of integrating a plastid transformation vector comprising, as operably linked components, a first flanking sequence, a 5' regulatory sequence comprising a Prrn 16S rRNA promoter functional in proplastids and chloroplasts in light and in dark, a DNA sequence coding for a foreign gene, a DNA sequence encoding a selectable marker, and a second flanking sequence, into a plastid genome of a carrot plastid.

19. A method of transforming a plastid through somatic embryogenesis comprising the steps of: integrating a plastid transformation vector comprising, as operably linked components, a first flanking sequence, a 5' regulatory sequence comprising a Prrn 16S rRNA promoter functional in proplastids and chloroplasts in light and in dark, a DNA sequence coding for a foreign gene, a DNA sequence encoding a selectable marker, and a second flanking sequence, into a plastid genome of a non-green carrot cell plastid, wherein said first and second flanking sequence are substantially homologous to sequences in a spacer region of said plastid genome.

## Patentansprüche

1. Homoplasmischer somatischer Embryo einer nicht-grünen Karottenzelle, die mit einem Plastiden-Transformationsvektor transformiert ist, welcher als operativ verknüpfte Bestandteile umfasst:
eine erste flankierende Sequenz, eine 5'-regulatorische Sequenz, die einen Prrn-16S-rRNA-Promotor umfasst, der in Proplastiden und Chloroplasten im Licht und in der Dunkelheit funktional ist, eine DNA-Sequenz, die ein fremdes Gen kodiert, eine DNA-Sequenz, die einen selektierbaren Marker kodiert, und eine zweite flankierende Sequenz,
wobei der Vektor eine stabile Integration in ein Plastidengenom der Karotte erleichtert, und wobei die erste und zweite flankierende Sequenz im Wesentlichen homolog zu Sequenzen in einer Spacer-Region des Plastidengenoms sind.

2. Homoplasmischer somatischer Embryo nach Anspruch 1, wobei die erste und zweite flankierende Sequenz speziesspezifische flankierende Sequenzen aus der Chloroplasten-DNA der Karotte sind.

3. Homoplasmischer somatischer Embryo nach Anspruch 1, wobei die regulatorische Sequenz psbA-5'- und psbA-3'-Elemente umfasst.

4. Homoplasmischer somatischer Embryo nach Anspruch 1, wobei die regulatorischen Sequenzen zusätzlich eine 5'UTR umfassen, die in der Lage ist, eine Transkriptions- und Translationsverstärkung der DNA-Sequenz, die ein fremdes Gen kodiert, zu bewirken.

5. Homoplasmischer somatischer Embryo nach Anspruch 1, wobei die regulatorischen Sequenzen zusätzlich eine 3'-untranslatierte Region (UTR) umfassen, die in der Lage ist, der DNA-Sequenz, die ein fremdes Gen kodiert, Transkriptstabilität zu verleihen.

6. Homoplasmischer somatischer Embryo nach Anspruch 1, wobei die regulatorische Sequenz zusätzlich eine Gen-10-5'UTR umfasst.

7. Homoplasmischer somatischer Embryo nach Anspruch 6, wobei die regulatorische Sequenz zusätzlich eine Gen-10-5'UTR/rps-16-3'UTR umfasst.

8. Homoplasmischer somatischer Embryo nach Anspruch 1, wobei die erste flankierende Sequenz ungefähr 4 kb groß ist.

9. Homoplasmischer somatischer Embryo nach Anspruch 1, wobei die zweite flankierende Sequenz ungefähr 4 kb groß ist.

10. Homoplasmischer somatischer Embryo nach Anspruch 4, wobei die 5'UTR eine 5'UTR von psbA ist.

11. Homoplasmischer somatischer Embryo nach Anspruch 5, wobei die 3'UTR eine 3'UTR von psbA ist.

12. Homoplasmischer somatischer Embryo nach Anspruch 1, wobei der selektierbare Marker ein antibiotikumfreier selektierbarer Marker ist.

13. Homoplasmischer somatischer Embryo nach Anspruch 12, wobei der antibiotikumfreie selektierbare Marker Betain-Aldehyd-Dehydrogenase (BADH) ist.

14. Homoplasmischer somatischer Embryo nach Anspruch 1, wobei die DNA-Sequenz, die den selektierbaren Marker kodiert, einen selektierbaren Antibiotikaresistenzmarker kodiert.

15. Homoplasmischer somatischer Embryo nach Anspruch 14, wobei der selektierbare Antibiotikaresistenzmarker aadA ist.

16. Homoplasmischer somatischer Embryo nach Anspruch 14, wobei der selektierbare Antibiotikaresistenzmarker eine Aminoglycosidase ist, die aus der Gruppe ausgewählt ist, die aus aphA-2 oder aphA-6 besteht.

17. Homoplasmischer somatischer Embryo nach Anspruch 16, wobei das Antibiotikum Kanamycin ist.

18. Verfahren zur Erzeugung eines homoplasmischen somatischen Embryos einer nicht-grünen Karottenzelle gemäß einem der Ansprüche 1 bis 17 durch somatische Embryogenese, umfassend den Schritt des Integrierens eines Plastiden-Transformationsvektors, welcher als operativ verknüpfte Bestandteile eine erste flankierende Sequenz, eine 5'-regulatorische Sequenz, die einen Prrn-16S-rRNA-Promotor umfasst, der in Proplastiden und Chloroplasten im Licht und in der Dunkelheit funktional ist, eine DNA-Sequenz, die ein fremdes Gen kodiert, eine DNA-Sequenz, die einen selektierbaren Marker kodiert, und eine zweite flankierende Sequenz umfasst, in ein Plastidengenom eines Karottenplastiden.

19. Verfahren zum Transformieren eines Plastiden durch somatische Embryogenese, umfassend die Schritte:
integrieren eines Plastiden-Transformationsvektors, welcher als operativ verknüpfte Bestandteile eine erste flankierende Sequenz, eine 5'-regulatorische Sequenz, die einen Prrn-16S-rRNA-Promotor umfasst, der in Proplastiden und Chloroplasten im Licht und in der Dunkelheit funktional ist, eine DNA-Sequenz, die ein fremdes Gen kodiert, eine DNA-Sequenz, die einen selektierbaren Marker kodiert, und eine zweite flankierende Sequenz umfasst, in ein Plastidengenom eines Plastiden einer nicht-grünen Karottenzelle,
wobei die erste und zweite flankierende Sequenz im Wesentlichen homolog zu Sequenzen in einer Spacer-Region des Plastidengenoms sind.

## Revendications

1. Embryon somatique homoplasmique d'une cellule de carotte non-verte transformée avec un vecteur de transformation de plastides comprenant, à titre de composants liés de manière opérationnelle, une première séquence d'encadrement, une séquence régulatrice 5' comprenant un promoteur d'ARNr Prrn 16S fonctionnel dans les proplastides et les chloroplastes à la lumière et dans l'obscurité, une séquence d'ADN codant pour un gène étranger, une séquence d'ADN codant pour un marqueur sélectionnable, et une deuxième séquence d'encadrement, tandis que ledit vecteur facilite l'intégration stable dans un génome de plasmide de carotte, et tandis que lesdites première et deuxième séquences d'encadrement sont substantiellement homologues des séquences dans une région d'espaceur dudit génome de plastide.

2. Embryon somatique homoplasmique selon la revendication 1, dans lequel lesdites première et deuxième séquences d'encadrement sont des séquences d'encadrement spécifiques pour une espèce provenant de l'ADN des chloroplastes de carotte.

3. Embryon somatique homoplasmique selon la revendication 1, dans lequel ladite séquence régulatrice comprend des éléments psbA 5' et psbA 3';

4. Embryon somatique homoplasmique selon la revendication 1, dans lequel lesdites séquences régulatrices comprennent en outre une 5'UTR capable de procurer un accroissement de transcription et de traduction de ladite séquence d'ADN codant pour un gène étranger.

5. Embryon somatique homoplasmique selon la revendication 1, dans lequel lesdites séquences régulatrices comprennent en outre une région 3'non-traduite (UTR) capable de conférer de la stabilité vis-à-vis de la transcription à ladite séquence d'ADN codant pour un gène étranger.

6. Embryon somatique homoplasmique selon la revendication 1, dans lequel ladite séquence régulatrice comprend en outre une 5'UTR de gène 10.

7. Embryon somatique homoplasmique selon la revendication 6, dans lequel ladite séquence régulatrice comprend en outre une 5'UTR de gène 10/3'UTR de rps 16.

8. Embryon somatique homoplasmique selon la revendication 1, dans lequel ladite première séquence d'encadrement est d'environ 4kb.

9. Embryon somatique homoplasmique selon la revendication 1, dans lequel ladite deuxième séquence d'encadrement est d'environ 4kb.

10. Embryon somatique homoplasmique selon la revendication 4, dans lequel ladite 5'UTR est une 5'UTR de psbA.

11. Embryon somatique homoplasmique selon la revendication 5, dans lequel ladite 3'UTR est une 3'UTR de psbA.

12. Embryon somatique homoplasmique selon la revendication 1, dans lequel ledit marqueur sélectionnable est un marqueur sélectionnable exempt d'antibiotiques.

13. Embryon somatique homoplasmique selon la revendication 12, dans lequel ledit marqueur sélectionnable exempt d'antibiotiques est la bétaïne aldéhyde déshydrogénase (BADH).

14. Embryon somatique homoplasmique selon la revendication 1, dans lequel ladite séquence d'ADN codant pour ledit marqueur sélectionnable code pour un marqueur sélectionnable de la résistance aux antibiotiques.

15. Embryon somatique homoplasmique selon la revendication 14, dans lequel ledit marqueur sélectionnable de la résistance aux antibiotiques est aadA.

16. Embryon somatique homoplasmique selon la revendication 14, dans lequel ledit marqueur sélectionnable de la résistance aux antibiotiques est une aminoglycosidase choisie dans le groupe consistant en aphA-2 ou aphA-6.

17. Embryon somatique homoplasmique selon la revendication 16, dans lequel ledit antibiotique est la kanamycine.

18. Procédé pour la production d'un embryon somatique homoplasmique d'une cellule de carotte non-verte selon l'une quelconque des revendications 1 à 17 par embryogenèse somatique comprenant l'étape d'intégration d'un vecteur de transformation des plastides comprenant, en tant que composants liés de manière opérationnelle, une première séquence d'encadrement, une séquence régulatrice en 5' comprenant un promoteur d'ARNr Prrn 16S fonctionnel dans les proplastides et les chloroplastes à la lumière et dans l'obscurité, une séquence d'ADN codant pour un gène étranger, une séquence d'ADN codant pour un marqueur sélectionnable, et une deuxième séquence d'encadrement, dans un génome plastidique d'un plastide de carotte.

19. Procédé pour la transformation d'un plastide par l'intermédiaire d'une embryogenèse somatique comprenant les étapes de: intégration d'un vecteur de transformation des plastides comprenant, en tant que composants liés de manière opérationnelle, une première séquence d'encadrement, une séquence régulatrice en 5' comprenant un promoteur d'ARNr Prrn 16S fonctionnel dans les proplastides et les chloroplastes à la lumière et dans l'obscurité, une séquence d'ADN codant pour un gène étranger, une séquence d'ADN codant pour un marqueur sélectionnable, et une deuxième séquence d'encadrement, dans un génome plastidique d'un plastide de cellule de carotte non-verte, tandis que lesdites première et deuxième séquences d'encadrement sont substantiellement homologues vis-à-vis de séquences dans une région d'espaceur dudit génome de plastides.
